# FASCICULE DE BREVET EUROPEEN

(11) **EP 2 260 048 B1**
(45) Date de publication et mention de la délivrance du brevet: **28.03.2012**
(21) Numéro de dépôt: 09738312.9
(22) Date de dépôt: 31.03.2009
(51) Int. Cl.: C07F 15/02, A61K 31/295, A61P 31/04, A61P 7/02, A61K 8/19

(54) **SOLIDE HYBRIDE CRISTALLIN POREUX POUR L'ADSORPTION ET LA LIBERATION DE GAZ A INTERET BIOLOGIQUE**
PORÖSER UND KRISTALLINER HYBRIDFESTKÖRPER ZUR AUFNAHME UND FREIGABE EINES GASES VON BIOLOGISCHEM INTERESSE
POROUS CRYSTALLINE HYBRID SOLID FOR ADSORBING AND RELEASING GAS OF BIOLOGICAL INTEREST

(30) Priorité: 01.04.2008 FR 0852150; 10.06.2008 FR 0803214
(43) Date de publication de la demande: 15.12.2010
(73) Titulaire: Centre National de la Recherche Scientifique - CNRS, 75794 Paris Cedex 16 (FR); University of St. Andrews, St Andrews, Fife KY16 9SR (GB); Université de Versailles Saint-Quentin-en-Yvelines, 78053 Versailles Cédex (FR); Ensi Caen, 14050 Caen Cedex 4 (FR)
(72) Inventeur: MORRIS, Russel, Gauldry DD6 8SH (GB); SERRE, Christian, F-78730 Plaisir (FR); HORCAJADA CORTES, Patricia, 92370 Chaville (FR); VIMONT, Alexandre, F-14810 Merville-Franceville (FR); DEVIC, Thomas, F-91140 Villebon sur Yvette (FR); FEREY, Gerard, F-75015 Paris (FR)
(74) Mandataire: Novagraaf Technologies
(86) Numéro de dépôt international: PCT/FR2009/000381
(87) Numéro de publication internationale: WO 2009/133278

(56) Documents cités:
- WO-A-2008/020218
- XIAO BO ET AL: "High-capacity hydrogen and nitric oxide adsorption and storage in a metal-organic framework" JOURNAL OF THE AMERICAN CHEMICAL SOCIETY, vol. 129, no. 5, 7 février 2007 (2007-02-07), pages 1203-1209, XP009093205 ISSN: 0002-7863 cité dans la demande
- HORCAJADA PATRICIA ET AL: "Synthesis and catalytic properties of MIL - 100 ( Fe ), an iron(III) carboxylate with large pores" CHEMICAL COMMUNICATIONS - CHEMCOM, no. 27, 1 janvier 2007 (2007-01-01), pages 2820-2822, XP002476454 ISSN: 1359-7345 [extrait le 2007-05-15]
- LLEWELLYN PHILIP L ET AL: "High Uptakes of CO2 and CH4 in Mesoporous Metal-Organic Frameworks MIL-100 and MIL-101" LANGMUIR, 21 mars 2008 (2008-03-21), pages A-F, XP002476457 ISSN: 0743-7463 [extrait le 2008-03-21]
- HORCAJADA PATRICIA ET AL: "Metal-organic frameworks as efficient materials for drug delivery" ANGEWANDTE CHEMIE. INTERNATIONAL EDITION, vol. 45, no. 36, 1 janvier 2006 (2006-01-01), pages 5974-5978, XP002476583 ISSN: 1433-7851
- MELLOT-DRAZNIEKS CAROLINE ET AL: "Very Large Swelling in Hybrid Frameworks: A Combined Computational and Powder Diffraction Study" JOURNAL OF THE AMERICAN CHEMICAL SOCIETY, vol. 127, no. 46, 1 janvier 2005 (2005-01-01), pages 16273-16278, XP002476453 ISSN: 0002-7863 [extrait le 2005-10-28]

## Description

### Domaine technique

La présente invention se rapporte à des solides constitués de réseau métal-organique ou « Metal-Organic Framework » (MOF), cristallin poreux chargé en au moins un gaz d'intérêt biologique, ainsi, qu'à leur procédé de préparation.

Les solides MOF de la présente invention sont capables d'adsorber et de relarguer de manière contrôlée des gaz à intérêt biologique. Ils peuvent être utilisés dans le domaine pharmaceutique et/ou pour des applications dans le domaine cosmétique. Ils peuvent également être utilisés dans le domaine alimentaire.

Les références entre crochets [X] renvoient à la liste des références à la fin des exemples.

### État de la technique

Les réseaux métal-organique ou « Metal-Organic Framework » (MOF) sont des polymères de coordination de charpente hybride inorganique-organique comprenant des ions métalliques et des ligands organiques coordinés aux ions métalliques. Ces matériaux sont organisés en réseau mono-, bi- ou tridimensionnels où les clusters métalliques sont reliés entre eux par des ligands espaceurs de façon périodique. Ces matériaux ont une structure cristalline, sont le plus souvent poreux et sont utilisés dans de nombreuses applications industrielles telles que le stockage de gaz, l'adsorption de liquides, la séparation de liquides ou de gaz, la catalyse, etc.

On peut citer par exemple la demande de brevet US 10/039,733 [1] qui décrit un procédé réactionnel faisant intervenir un système de catalyse comprenant un matériau MOF à base de Zinc. Ce même matériau est également utilisé pour le stockage de gaz dans la demande de brevet US 10/061,147 [2].

En outre, les matériaux MOF basés sur des réseaux de même topologie sont qualifiés de « isoréticulaires ». Ces réseaux organisés dans l'espace ont permis d'obtenir une porosité plus homogène. Ainsi, la demande de brevet US 10/137,043 [3] décrit plusieurs matériaux IRMOF (Isoreticular Metal-Organic Framework) à base de zinc utilisés pour le stockage de gaz.

Par ailleurs, les gaz d'intérêt biologique tels que le NO, CO et H₂S sont d'une importance extrême pour le fonctionnement biologique des mammifères. Ils interviennent dans un grand nombre de processus comme par exemple la vasodilatation, la prévention de l'agrégation des plaquettes et la formation de thrombose, la neurotransmission et la guérison des plaies.

Il est connu que tant le monoxyde de carbone (CO) comme l'oxyde nitrique (NO) à très faibles concentrations ont une activité importante comme molécules de signalisation dans le corps.

NO a été amplement étudié dans la biologie [32]. L'activité biologique du NO comprend [33] :
- activité anti-inflammatoire,
- régulation de la dysfonction sexuelle,
- indications cardiovasculaires (traitement de l'angine de poitrine.

De plus, NO est impliqué dans l'activité de nombreux médicaments (bloqueur de chanel de calcium, inhibiteurs de l'ACE et antagonistes du récepteur ANGII type 1, β-bloqueur et inhibiteurs de la réductase hydroxymethylglutaryl-CoA).

Concernant le CO, même si le mécanisme d'action doit encore être élucidé pour le CO, son implication dans nombreux effets physiologiques est connue et décrite [34]. Ainsi, le CO est impliqué dans des activités biologiques comme par exemple :
1. l'activité anti-inflamanatoire
   - diminue le endotoxique shock [35],
   - diminue l'inflammation allergique [36] ;
2. la suppression du rejet des organes greffés [37] ;
3. la protection contre l'hyperoxia [38] ;
4. la protection contre l'ischémie [39] ;
5. la protection des cellules bêta pancréatiques de l'apoptose [40] ;
6. la modulation de la spermatogenèse dans des conditions de stress à travers des cellules de Leydig [41] ;
7. la diminution de la pression de perfusion dans les régions isolées de la placenta humaine [42] ;
8. la protection contre le choc septique et des lésions pulmonaires chez les modèles animaux,
9. la modulation du tonus des muscles lisses vasculaires [44] ;
10. la regulation de la pression artérielle en situation de stress [45] ;
11. la suppression des lésions artériosclérotiques associés aux maladies chroniques et le rejet de greffe [46].

Les recherches scientifique sur le rôle des émissions de CO dans l'organisme sont encore à un stade précoce. Il y a des recherches qui laissent entendre qu'il peut aussi intervenir significativement dans d'autres domaines de la médecine y compris la chirurgie de transplantation, la neuroprotection dans les accidents vasculaires cérébraux, la maladie d'Alzheimer [47]. Le CO est aussi impliqué dans le contrôle de la fonction vasculaire placentaire [48].

Le rôle bénéfique de CO dans l'organisme a également été décrit dans trois demandes de brevet : US Patent 2002155166, WO 0278684, WO 02092075. Ces demandes décrivent l'application du gaz CO dans le domaine médicale. US 2002155166 décrit par exemple l'utilisation de CO en tant que marqueur biologique et agent thérapeutique pour différentes types de maladies et dans le domaine de la transplantation. WO 0278684 porte sur les méthodes et les compositions pour traiter les maladies vasculaire, inflammatoire et du système immunitaire utilisant des composés capables de générer du CO *in vivo.* CH₂Cl₂ est décrit comme étant le composé préféré capable de générer du CO *in vivo.* En effet, le CH₂Cl₂ est métabolisé en CO et fournit ainsi une source de gaz. WO 02092075 utilise les métaux carbonylés comme la source des émissions de CO gaz.

L'intérêt du H₂S comme une molécule de signalisation est de plus en plus important [49]. Le pKa de H₂S étant de 6,8, à pH physiologique il est surtout présent en tant que H₂S et [HS]₂. La concentration de H₂S est de 50-160 mM dans le tissu cérébral et de 10-100 mM dans le sang.

H₂S est actif dans le système cardio-vasculaire et dans le système nerveux central. Par exemple, il peut causer une vasodilatation [50]. Par ailleurs, il est capable de coordonner facilement les groupes hemo et certains cytochromes. Plus de recherche est nécessaire pour démontrer l'importance de H₂S en tant que molécule de signalisation.

L'utilisation de ces gaz exogènes ouvre de très nombreuses possibilités et est devenu un enjeu important pour le développement notamment de nouveaux médicaments ou des procédés prophylactiques et thérapeutiques incluant des applications potentielles dans les procédés anti-thrombogéniques, l'amélioration de l'efficacité du traitement de plaies et des ulcères, le traitement des infections fongiques et bactériennes etc. La libération contrôlée de ce type de gaz, en particulier NO, de par ses propriétés anti-bactériennes, pourrait également s'avérer utile pour des applications en cosmétiques, en particulier pour les crèmes cosmétiques [4], mais également pour la conservation des aliments (effet antibactérien et antioxydant) [5].

Dans le cas particulier de NO, la délivrance homogène de ce gaz à partir d'une solution est déjà connue pour certaines pathologies (i.e. à partir de trinitrate de glycéryl pour traiter l'angine). Toutefois, cette approche est restreinte à cause d'effets secondaires indésirables comme conséquence de la grande variété d'effets qu'il a en fonction de la localisation (vasodilatateur et inhibiteur de l'agrégation plaquettaire, endothélium ; microbicide, macrophages dans certaines circonstances le NO peut provoquer des effets secondaires nuisibles : c'est le cas dans les septicémies, où la production excessive de NO par les macrophages conduit à une vasodilatation massive, cause principale de l'hypotension rencontrée dans le choc septique ; neurotransmetteur, cellules nerveuses ; relaxante des muscles lisses, tube digestif ; régulateur de l'apoptose, antiapoptotique ou apoptotique en fonction de la présence ou non de réducteurs cellulaires).

L'inhalation de NO gaz a également été utilisée pour traiter certaines pathologies des poumons. Cependant, la délivrance de NO sous forme gazeuse à partir de bouteilles de gaz n'est pas pratique et limite l'intérêt d'une telle méthode.

Par ailleurs, une part significative des thérapies liées au gaz d'intérêt biologique nécessite un relargage ou libération contrôlé et ciblé dudit gaz dans des zones spécifiques du corps humain évitant ainsi des effets systémiques [6]. Dans le cas particulier de NO, ceci est très important car NO a une durée de vie biologique courte.

Les technologies actuelles possèdent de nombreux inconvénients. Par exemple, les polymères de type NONOate ou à composante métallique ont une faible capacité de stockage, nécessitent une pression élevée pour charger le NO, sont relativement coûteux et potentiellement toxiques [7,8].

Dans des applications dermatologiques, les crèmes acides à base de nitrates, qui sont des donneurs potentiels de NO, sont pro-inflammatoires et ne sont pas adaptées aux peaux sensibles [9].

Récemment, l'utilisation de solides poreux de types zéolites ou de solides hybrides pour le stockage de NO a été décrite [10, 11, 12]. Ces solides possèdent une capacité de stockage qui surpasse celle des autres matériaux, une durée de vie longue (la capacité de relargage de NO après 2 ans de stockage reste intacte), un faible coût et ne semblent pas présenter de toxicité les rendant ainsi très attractifs.

De plus, les tests dermatologiques ont montré la compatibilité des zéolites chargés de NO avec 1a. peau humaine y compris avec les peaux sensibles [13].

Malgré les avantages précités, la délivrance de NO avec les zéolithes ne peut se faire que sur une courte durée, les rendant ainsi inadaptés pour une application pour laquelle une libération de longue durée est souhaitée

L'adsorption, le stockage et la libération du NO par des réseaux métal-organique poreux à base de cuivre ou aluminium pour inhiber l'agrégation plaquettaire ont également été décrites. Malgré la grande capacité d'adsorption et de stockage de NO par ces solides (adsorption significativement améliorée par rapport aux autres solides comme les polymères organiques ou les zéolithes), une fois en contact avec une solution biologique/physiologique (plasma riche en plaquette), ces solides montre une faible stabilité.

Comme mentionné, l'une des applications particulièrement importante dans le domaine du relargage des gaz à intérêt biologique et en particulier du NO, concerne les appareillages antithrombotiques comme par exemple les stents, les cathéters et les canules qui sont insérés dans le flux sanguin avec des durées variables pour des motifs thérapeutiques ou diagnostiques ainsi que des circuits extracorporels utilisés pour la dialyse rénale et en chirurgie.

En effet, la prévention des thromboses est d'une importance vitale après l'insertion des stents, cathéters, conduits prosthétiques et autres implants médicaux dans le corps, pendant l'opération chirurgicale, ce qui peut conduire souvent à des complications dangereuses notamment pour cause d'occlusion des vaisseaux sanguins.

Parmi les inconvénients des systèmes déjà connus, on peut citer par exemple :
- manque de libération ciblée des gaz, qui conduit à de nombreux effets secondaires indésirables,
- quantité libérée mal contrôlée, et donc potentiellement non adaptée à l'application requise,
- durée de libération des gaz en milieu physiologique courte ou accompagné de libération de substances indésirables, et/ou
- une faible stabilité en milieu biologique/physiologique limitant ainsi leur utilisation dans de tels milieux.

Il existe donc un besoin réel de développer des systèmes permettant l'adsorption et le relargage de gaz d'intérêt biologique, dans lesquels lesdits systèmes possèdent une forte capacité d'adsorption et le relargage des gaz peut se faire de manière continue et ciblée.

De plus, il existe un besoin réel de disposer de systèmes permettant de délivrer de manière contrôlée la quantité optimale de gaz nécessaire pour une application donnée.

En outre, il existe un réel besoin de disposer de systèmes permettant de contrôler la durée de libération des gaz en milieu physiologique, notamment pour pouvoir avoir des libérations de longue durée pouvant aller au-delà de 6 heures tout en préservant leur stabilité en milieu biologique/physiologique pendant toute la durée de cette libération.

De plus, il existe un réel besoin de disposer de systèmes ayant des capacités de charge suffisantes surtout si l'on envisage des administrations répétées de gaz.

### Exposé de l'invention

La présente invention a précisément pour but de répondre à ces besoins et inconvénients de l'art antérieur en fournissant des solides MOF cristallins poreux chargés en au moins un gaz base de Lewis choisi dans le groupe comprenant NO, CO et H₂S dont au moins une partie se coordine avec M, ledit solide comprenant une succession tridimensionnelle de motifs répondant à la formule (I) suivante :

MₘOₖXₗLₚ (I)

dans laquelle :
- chaque occurrence de M, représente un ion d'un métal de transition M dans laquelle M est Fe, et dans lequel z est de 2 à 4;
- m est 1 à 12 ;
- k est 0 à 4 ;
- l est 0 à 18 ;
- p est 1 à 6 ;
- X est un anion choisi dans le groupe comprenant OH⁻, Cl⁻, F⁻, I⁻, Br⁻, SO₄²⁻, NO₃⁻, ClO₄⁻, PF₆⁻, BF₄⁻, R-(COO)ₙ⁻ où R est tel que défini ci-dessous, R¹-(COO)ₙ⁻, R¹-(SO₃)ₙ⁻,R¹-(PO₃)ₙ⁻,où R¹ est un hydrogène, un alkyle en C₁ à C₁₂, linéaire ou ramifié, éventuellement substitué, un aryle, où n est un nombre entier de 1 à 4 ;
- L est un ligand espaceur comprenant un radical R
comportant q groupements carboxylates 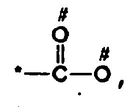 où
- q est 2, 3, 4, 5 ou 6 ; * désigne le point d'attachement du carboxylate avec le radical R ;
- # désigne les points d'attachement possibles du carboxylate à l'ion métallique ;
- R représente :
   (i) un radical C₁₋₁₂alkyle, C₂₋₁₂alcène ou C₂₋₁₂alcyne ;
   (ii) un radical aryle mono- ou poly-cyclique, fusionné ou non, comprenant 6 à 50 atomes de carbone ;
   (iii) un hétéroaryle mono- ou poly-cyclique, fusionné ou non, comprenant 1 à 50 atomes de carbone ;
   (iv) un radical organique comprenant un élément métallique choisi dans le groupe comprenant le ferrocène, la porphyrine, la phthalocyanine;
   le radical R étant éventuellement substitué par un ou plusieurs groupes R², indépendamment choisis dans le groupe comprenant C₁₋₁₀alkyle; C₂₋₁₀alcène; C₂₋₁₀alcyne; C₃₋₁₀cycloalkyle; C₁₋₁₀hétéroalkyle; C₁₋₁₀haloalkyle; C₆₋₁₀aryle; C₃₋₂₀hétérocyclique; C₁₋₁₀alkylC₆₋₁₀aryle; C₁₋₁₀alkylC₃₋₁₀hétéroaryle ; F ; Cl ; Br; I; -NO₂ ; -CN ; -CF₃; -CH₂CF₃ ; -OH ; -CH₂OH ; -CH₂CH₂OH ; -NH₂ ; -CH₂NH₂ ; -NHCHO ; -COOH ; -CONN₂ ; -SO₃H ; -CH₂SO₂CH₃ ; -PO₃H₂ ; ou une fonction -GR^{G1} dans laquelle G est -O-, -S-, -NR^{G2}-, -C(=O)-, -S(=O)-, -SO₂-, -G(=O)O-, -C(=O)NR^{G2}-, -OC(=O)-, -NR^{G2}C(=O)-, -OC(=O)O-, -OC(=O)NR^{G2}-, -NR^{G2}C(=O)O-, -NR^{G2}C(=O)NR^{G2}-, -C(=S)-, où chaque occurrence de R^{G2} est indépendamment des autres occurrences de R^{G2} un atome d'hydrogène ; ou une fonction C₁₋₁₂alkyle, C₁₋₁₂hétéroalkyle, C₂₋₁₀alcène ou C₂₋₁₀alcyne, linéaire, ramifiée ou cyclique, éventuellement substituée ; ou un groupe C₆₋₁₀aryle, C₃₋₁₀hétéroaryle, C₅₋₁₀hétérocyclique, C₁₋₁₀alkyleC₆₋₁₀aryle ou C₁₋₁₀alkyleC₃₋₁₀hétéroaryle dans lequel le radical aryle, hétéroaryle ou hétérocyclique est éventuellement substitué ; ou bien, lorsque G représente -NR^{G2}-, R^{G1} et R^{G2} conjointement avec l'atome d'azote auquel ils sont liés forment un hétérocycle ou un hétéroaryle éventuellement substitué.

Les MOF de selon invention, présente, entre autres, l'avantage :
- d'être à base de métaux non toxiques et donc adaptés à une application dans les domains pharmaceutique, médicales et/ou cosmétiques,
- présenter une stabilité supérieure à celle décrites pour les métaux tels que le cuivre ou l'aluminium,
- présenter une stabilité modulable en fonction du choix de la structure et du ligand organique, permettant ainsi d'adapter les MOF aux différences applications souhaitées.

Dans le cadre de la présente invention, les termes « relarguer/relargage », « libérer/libération » et «délivrer/délivrance » seront indifféremment utilisés pour signifier le dégagement partiel ou total des gaz présents dans les solides MOF.

Par « partiel », on entend une libération de moins de 100% de la quantité initialement adsorbée.

Le terme « substitué » désigne par exemple le remplacement d'un radical hydrogène dans une structure donnée par un radical R² tel que défini précédemment. Lorsque plus d'une position peut être substituée, les substituants peuvent être les mêmes ou différents à chaque position.

On entend par « ligand espaceur » au sens de la présente invention, un ligand (incluant par exemple les espèces neutres et les ions) coordiné à au moins deux métaux, participant à l'éloignement entre ces métaux et à la formation d'espaces vides ou pores. Le ligand espaceur peut comprendre 1 à 6 groupements carboxylates, tels que définis précédemment, qui peuvent être monodentates ou bidentates, c'est-à-dire comprendre un ou deux points d'attachement au métal. Les points d'attachement au métal sont représentés par le sigle # dans les formules. Lorsque la structure d'une fonction A comporte deux points d'attachement #, cela signifie que la coordination au métal peut se faire par l'un, l'autre ou les deux points d'attachement.

On entend par « alkyle » au sens de la présente invention, un radical carboné linéaire, ou ramifié saturé, éventuellement substitué, comprenant 1 à 12 atomes de carbone, par exemple 1 à 10 atomes de carbone, par exemple 1 à 8 atomes de carbone, par exemple 1 à 6 atomes de carbone.

On entend par « alcène » au sens de la présente invention, un radical alkyle, tel que défini précédemment, présentant au moins une double liaison carbone-carbone.

On entend par « alcyne » au sens de la présente invention, un radical alkyle, tel que défini précédemment, présentant au moins une triple liaison carbone-carbone.

On entend par « aryle » au sens de la présente invention, un système aromatique comprenant au moins un cycle satisfaisant la règle d'aromaticité de Hückel. Ledit aryle est éventuellement substitué et peut comprendre de 6 à 50 atomes de carbone, par exemple 6 à 20 atomes de carbone, par exemple 6 à 10 atomes de carbone.

On entend par « hétéroaryle » au sens de la présente invention, un système comprenant au moins un cycle aromatique de 5 à 50 chaînons parmi lesquels au moins un chaînon du cycle aromatique est un hétéroatome, notamment choisi dans le groupe comprenant le soufre, l'oxygène, l'azote, le bore. Ledit hétéroaryle est éventuellement substitué et peut comprendre de 1 à 50 atomes de carbone, de préférence 1 à 20 atomes de carbone, de préférence 3 à 10 atomes de carbone.

On entend par « cycloalkyle » au sens de la présente invention, un radical carboné cyclique, saturé éventuellement substitué, qui peut comprendre 3 à 20 atomes de carbone, de préférence 3 à 10 atomes de carbone.

On entend par « haloalkyle » au sens de la présente invention, un radical alkyle tel que défini précédemment, ledit système alkyle comprenant au moins un halogène.

On entend par « hétéroalkyle » au sens de la présente invention, un radical alkyle tel que défini précédemment, ledit système alkyle comprenant au moins un hétéroatome, notamment choisi dans le groupe comprenant le soufre, l'oxygène, l'azote, le bore.

On entend par « hétérocycle » au sens de la présente invention, un radical carboné cyclique comprenant au moins un hétéroatome, saturé ou insaturé, éventuellement substitué, et qui peut comprendre 2 à 20 atomes de carbone, de préférence 5 à 20 atomes de carbone, de préférence 5 à 10 atomes de carbone. L'hétéroatome peut être par exemple choisi dans le groupe comprenant le soufre, l'oxygène, l'azote, le bore.

On entend par « alkoxy », « aryloxy », «hétéroalkoxy » et « hétéroaryloxy » au sens de la présente invention, respectivement un radical alkyle, aryle, hétéroalkyle et hétéroaryle liés à un atome d'oxygène.

On entend par « alkylthio », « arylthio », « hétéroalkylthio » et « hétéroarylthio » au sens de la présente invention, respectivement un radical alkyle, aryle, hétéroalkyle et hétéroaryle liés à un atome de soufre.

Les gaz de Lewis selon l'invention sont des gaz à intérêt biologique choisis dans le groupe comprenant NO, CO et H₂S. De préférence, ledit gaz est du NO.

La structure cristalline particulière des solides MOF selon l'invention procure à ces matériaux des propriétés spécifiques.

Dans les solides MOF décrits dans la présente, M est choisi dans le groupe comprenant Fe, Ti, Mn, Zr. M peut également être un mélange de ces métaux. Dans les solides MOF de l'invention M est le Fe.

Comme indiqué précédemment, M est un ion de métal de transition M¹⁺ dans lequel z est de 2 à 4. Dans la présente, il est décrit que lorsque M est un mélange de métaux, pour chaque métal, z peut être avoir une valeur identique ou différente.

Dans la présente, il est décrit que les solides comprennent une succussion tridimensionnelle de motifs de formule (I) dans laquelle M peut représenter un seul type d' ion M²⁺, par exemple le Fe, dans lequel z peut être identique ou différent, par exemple 2, 3 ou un mélange de 2 et de 3. Dans la présente, il est décrit que les solides peuvent comprendre une succession tridimensionnelle de motifs de formule (I) dans laquelle M peut représenter un mélange de différents ions M²⁺, par exemple le Fe et le Ti, dans lequel pour chaque ion métallique z peut être identique ou différent, par exemple 2, 3, 4 ou un mélange de 2, 3 et 4.

Dans la présente, il est décrit que M²⁺ représente le Fe trivalent octaédrique avec z égal à 3, le Fe présentant un nombre de coordination de 6.

Par le nombre coordination, on entend le nombre de liaison pour laquelle les deux électrons partagés dans la liaison proviennent du même atome. L'atome donneur d'électrons acquiert une charge positive alors que l'atome accepteur d'électrons acquiert une charge négative.

Les ions métalliques peuvent être isolés ou regroupés en « clusters » métalliques. Les solides MOF selon l'invention peuvent par exemple êtres construits à partir de chaînes d'octaèdres ou de trimères d'octaèdre.

On entend par « cluster métallique » au sens de la présente invention un ensemble d'atomes contenant au moins deux ions métalliques liés par des liaisons ionocovalentes, soit directement par des anions, par exemple O, OH, Cl, etc., soit par le ligand organique.

De plus, les solides MOF selon l'invention peuvent se présenter sous différentes formes ou « phases » compte tenu des diverses possibilités d'organisation et de connections des ligands à l'ion métallique ou au groupement métallique.

On entend par « phase » au sens de la présente invention une composition hybride comprenant au moins un métal et au moins un ligand organique possédant une structure cristalline définie.

L'organisation spatiale cristalline des solides de la présente invention est à la base des caractéristiques et propriétés particulières de ces matériaux. Elle régit notamment la taille des pores, qui a une influence sur la surface spécifique des matériaux et sur les caractéristiques d'adsorption. Elle régit également la densité des matériaux, celle-ci étant relativement faible, la proportion de métal dans ces matériaux, la stabilité des matériaux, la rigidité et la flexibilité des structures, etc.

En outres, la taille des pores peut être ajustée par le choix de ligands L appropriés.

En particulier, le ligand L du motif de formule (I) de la présente invention peut être un ligand di-, tri-, tétra- ou hexa-carboxylate choisi dans le groupe comprenant : C₂H₂(CO₂⁻)₂ (fumarate), C₂H₄(CO₂⁻)₂ (succinate), C₃H₆(CO₂⁻)₂ (glutarate), C₄H₄(CO₂⁻)₂ (muconate), C₄H₈(CO₂⁻)₂ (adipate), C₅H₃S(CO₂⁻)₂ (2,5-thiophènedicarboxylate), C₆H₄(CO₂⁻)₂ (téréphtalate), C₆H₂N₂(CO₂⁻)₂ (2,5-pyrazine dicarboxylate), C₁₀H₆(CO₂⁻)₂ (naphtalène-2,6-dicarboxylate), C₁₂H₈(CO₂⁻)₂ (biphényle-4,4'-dicarboxylate), C₁₂H₈N₂(CO₂⁻)₂ (azobenzènedicarboxylate), C₁₂H₆Cl₂N₂(CO₂⁻)₂ (dichloroazobenzènedicarboxylate), C₁₂H₆N₂(CO₂⁻)₄ (azobenzènetetracarboxylate), C₁₂H₆N₂(OH)₂(CO₂⁻)₂ (dihydroxoazobenzènedicarboxylate), C₆H₃(CO₂⁻)₃(benzène-1,2,4-tricarboxylate), C₆H₃(CO₂⁻)₃ (benzène-1,3,5-tricarboxylate), C₂₄H₁₅(CO₂⁻)₃ (benzène-1,3,5-tribenzoate), C₄₂H₂₇(CO₂⁻)₃ (1,3,5-tris[4'-carboxy(1,1'-biphenyl-4-yl)benzene), C₆H₂(CO₂⁻)₄ (benzène-1,2,4,5-tétracarboxylate, C₁₀H₄(CO₂⁻)₄ (naphtalène-2,3,6,7-tétracarboxylate), C₁₀H₄(CO₂⁻)₄ (naphtalène-1,4,5,8-tétracarboxylate), C₁₂H₆(CO₂⁻)₄ (biphényl-3,5,3',5'-tétracarboxylate, et les analogues modifiés choisis dans le groupe comprenant le 2-aminotéréphtalate, le 2-nitrotéréphtalate, le 2-méthyltéréphtalate, le 2-chlorotéréphtalate, le 2-bromotéréphtalate, le 2,5-dihydroxotéréphtalate, le tétraflorotéréphtalate, le 2,5-dicarboxytéréphtalate, le diméthyl-4,4'-biphénydicarboxylate, le tétraméthyl-4,4'-biphénydicarboxylate, le dicarboxy-4,4'-biphénydicarboxylate.

En particulier, l'anion X du motif de formule (I) de la présente invention peut être choisi dans le groupe comprenant OH⁻, Cl⁻, F⁻, R-(COO)ₙ⁻, PF₆⁻, ClO₄⁻, avec R et n tel que défini précédemment.

En particulier, le solide MOF selon l'invention, peut comprendre un pourcentage massique de M en phase sèche de 5 à 50%, de préférence de 18 A 31%.

Le pourcentage massique (%m) est une unité de mesure utilisée en chimie et en métallurgie pour désigner la composition d'un mélange ou d'un alliage, c'est-à-dire les proportions de chaque composant dans le mélange.
1 %m d'un composant = 1g du composant pour 100 g de mélange ou encore 1 kg dudit composant pour 100 kg de mélange.

Les solides MOF de la présente invention présentent notamment l'avantage d'avoir une stabilité thermique jusqu'à une température de 350°C. Plus particulièrement, ces solides présentent une stabilité thermique de 120°C et 350°C).

En particulier, le solide MOF selon l'invention peut avoir une taille de pores de 0,4 à 6 nm, de préférence de 0,5 à 5,2 nm, et de manière plus préférée de 0,5 à 3,4 nm.

En particulier, le solide MOF selon l'invention peut avoir une surface spécifique (BET) de 5 à 6000 m²/g, de préférence de 5 à 4500 m²/g.

En particulier, le solide MOF selon l'invention peut avoir un volume poreux de 0 à 4 cm³/g, de préférence de 0,05 à 2 cm³/g.
Dans le cadre de l'invention, le volume poreux signifie le volume accessible pour les molécules de gaz.

Le solide MOF de l'invention peut avoir une capacité de charge en gaz de 0,5 à 50 mmol de gaz par gramme de solide sec.

Dans le cadre de la présente invention, la capacité de charge signifie la capacité de stockage de gaz ou la quantité de gaz adsorbé dans le matériau. La capacité de charge peut être exprimée en capacité massique (gramme/gramme) ou en capacité molaire (mol/mol) ou en d'autres ( mol/gramme, gramme/mol, etc.)

Comme indiqué précédemment, dans les solides MOF de l'invention, au moins une partie du ou des gaz base de Lewis se coordine avec M. Avantageusement, au moins 1 à 5 mmol de gaz par gramme de solide sec se coordine avec M.

La partie du ou des gaz qui ne se coordine pas avec M peut remplir avantageusement l'espace libre dans les pores.

Le solide MOF de la présente invention peut se présenter sous la forme d'une structure robuste, qui a une charpente rigide et ne se contracte que très peu lorsque les pores se vident de leur contenu qui peut être, par exemple, du solvant, de l'acide carboxylique non coordiné, etc. Il peut également se présenter sous la forme d'une structure flexible, qui peut se gonfler et se dégonfler faisant varier l'ouverture des pores en fonction de la nature des molécules adsorbées qui peuvent être, par exemple, des (solvants et/ou des gaz).

On entend par « structure rigide », au sens de la présente invention des structures qui se gonflent ou se contractent que très faiblement, c'est-à-dire avec une amplitude jusqu'à 10%.

En particulier, le solide MOF selon l'invention, peut avoir une structure rigide qui se gonfle ou se contracte avec une amplitude allant de 0 à 10%.

Les structures rigides peuvent par exemple être construites à base de chaînes ou de trimères d'octaèdres.

Selon un mode de réalisation de l'invention, le solide MOF de structure rigide peut avoir un pourcentage massique de M en phase sèche de 5 à 50%, de préférence de 18 à 31%. Avantageusement, M représentera ici le fer.

Le solide MOF de structure rigide selon l'invention peut avoir une taille de pores de 0,4 à 6 nm, en particulier de 0,5 à 5,2 nm, plus particulièrement de 0,5 à 3,4 nm.

Le solide MOF de structure rigide selon l'invention peut avoir un volume poreux de 0,5 à 4 cm³/g, en particulier de 0,05 à 2 cm³/g.

On entend par **«** structure flexible », au sens de la présente invention des structures qui se gonflent ou se contractent avec une grande amplitude, notamment avec une amplitude supérieure à 10%, de préférence supérieure à 50%.

Les structures flexibles peuvent par exemple être construites à base de chaînes ou de trimères d'octaèdres.

En particulier, le matériau MOF selon l'invention, peut avoir une structure flexible qui se gonfle ou se contracte avec une amplitude de 10% à 300%, de préférence de 50 à 300%.

Dans un mode de réalisation particulier de l'invention, le solide MOF de structure flexible peut avoir un pourcentage massique de M en phase sèche de 5 à 40%, de préférence de 18 à 31%. Avantageusement, M représentera ici le fer.

Par exemple, dans le cadre de l'invention, le solide MOF de structure flexible peut avoir une taille de pores de 0,4 à 6 nm, en particulier de 0,5 à 5,2 nm, et plus particulièrement de 0,5 à 1,6 nm.

Par exemple, le solide MOF de structure flexible selon l'invention peut avoir un volume poreux de 0 à 3 cm³/g, en particulier de 0 à 2 cm³/g.

En outre, les inventeurs ont mis en évidence expérimentalement que l'amplitude de la flexibilité dépend de la nature du ligand et du solvant utilisé, comme décrit dans la partie « Exemples » ci-dessous.

Différents matériaux MOF ont été élaborés par les inventeurs à l'Institut Lavoisier de Versailles nommées « MIL » (pour « Matériau Institut Lavoisier »). L'appellation « MIL » de ces structures est suivie d'un nombre arbitraire n donnée par les inventeurs pour identifier les différents solides.

Dans le cadre de la présente invention, les inventeurs ont mis en évidence que des solides MOF peuvent comprendre une succession tridimensionnelle de motifs répondant à la formule (I).

Dans un mode de réalisation de l'invention, les solides MOF peuvent comprendre une succession tridimensionnelle de carboxylates de fer(III) répondant à la formule (I). Ces carbolytes de fer (III) peuvent être choisis dans le groupe comprenant MIL-88, MIL-89, MIL-96, MIL-100, MIL-101 et MIL-102, et plus particulièrement dans le groupe comprenant MIL-88A, MIL-88B, MIL-88Bt, MIL-88C, MIL-88D, MIL-88E, MIL-89, MIL-96, MIL-100, MIL-101, MIL-102. Ces motifs sont présentés dans la partie « Exemples ».

En particulier, les solides MOF peuvent comprendre une succession tridimensionnelle de motifs répondant à la formule (I), choisis dans le groupe comprenant :
- Fe₃OX[C₂H₂(CO₂)₂]₃ de structure flexible, par exemple MIL-88A
- Fe₃OX[C₆H₄(CO₂)₂]₃ de structure flexible, par exemple MIL-88B
- Fe₃OX[C₁₀H₆(CO₂)₂]₃ de structure flexible, par exemple MIL-88C
- Fe₃OX[C₁₂H₈(CO₂)₂]₃ de structure flexible, par exemple MIL-88D
- Fe₃OX(C₄H₄(CO₂)₂]₃ de structure flexible, par exemple MIL-89
- Fe₁₂O(OH)₁₈(H₂O)₃[C₆H₃(CO₂)₃]₆ de structure rigide, par exemple MIL-96
- Fe₃OX[C₆H₃(CO₂)₃]₂ de structure rigide, par exemple MIL-100
- Fe₃OX[C₆H₄(CO)₂]₃ de structure rigide, par exemple MIL-101
- Fe₆O₂X₂(C₁₀H₂(CO₂)₄]₃ de structure rigide, par exemple MIL-102
dans laquelle X est tel que défini précédemment.

En outre, à partir d'un même ligand acide carboxylique L et des mêmes bases de fer (trimères), les inventeurs ont pu obtenir des matériaux MOF de même formule générale (I) mais de structures différentes. C'est par exemple le cas des solides MIL-88B et MIL-101. En effet, la différence des solides MIL-88B et MIL-101 réside dans le mode de connexions des ligands aux trimères d'octaèdre : dans le solide MIL-101, les ligands L s'assemblent sous forme de tétraèdres rigides, tandis que dans le solide MIL-88B, ils forment des bipyramides trigonales, rendant possible l'écartement entre les trimères.

Ces différents matériaux sont présentés dans la partie « Exemples » ci-dessous. Le mode d'assemblage de ces ligands peut être contrôlé lors de la synthèse par exemple par l'ajustement du pH. Par exemple, le solide MIL-88 est obtenu en milieu moins acide que le solide MIL-101 comme décrit dans la partie « Exemples » ci-dessous.

En outre, les solides MOF de l'invention peuvent rendre possible le greffage de molécules à leur surface afin de répondre aux besoins liés à la vectorisation de composés vers des cibles biologiques spécifiques et/ou à la furtivité des particules. Ceci permet ainsi d'améliorer la biodistribution des principes actifs de manière plus ciblée.

Ainsi, selon un mode de réalisation particulier, les solides MOFs selon l'invention, peuvent éventuellement comprendre à leur surface au moins un agent de surface organique. Celui-ci peut être greffé ou déposé à la surface des solides, par exemple adsorbé en surface ou lié par liaison covalente, par liaison hydrogène, par liaison de Van der Waals, par interaction électrostatique. L'agent de surface peut également être incorporé par enchevêtrement lors de la fabrication des solides MOF [10, 28].

On entend par « agent de surface » selon l'invention une molécule recouvrant en partie ou en totalité la surface du solide permettant de moduler les propriétés de surface du matériau, par exemple :
- de modifier sa biodistribution, par exemple pour éviter sa reconnaissance par le système réticulo endothélial (« furtivité »), et/ou
- de lui conférer des propriétés de bioadhésion intéressantes lors de l'administration par voies orale, oculaire, nasale, et/ou
- de lui permettre un ciblage spécifique de certains organes/tissus malades, etc.

Selon l'invention, plusieurs agents de surface peuvent être utilisés pour combiner les propriétés précitées.

Selon l'invention, l'agent de surface organique peut être choisi par exemple dans le groupe comprenant:
- un oligosaccharide comme les cyclodextrines,
- un polysaccharide comme le chitosan, le dextran, le fucoïdane, l'alginate, la pectine, l'amylose, l'amidon, la cellulose, le xylane,
- un glycosaminoglycanne comme l'acide hyaluronique, l'héparine,
- un polymère comme le polyéthylène glycol (PEG), l'alcool polyvinylique, le polyéthylène imine,
- un tensioactif comme le pluronic, la lécithine.
- des vitamines comme la biotine
- des coenzymes comme l'acide lipoique
- des anticorps ou fragments d'anticorps
- des aminoacides ou peptides.

L'agent de surface peut également être une molécule de ciblage, c'est-à-dire une molécule reconnaissant ou étant reconnue spécifiquement par une cible biologique. L'association des solides MOF de l'invention avec une molécule de ciblage permet ainsi de vectoriser les produits vers cette cible biologique, cellule, tissus, organe.

Ainsi, l'agent de surface organique peut être une molécule de ciblage choisie dans le groupe comprenant la biotine, le chitosan, l'acide lipoique, un anticorps ou fragment d'anticorps, un peptide.

Par exemple, la présence de la biotine en surface peut être mise à profit afin de coupler des ligands de manière aisée, par exemple par simple incubation. Pour cela, il est possible d'utiliser des protocoles décrits dans les publications [29, 30].

Cette méthode de modification de surface présente l'avantage de ne pas perturber le coeur des solides MOF, même lorsqu'ils contiennent du gaz, et de pouvoir se faire après la synthèse des solides MOF, et donc d'offrir une variété de recouvrements possibles.

Il est également possible d'utiliser un mélange de polymères portant des fonctions susceptibles d'interagir avec la particule (MOF) en tant qu'agent de surface afin de répondre à un cahier de charge précis, par exemple la bioadhésion, la reconnaissance spécifique, etc.

L'invention a également pour objet un procédé de préparation de solides MOF tels que définis dans la présente invention, comprenant au moins une étape réactionnelle consistant
(i) à mélanger dans un solvant polaire :
   - au moins une solution comprenant au moins un précurseur inorganique métallique se présentant sous la forme de métal M, d'un sel métallique de M ou d'un complexe de coordination comprenant un ion métallique de M
   - au moins un ligand L' comprenant un radical R comportant q groupements *-C(=O)-R³, où
      - q et R sont tels que définis précédemment,
      - * désigne le point d'attachement du groupement avec le radical R,
      - R³ est choisi dans le groupe comprenant un OH, un OY, avec Y est un cation alcalin, un halogène, un radical -OR⁴, -O-C(=O)R⁴, -NR⁴R⁴', où R⁴ et R^{4'} sont des radicaux alkyles en C₁₋₁₂,
   pour obtenir un matériau MOF ;
(ii) à activer le matériau MOF obtenu en (i) ; et
(iii) à mettre en contact le matériau. MOF obtenu à l'étape (ii) avec un gaz base de Lewis dont au moins une partie se coordine avec M, de façon à obtenir ledit solide.

M est un ion d'un métal de transition comme défini précédemment.

La préparation de matériaux MOF peut être de préférence réalisée en présence d'énergie qui peut être apportée par exemple par le chauffage, comme par exemple des conditions hydrothermales ou solvothermales, mais également par micro-ondes, par ultrasons, par broyage, par un procédé faisant intervenir un fluide supercritique, etc. Les protocoles correspondants sont ceux connus de l'homme du métier. Des exemples non limitatifs de protocoles utilisables pour les conditions hydrothermales ou solvothermales sont décrits par exemple dans [20]. Pour la synthèse par voie micro-ondes, des exemples non limitatifs de protocoles utilisables sont décrits par exemple [21, 22, 23, 24] Les conditions en présence d'un broyeur à cylindre peuvent par exemple se référer aux publications [25, 26, 27].

Les conditions hydrothermales ou solvothermales, dont les températures de réactions peuvent varier entre 0 et 220°C. sont généralement effectuées dans des récipients en verre (ou en plastique) lorsque la température est inférieure à la température d'ébullition du solvant. Lorsque la température est supérieure ou lorsque la réaction s'effectue en présence de fluor, des corps en téflon insérés dans des bombes métalliques sont employés [20].

Les solvants utilisés sont généralement polaires. Notamment les solvants suivants peuvent être utilisés : l'eau, les alcools, le diméthylformamide, le diméthylsulfoxide, l'acétonitrile, le tétrahydrofurane, le diéthylformamide, le chloroforme, le cyclohexane, l'acétone, le cyanobenzène, le dichlorométhane, le nitrobenzène, l'éthylèneglycol, le diméthylacétamide ou des mélanges de ces solvants.

Un ou plusieurs co-solvants peuvent également être ajoutés à n'importe quelle étape de la synthèse pour une meilleure solubilisation des composés du mélange. IL peut s'agir notamment d'acides monocarboxyliques, tels que l'acide acétique, l'acide formique, l'acide benzoïque, etc.

Un ou plusieurs additifs peuvent également être ajoutés au cours de la synthèse afin de moduler le pH du mélange. Ces additifs sont choisis parmi les acides minéraux ou organiques ou les bases minérales ou organiques. À titre d'exemple, l'additif peut être choisi dans le groupe comprenant : HF, HCl, HHO₃, H₂SO₄, NaOH, KOH, la lutidine, l'éthylamine, la méthylamine, l'ammoniac, l'urée, l'EDTA, la tripropylamine, pyridine.

De préférence, l'étape réactionnelle (i) peut être réalisée suivant au moins une des conditions réactionnelles suivantes :
- avec une température de réaction de 0°C à 220°C, de préférence de 50 à 150°C ;
- avec une vitesse d'agitation de 0 à 1000 rpm (ou rotation par minute), de préférence de 0 à 500 rpm ;
- avec un temps de réaction de 1 minute à 144 heures, de préférence de 1 minute à 15 heures ;
- avec un pH de 0 à 7, de préférence de 1 à 5 ;
- avec l'addition d'au moins un co-solvant au solvant, au précurseur, au ligand ou au mélange de ceux-ci, ledit co-solvant étant choisi dans le groupe comprenant l'acide acétique, l'acide formique, l'acide benzoïque ;
- en présence d'un solvant est choisi dans le groupe comprenant l'eau, les alcools R^{s}-OH où R^{s} est un radical alkyle en C₁ à C₆ linéaire ou ramifié, le diméthylformamide, diméthylsulfoxide, l'acétonitrilé, le tétrahydrofurane, le diéthylformamide, le chloroforme, le cyclohexane, l'acétone, le cyanobenzène, le dichlorométhane, le nitrobenzène, l'éthylèneglycol, le diméthylacétamide ou des mélanges de ces solvants, miscibles ou non ;
- dans un milieu supercritique, par exemple dans le CO₂ supercritique ;
- sous micro-ondes et/ou sous ultrasons ;
- dans des conditions d'électrolyse électrochimique ;
- dans des conditions utilisant un broyeur à cylindres ;
- dans un flux gazeux.

Comme indiqué, préalablement à la mise en contact du matériau MOF avec le gaz base de Lewis dans l'étape (iii), il est nécessaire que le matériau issu de l'étape (i) soit activé dans l'étape (ii).

Cette étape d'activation (ii) permet de vider les pores du matériau MOF et de les rendre accessibles pour la coordination du ou des gaz. Le vidage peut se faire, par exemple, par le départ des molécules d'eau et/ou des solvants présents dans le milieu réactionnel soit par activation sous vide primaire ou secondaire ou sous flux gazeux (hélium, azote, air_), avec ou non le chauffage du solide à une température de 25 à 300°C, en particulier de 50 à 250°C, et plus particulièrement de 100 à 250°C. Le chauffage peut être effectué pendant une période de temps entre 1 heure et 96 heures, typiquement entre 3 et 15 heures.

L'étape (ii) peut également être une étape de réduction des centres métalliques M dudit matériau MOF en ions M²⁺ dans lequel z est de 2 à 4. Selon les conditions d'activation, les centres métalliques peuvent être partiellement voire totalement réduits.

La présente décrit que les centres métalliques peuvent être constitués de métaux identiques ou différents, par exemple uniquement le fer, ou un mélange d'un ou plusieurs métaux tels que le fer en présence de titane, de manganèse ou de zirconium.

Lorsque les centres métalliques sont en partie réduits, en particulier si une partie du fer est au degré d'oxydation +II (z=2) ou une partie du manganèse au degré d'oxydation +III (z=3), une partie des molécules de gaz peuvent alors se coordiner plus fortement avec le métal par effet de rétrodonation. Par rétrodonation, on entend le transfert de la densité électronique du métal M dans une orbitale antiliante du gaz. Cela conduit à une augmentation du nombre de molécules de gaz coordinées par centre métallique. Le solide MOF final comportera alors une plus grande quantité de molécules de gaz coordinées aux ions métalliques réduits. Les solides MOF résultants posséderont alors une plus forte capacité de stockage de gaz.

Par ailleurs, la réduction partielle des centres métalliques a une influence sur la durée du relargage des gaz par le solide MOF, celle-ci augmentant de manière conséquente.

L'étape d'activation (ii) peut, en outre, être réalisée à une température élevée et sous pression réduite. Par pression réduite, on entend une pression allant de 1 à 10⁻² Pa, avantageusement de 10⁻³ à 10⁻⁵ Pa.

Par exemple, l'activation peut se faire à 50-250°C sous une pression de 1 à 10⁻² Pa, ou de 10⁻³ à 10⁻⁵ Pa.

Dans l'étape (iii) du procédé de l'invention, le matériau MOF activé dans l'étape (ii) est mis en contact avec au moins un de gaz base de Lewis. Le gaz peut être sous forme pure ou en mélange avec un gaz inerte.

La mise en contact du solide avec le gaz dans l'étape (iii) peut être réalisée à une température allant de -150 à 100 °C.

L'étape (iii) peut également être réalisée à une pression allant de 10⁴ à 10⁷ Pa.

Dans un mode de réalisation particulier, le gaz base de Lewis est de préférence le NO. L'étape (iii) peut alors être réalisée à une température allant de -100°C à +50°C. La mise en contact du NO avec le solide peut se faire à une pression allant de 10³ à 10⁶ Pa.

Selon l'application envisagée, on peut utiliser un mélange de gaz base de Lewis dans l'étape (iii) du procédé.

Le solide MOF selon l'invention peut avoir une capacité de charge en gaz de 0,5 à 50 mmol de gaz par gramme de solide sec.

Comme indiqué précédemment, au moins une partie du gaz peut se coordiner avec M. Avantageusement, le solide selon l'invention peut avoir au moins 1 à 5 mmol de gaz par gramme de solide sec coordiné avec M.

Selon un mode particulier de réalisation de l'invention, lorsque les solides MOFs de l'invention, comprennent à leur surface au moins un agent de surface, le procédé de préparation des solides MOF selon l'invention, peut comprendre en outre une étape (iv) de fixation sur ledit solide d'au moins un agent de surface organique.

Cette étape de fixation (iv) peut être réalisée pendant ou après l'étape réactionnelle (i) ou encore après l'étape d'activation (ii) et avant l'étape (iii) de mise en contact du matériau MOF avec le gaz. Des exemples sont fournis ci-dessous.

Le procédé de préparation de l'invention a l'avantage de permettre l'obtention des solides MOF cristallins purs, et homogènes, en un nombre réduit d'étapes et avec des rendements élevés. Ceci réduit la durée de synthèse et les coûts de fabrication.

Par ailleurs, les inventeurs ont également mis en évidence que les caractéristiques structurales particulières des solides de la présente invention, notamment en termes de flexibilité ou de taille des pores, en font des adsorbants de grande capacité de charge, de grande sélectivité et de grande pureté. Ils rendent donc possible l'adsorption sélective de molécules de gaz base de Lewis à intérêt biologique, comme par exemple des molécules de NO, CO ou H₂S, avec un coût énergétique favorable et un temps de relargage plus élevé. Ainsi, les travaux de recherche menés par les inventeurs leur ont permis de mettre en évidence l'intérêt les matériaux MOF selon l'invention pour l'adsorption et la libération contrôlée, en termes de quantité de gaz et de durée de libération, de gaz à intérêt biologique.

De par leur structure, les solides MOF de l'invention permettent de contrôler la durée de libération des gaz. Ainsi, avec les solides MOF de l'invention la durée de libération des gaz peuvent aller de 1 à 100 heures alors qu'avec les zéolithes de l'état de la technique, la durée de cette libération n'excède pas 10 heures sous une pression de vapeur d'eau [10, 11].

Par ailleurs, les solides MOF de l'invention ont une capacité d'adsorption et de stockage de gaz base de Lewis sensiblement supérieure à celle des zéolithes connus. Par exemple, les MOF de l'invention peuvent avoir une capacité d'adsorption du NO allant de 2,5 à 4,5 mmol/g alors qu'avec les zéolithes connus, cette capacité est inférieure à 1,5 mmol/g. Ceci permet de diminuer la quantité de matériau à utiliser pour une application donnée.

De plus, cette plus grande capacité d'adsorption permet de disposer de matériaux dans lesquels la libération des gaz peut se faire de manière continue. La libération des gaz peut également être ciblée lorsque les matériaux comprennent à leur surface au moins un agent de ciblage tel que défini précédemment.

L'invention concerne également l'utilisation de solides MOF selon l'invention, chargés en au moins un gaz base de Lewis à intérêt biologique dont au moins une partie se coordine avec M comme médicament. Ledit gaz ou le mélange de gaz peut être contenu dans les pores et au moins en partie coordiné avec M l'invention.

En effet, les solides MOF selon l'invention ont l'avantage de présenter de grandes capacités d'adsorption. En outre, ils permettent d'adsorber efficacement des molécules de gaz qui peuvent présenter des difficultés particulières, par exemple en raison de leur instabilité, leur grande réactivité, leur faible solubilité, etc.

En outre, le gaz base de Lewis peut être tout gaz donneur d'électrons ayant un intérêt biologique, comme par exemple, le NO, CO, H₂S. [10, 28].

Une utilisation particulièrement intéressante des MOF selon l'invention est dans la prévention des thromboses, notamment après l'insertion des stents, cathéters, conduits prosthétiques et autres implants médicaux dans le corps suite à une intervention chirurgicale. Dans ce type d'utilisation, les solides MOF de l'invention peuvent servir, par exemple, de revêtement pour les articles médicaux précités. En tant que revêtement, ils peuvent être utilisés seuls ou en association avec d'autres agents thérapeutiques. Les MOF étant chargés en gaz, sont capables de relarguer le gaz (ou le mélange de gaz). Ils peuvent alors constituer un moyen efficace pour prévenir la formation des thromboses au contact du corps étranger. Les solides MOF de l'invention peuvent donc être utilisés comme médicament antithrombotique. On peut également envisager de les utiliser seuls ou en association avec d'autres agents antithrombotiques connues comme par exemple le clopidogrel.

L'invention s'étend également aux articles médicaux, comme par exemple les stents, cathéters, conduits prosthétiques et autres implants médicaux dans le corps suite à une intervention chirurgicale, comprenant un solide MOF selon l'invention. Keefer L. K., Nat. Mater. 2003, VOL 2, 357 [31].

En outre, le solide MOF selon l'invention peut être chargé en au moins un gaz base de Lewis à intérêt biologique pour une utilisation dans le domaine de la cosmétique ou de la dermatologie. L'activité antibactérienne du NO peut, par exemple, être très intéressante pour des applications notamment dans le domaine des crèmes cosmétiques. Selon la teneur en eau de l'émulsion dans laquelle les particules de solides MOF de l'invention peuvent être dispersées, un relargage modulable du NO va permettre de désinfecter la peau de manière prolongée. De plus, le caractère inorganique-organique des solides MOFS va faciliter leur dispersion dans les crèmes cosmétiques.

Les effets cosmétiques ou dermatologiques bénéfiques pouvant résulter de l'utilisation d'un solide MOF selon l'invention chargé en NO sont, par exemple :
- les effets antirides, la reconstitution de la plénitude des lèvres et l'exaltation de leur couleur rouge naturelle, la stimulation de la couleur rose naturelle de la peau et l'homogénéisation du teint, par une action vasodilatation et/ou augmentation de la circulation sanguine de la peau ;
- la réduction des dommages de la peau causés par la lumière UV ;
- l'effet anti-bactérien dans le traitement de l'acné.

Comme déjà indiqué, les solides MOF de l'invention ont l'avantage de présenter des capacités de charge inattendues, encore jamais atteintes dans les matériau de l'art antérieur.

Ils présentent, en outre, l'avantage de permettre des temps de relargage modulables grâce à leur structure. En effet, la nature rigide ou flexible des structures des solides MOFs de l'invention a une influence sur la cinétique de relargage de gaz et la teneur en gaz libéré. Les solides MOF avec une structure flexible peuvent notamment permettre une libération avec une durée modulable. Ainsi, des solides MOF de structure flexible comprenant des ligands hydrophobes, vont permettre de moduler la durée de la libération du gaz.

L'utilisation des MOFs de structures flexibles tels que définis précédemment peut ainsi constituer une alternative crédible pour un relargage de gaz d'intérêt biologique comme le NO sur une plus longue durée.

Chargés en un gaz (ou un mélange de gaz) approprié, ces solides, dont les premiers tests de toxicité montrent qu'ils ne sont *a priori* pas toxiques, peuvent également être d'un grand intérêt dans l'industrie alimentaire, notamment en tant qu'agent antibactérien, par exemple pour la conservation des aliments et des préparations industrielles. En particulier, lors de la mise sous vide de préparations alimentaires, l'introduction d'une faible quantité de solide MOF selon l'invention combinée à la présence d'eau naturellement présente dans les aliments peut conduire à un relargage lent et continu de NO permettant d'inhiber la croissance bactérienne et de détruire les microorganismes.

Un autre objet de l'invention est un composition pharmaceutique, cosmétique ou dermatologique comprenant un solide MOF selon l'invention et un véhicule pharmaceutiquement ou cosmétiquement acceptable.

De plus, les solides MOF selon l'invention ont fait l'objet d'études de toxicité très positives, décrites dans la partie « Exemples ». Ils semblent également biodégradables et les études de dégradabilité sont encore en cours.

Ainsi, les solides MOF de la présente invention utilisés pour le relargage de gaz à intérêt biologique permettent de pallier les problèmes liés à la toxicité dans l'art antérieur cités précédemment.

En effet, l'utilisation de MOFs à base de métaux de basse toxicité permettent d'envisager des applications en conditions biologiques.

Dans un mode de réalisation particulier de l'invention, les solides MOF peuvent être à la fois chargés en gaz et en principe pharmaceutiquement actif.

Le principe pharmaceutiquement actif peut être contenu dans les pores. Cela permet d'obtenir un effet thérapeutique combiné.

En particulier, les solides MOF selon l'invention peut être chargée en principe pharmaceutiquement actif avec une capacité de charge de 1 à 200% en poids de solide sec, par exemple de 1 à 70% en poids de solide sec, soit près de 10 à 700 mg par gramme de solide sec.

Ainsi, l'invention s'étend à l'utilisation de tels solides comme médicament.

Lorsque les solides MOF de l'invention sont chargés à la fois en gaz et en en principe pharmaceutiquement actif, leur procédé de préparation peut comprendre, en outre, une étape (v) d'introduction dans ledit solide, d'au moins une molécules d'intérêt, qui peut être un principe pharmaceutiquement actif.

Ladite étape d'introduction peut être réalisée au cours de l'étape réactionnelle (i) ou après celle-ci de façon à obtenir un solide chargé en molécule d'intérêt.

Toute méthode connue de l'homme du métier peut être utilisée au cours de l'étape d'introduction (v).

La molécule d'intérêt peut être par exemple introduite dans le matériau MOF de la présente invention :
- par imprégnation, en immergeant le matériau dans une solution de la molécule d'intérêt ;
- par sublimation de la molécule d'intérêt, puis le gaz est adsorbé par le matériau ; ou

Dans un autre mode de réalisation, les solides MOF de l'invention, chargés en un ou en un mélange de gaz, comprend un ligand L qui possède lui-même une activité thérapeutique. Un effet thérapeutique combiné peut alors être obtenu par le relargage du (ou des) gaz et l'activité de L après dissolution du matériau.

D'autres avantages pourront apparaître à l'homme du métier à la lecture des exemples ci-dessous, illustrés par les figures annexées, donnés à titre illustratifs.

### Brève description des figures

- La figure 1 représente un diffractogramme RX du solide MIL-100(Fe).
- La figure 2 représente un isotherme d'adsorption d'azote à 77 K du solide MIL-100 (Po=1 atm).
- La figure 3 représente une analyse thermogravimétrique (sous air, avec une vitesse de chauffe de 2°C/minute) du composé MIL-100(Fe).
- La figure 4 représente le diffractogramme RX du solide MIL-101 (Fe) (λ_{Cu}=1.5406 Å).
- La figure 5 représente une analyse thermogravimétrique (sous air, avec une vitesse de chauffe de 2°C/minute) du composé MIL-101(Fe).
- La figure 6 représente des diffractogrammes RX des solides MIL-88A brut (courbe du haut) et suspendu dans l'eau (courbe du bas).
- La figure 7 représente une analyse thermogravimétrique (sous air, avec une vitesse de chauffe de 2°C/minute) du composé MIL-88A(Fe) hydraté.
- La figure 8 des diffractogrammes RX des solides MIL-88B sec (courbe (b) du bas) et hydraté (courbe (a) du haut).
- La figure 9 représente une analyse thermogravimétrique (sous air, avec une vitesse de chauffe de 2°C/minute) du composé MIL-88B hydraté.
- La figure 10 représente des diffractogrammes RX des solides MIL-89 sec (courbe a), DMF (courbe b) et hydraté (courbe c).
- La figure 11 représente un diffractogramme RX du solide MIL-88C.
- La figure 12 représente une analyse thermogravimétrique (sous air, avec une vitesse de chauffe de 2°C/minute) du composé MIL-88C brut de synthèse.
- La figure 13 représente des diffractogrammes RX des solides MIL-88D brut (courbe (b) du bas) et hydraté (courbe (a) du haut).
- La figure 14 représente une analyse thermogravimétrique (sous air, avec une vitesse de chauffe de 2°C/minute) du composé MIL-88D(Fe) hydraté.
- La figure 15 représente des diffractogrammes RX du solide MIL-88B-NO₂ brut (courbe (a) en haut) et hydraté (courbe (b) en bas).
- La figure 16 représente une analyse thermogravimétrique (sous air, avec une vitesse de chauffe de 2°C/minute) du composé MIL-88B-NO₂(Fe) après lavage et séchage.
- La figure 17 représente des diffractogrammes RX du solide MIL-88B-20H brut (courbe (c) en bas), hydraté (courbe (b) du milieu) et séché sous vide (courbe (a) en haut).
- La figure 18 représente une analyse thermogravimétrique (sous air, avec une vitesse de chauffe de 2°C/minute) du composé MIL-88B-20H(Fe) hydraté.
- La figure 19 représente des diffractogrammes RX du solide MIL-88B-NH₂ brut (courbe (b) en bas) et du solide MIL-88B-NH₂ sec (courbe (a) en haut).
- La figure 20 représente une analyse thermogravimétrique (sous air, avec une vitesse de chauffe de 2°C/minute) du composé MIL-88B-NH₂(Fe) hydraté.
- La figure 21 représente des diffractogrammes RX des solides MIL-88B-CH₃ brut (courbe (a) en haut), hydraté (courbe (b) du milieu) et solvaté avec le DMF (courbe (c) en bas).
- La figure 22 représente des diffractogrammes RX du solide MIL-88B-C1 brut (courbe (b) en bas) et hydraté (courbe (a) en haut).
- La figure 23 représente une analyse thermogravimétrique (sous air, avec une vitesse de chauffe de 2°C/minute) du composé MIL-88B-Cl(Fe) hydraté.
- La figure 24 représente des diffractogrammes RX du solide MIL-88B-4CH₃ brut (courbe (b) en bas) et hydraté (courbe (a) en haut).
- La figure 25 représente une analyse thermogravimétrique (sous air, avec une vitesse de chauffe de 2°C/minute) du composé MIL-88B-4CH₃(Fe) hydraté.
- La figure 26 représente des diffractogramines RX des solides MIL-88B-4F brut (courbe (c) en bas), hydraté (courbe (b)) et solvaté avec ETHOH (courbe (a) en haut).
- La figure 27 représente une analyse thermogravimétrique (sous air, avec une vitesse de chauffe de 2°C/minute) du composé MIL-88B-4F (Fe) hydraté.
- La figure 28 représente des diffractogrammes RX des solides MIL-88B-Br brut (courbe (b) en bas) et hydraté (courbe (a) en haut).
- La figure 29 représente une analyse thermogravimétrique (sous air, avec une vitesse de chauffe de 2°C/minute) du composé MIL-88B-Br(Fe) hydraté.
- La figure 30 représente un diffractogramme RX du solide MIL-88E(Fe).
- La figure 31 représente des diffractogrammes RX des solides MIL-88F brut (courbe (b) en bas) et hydraté (courbe (a) en haut).
- La figure 32 représente une analyse thermogravimétrique (sous air, avec une vitesse de chauffe de 2°C/minute) du composé MIL-88F (Fe) hydraté.
- La figure 33 représente des diffractogrammes RX des solides MIL-88D- 4CH₃ brut (courbe (b) en bas) et hydraté (courbe (a) en haut).
- La figure 34 représente une analyse thermogravimétrique (sous air, avec une vitesse de chauffe de 2°C/minute) du composé MIL-88D-4CH₃ (Fe) hydraté.
- La figure 35 représente des diffractogrammes RX des solides MIL-88D-2CH₃ brute (courbe (c) en bas), hydraté (courbe (b)) et mouillé (courbe (a) en haut).
- La figure 36 représente une analyse thermogravimétrique (sous air, avec une vitesse de chauffe de 2°C/minute) du composé MIL-88D-2CH₃ (Fe) brut de synthèse.
- La figure 37 représente une diffractogramme RX du solide MIL-88G brut (courbe (c) en bas), solvaté avec le DMF (courbe (b) du milieu) et solvaté avec la pyridine (courbe (a) en haut).
- La figure 38 représente une analyse thermogravimétrique (sous air, avec une vitesse de chauffe de 2°C/minute) du composé MIL-88G (Fe) brut de synthèse.
- La figure 39 représente une diffractogramme RX du solide MIL-88G-2Cl brut (courbe (b) en bas) et sec (courbe (a) en haut).
- La figure 40 représente une analyse thermogravimétrique (sous air avec une vitesse de chauffe de 2°C/minute) du composé MIL-88G-2Cl (Fe) brut de synthèse.
- La figure 41 représente des diffractogrammes RX des solides MIL-102(Fe) brut (courbe (a)) et référence MIL-102(Cr) (courbe (b)).
- La figure 42 représente une analyse thermogravimétrique (sous air) du composé MIL-102 (Fe) brut de synthèse.
- La figure 43 représente un schéma réactionnel pour l'obtention de l'acide 3,5,3',5'-tétraméthylbiphényl 4,4'-dicarboxylique.
- La figure 44 représente un schéma de réaction pour l'obtention de l'acide 3,3'-dimethylbiphenyl 4,4'-dicarboxylique.
- La figure 45 représente un cliché obtenu par MEB (Microscopie Electronique à Balayage) du solide MIL-89nano.
- La figure 46 représente un cliché obtenu par MEB (Microscopie Electronique à Balayage) du solide MIL-88Anano.
- La figure 47 représente un cliché obtenu par MEB (Microscopie Electronique à Balayage) du solide MIL-100nano.
- La figure 48 représente un cliché obtenu par MEB du solide MIL-88Btnano.
- La figure 49 représente un cliché obtenu par MEB du solide MIL-884nano.
- La figure 50 représente une quantité de sites Fer insaturés présents dans le MIL-100 Fe activé sous vide à différentes températures.
- La figure 51 représente un isotherme d'adsorption de NO à 298 K pour le carboxylates de fer MIL-100(Fe) activé à 120°C pendant une nuit.
- La figure 52 représente un profil de relargage de NO (NOᵣₑₗ en mmol/g) sous pression de vapeur du solide MIL-100(Fe) en fonction du temps t (en heure).
- La figure 53 représente (à gauche) : un isotherme d'adsorption de NO à 298 K pour le MIL-100(Fe) activé à 250°C sous vide pendant une nuit ; (à droite) : un profil de relargage de NO sous pression de vapeur du solide MIL-100(Fe) activé à 250°C sous vide pendant une nuit.
- La figure 54 représente une vue schématique du phénomène de respiration (gonflement et contraction) dans les solides MIL-88A, MIL-88B, MIL-88C, MIL-88D et MIL-89. L'amplitude de gonflement entre formes sèches (en haut) et formes ouvertes (en bas) est représentée en % en bas de la figure.
- La figure 55 représente des isothermes d'adsorption de NO à 298 K pour les carboxylates de fer MIL-88A(Fe) et MIL-88B(Fe) activés à 150°C pendant une nuit.
- La figure 56 représente des profils de relargage de NO sous pression de vapeur d'eau des solides MIL-88A(Fe) (en haut) et MIL-88B(Fe) (en bas) activés à 150°C pendant une nuit.
- La figure 57 représente, en haut, une étude de la réversibilité du gonflement du solide MIL-88A par diffraction des RX (λ-1.79Å), en bas, des diffractogrammes RX du solide MIL-88A en présence de solvants (X-1.5406Å).
- La figure 58 représente un schéma explicatif de la flexibilité dans les phases hybrides MIL-53 (a) et MIL-88 (b et c).
- La figure 59 représente la structure cristallographique du MIL-126(Fe). Les polyèdres FeO₆ sont représentés avec ou sans étoile, en indiquant les deux réseaux MIL-88D. Les atomes de carbone sont en noir.
- La figure 60 représente une diffractogramme des rayons X du solide MIL-126(Fe) (λ_{Cu}=1.5406Å).
- La figure 61 représente une analyse thermogravimétrique (sous air, avec une vitesse de chauffe de 2°C/minute) du composé MIL-126(Fe).
- La figure 62 représente des isothermes d'adsorption d'azote pour le MIL-126(Fe) (P₀=1 atmosphère).
- La figure 63 représente une diffractogramme des rayons X du 3,3',5,5'-azobenzènetétracarboxylate de fer (λ_{Cu}=1.5406Å).
- La figure 64 représente le diffractogramme RX du solide 2,5-dihydroxotéréphthalate de fer brut. La phase, de symétrie trigonal, est un isotype de celle publiée par Dietzel et al. au cobalt et nickel (groupe de espace R3) [61].
- La figure 65 représente une analyse thermogravimétrique (sous air, avec une vitesse de chauffe de 2°C/minute) du composé 3,3',5,5'-azobenzènetetracarboxylate de fer.
- La figure 66 des isothermes d'adsorption d'azote pour le 3,3',5,5'-azobenzènetetracarboxylate de fer (P₀=1 atmosphère).
- La figure 67 représente les diagrammes de DRX du matériau MIL-88A non modifié avant (MIL88A) et après l'addition d'une goutte d'eau (MIL88A + H₂O); MIL-88A modifié avec le chitosane 7% avant (MIL88AQ100) et après l'addition d'une goutte d'eau (MIL88A Q100 + H₂O) ; MIL-88A modifié avec le chitosane 2% avant (MIL88AQ25) et après l'adition d'une goutte d'eau (MIL88A Q125 + H₂O).
- La figure 68 représente l'analyse thermogravimétrique du matériau MIL-88A non modifié (MIL88A ; vert), modifié avec le chitosane 2% (MIL-88A-Q25, noir) et modifié avec le chitosane 7% (MIL-88A-Q100, rouge).
- La figure 69 représente les images de microscopie confocale du matériau MIL-100(Fe) modifié superficiellement avec du Dextrane-Fluoresceine-Biotine.
- La figure 70 représente l'évolution de la taille des particules (P en nm) de MIL-88A à surface modifiée par le polyéthylène glycol, en fonction du temps (t en min).
- La figure 71 représente les profils de relargage de NO sous pression de vapeur d'eau (courbe (a)) et dans le tampon phosphate (courbe (b)) de solide MIL-88A(Fe). La quantité de NO relarguée (NOᵣₑₗ en mmol. g⁻¹, à gauche, et ppm NO, à droite) est exprimée en fonction du temps t (en heures).
- La figure 72 représente les profils de relargage de NO sous pression de vapeur d'eau (courbe (a)) et dans le tampon phosphate (courbe (b)) de solide MIL-88B(Fe). La quantité de NO relargué (NOᵣₑₗ en mmol. g⁻¹, a gauche, et ppm NO, a droite) est exprimée en fonction du temps t (en heures).
- La figure 73 représente le diffractogramme des rayons X du solide MIL-88A-nano obtenu par synthèse microondes.
- La figure 74 représente les isothermes d'adsorption de NO à 298 K pour les carboxylates de fer MIL-88A(Fe)-nano activé à 150°C sous vide pendant une nuit. La quantité de NO (NO_{abs} en mmol.g⁻¹) adsorbé (courbe (a)) et désorbé (courbe (b)) est représentée en fonction de la pression P (en mmHg).
- La figure 75 représente les profils de relargage de NO sous pression de vapeur d'eau des solides MIL-88A(Fe) (5 microns, courbe (a)) et MIL-88A(Fe)-nano (120 nm, courbe (b)). La quantité de NO relargué (NOᵣₑₗ en mmol.g⁻¹, à gauche, et ppm NO, à droite) est exprimée en fonction du temps t (en heures).
- La figure 76 représente les isothermes d'adsorption de NO à 298 K pour les carboxylates de fer MIL-88B(Fe)-NO2 activé à 150°C sous vide pendant une nuit. La quantité de NO (NO_{abs} en mmol.g⁻¹) adsorbé (courbe (a)) et désorbé (courbe (b)) est représentée en fonction de la pression P (en mmHg).
- La figure 77 représente les isothermes d'adsorption de NO à 298 K pour les carboxylates de fer MIL-88B(Fe)-20H activé à 80°C sous vide pendant une nuit. La quantité de NO (NO_{abs} en mmol. g⁻¹) adsorbé (courbe (a)) et désorbé (courbe (b)) est représentée en fonction de la pression P (en mmHg).
- La figure 78 représente les profils de relargage de NO sous pression de vapeur d'eau (courbe (a)) et dans le tampon phosphate (courbe (b)) de solide MIL 88B (Fe)-NO2. La quantité de NO relargué (NOᵣₑₗ en mmol.g⁻¹, à gauche, et en ppm NO, a droite) est exprimée en fonction du temps t (en heures).
- La figure 79 représente les profils de relargage de NO sous pression de vapeur d'eau (courbe (a)) et dans le tampon phosphate (courbe (b)) de solide MIL-88B(Fe)-20H. La quantité de NO relargué (NOᵣₑₗ en mmol.g⁻¹, à gauche, et en ppm NO, à droite) est exprimée en fonction du temps t (en heures).
- La figure 80 représente les profils de relargage de NO sous pression de vapeur d'eau des solides MIL-100Fe (courbe (a)), MIL-88A (courbe (b)), MIL-88B (courbe (c)), MIL-88-20H (courbe (d)) et MIL-88B-NO2 (courbe (e)). La quantité de NO relargué (NOᵣₑₗ en mmol.g⁻¹) est exprimée en fonction du temps t (en heures).
- La figure 81 représente les isothermes d'adsorption de NO à 298 K pour le solide MIL-22 activé à 350°C sous vide pendant une nuit. La quantité de NO (NO_{abs} en mmol.g⁻¹) adsorbé (courbe (a)) et désorbé (courbe (b)) est représentée en fonction de la pression P (en mm Hg).
- La figure 82 représente les profils de relargage de NO par solide MIL-22 sous pression de vapeur d'eau. La quantité de NO relarguée (NOᵣₑₗ en mmol.g⁻¹ à gauche et en ppm NO à droite) est exprimée en fonction du temps t (en heures).
- La figure 83 représente l'isotherme d'adsorption de CO (CO_{ads} en mmol/g) pour le solide MIL-100(Fe) à la température de 303 K en fonction de la pression P (en bar) pour le carboxylate de fer MIL-100(Fe) activé à 100°C pendant une 12h (courbe 100°C), 250°C pendant 12h (courbe 205°C(1)) et 25°C pendant 20h 20h (courbe 250°C(2)).
- La figure 84 représente les coupes histologiques du foie sont observées par coloration de Proust (fer en bleu). Elles montrent une accumulation de fer dans le foie.
- La figure 85 représente représente les isothermes d'adsorption de NO à 298 K pour les solides CPO-27(dihydroxotéréphthalate de Co) (courbes (a) et (b)), CPO-27(2,5-dihydroxotéréphthalate de Ni ; M₂(dhtp)(H₂O).xH₂O (M = Ni or Co dhtp = acide 2,5-dihydroxytéréphthalique, x-8)) (courbes (c) et (d)), MIL-100(trimésate de Fe) (courbes (e) et (f)), HKUST(trimésate de Cu) (courbes (g) et (h)), MIL-53(téréphthalate de Al) (courbes (i) et (j)) et MIL-53(téréphthalate de Cr) (courbes (k) et (1)).
- La figure 86 représente les profils de relargage de NO sous pression de vapeur d'eau du solide CPO-27(dihydroxotéréphthalate de Co) (courbe (a)), CPO-27(2,5-dihydroxotéréphthalate de Ni ; M₂(dhtp)(H₂O).xH₂O (M = Ni or Co dhtp = acide 2,5-dihydroxytéréphthalique acid, x-8)) (courbe (b)), HRUST-1 (trimésate de Cu) (courbe (c)), MIL-53(téréphthalate de Al) (courbe (d)) et MIL-53(téréphthalate de Cr) (courbe (e)). La quantité de NO relarguée (NOᵣₑₗ en mmol.g⁻¹) est exprimée en fonction du temps t (en heures).
- La figure 87 représente les profils de relargage de NO sous pression de vapeur d'eau du solide MIL-88A (3 échantillons sous les mêmes conditions) sous forme de crème. La quantité de NO relargué (NOᵣₑₗ en mmol.g⁻¹) est exprimée en fonction du temps t (en heures).
- La figure 88 représente les profils de relargage de NO sous pression de vapeur d'eau du solide MIL-88A-nano sous forme de crème (courbe (b)) et sous forme de poudre (courbe (a)) en comparaison avec la libération dans une solution PBS (courbe (c)). La quantité de NO relarguée (NOᵣₑₗ en mmol.g⁻¹) est exprimée en fonction du temps t (en heures).
- La figure 89 représente une diffractogramme des rayons X de nicotinate de fer 2 : Groupe de space P 21/n : a = 16.422899, b = 21.423401, c = 11.048300 beta = 91.806999.

### EXEMPLES

### Exemple 1 : Synthèse et données sur les carboxylates de fer de la présente invention

Cet exemple décrit la synthèse de divers carboxylates de fer. Les solides obtenus ont ensuite été caractérisés selon les méthodes décrites ci-dessous.

L'analyse de la structure cristalline des solides carboxylates de fer a été effectuée par diffraction des rayons X (RX) à l'aide d'un diffractomètre Siemens D5000 (radiation CuKα λ_{Cu}=1,5406 Å, *mode θ-2θ)*, à température ambiante à l'air. Les diagrammes sont représentés soit en distances angulaires (2θ, en degrés °) soit en distances inter-réticulaire (d, en Å ou Angström).

La caractérisation de la porosité (surface spécifique Langmuir et volume de pore) des solides a été mesurée par l'adsorption d'azote à 77 K avec un appareil Micromeretics ASAP-2010. Les solides ont été préalablement déshydratés à 150°C sous vide primaire pendant une nuit. L'isotherme d'adsorption d'azote par les solides est donnée par une courbe représentant le volume d'azote adsorbé V (en cm³/g) en fonction du rapport de la pression P sur la pression de référence P₀=1 atm.

L'analyse thermogravimétrique a été effectuée sous atmosphère d'air à l'aide d'un appareil Modèle TA-instrument 2050. La vitesse de chauffe était de 2°C/minute. La courbe résultant de l'analyse thermogravimétrique des solides représente la perte de masse Pm (en %) en fonction de la température T (en °C).

L'analyse élémentaire des solides a été effectuée par le Service Central d'Analyse du CNRS de Vernaison :

### Analyse organique :

Microanalyses C,H,N,O,S dans les produits pharmaceutiques, les polymères et d'une façon générale les produits de synthèse, par détections coulométrique, catharométrique ou cellule infrarouge.

### Analyse inorganique:

Les principales techniques misent en oeuvre :
- ICP-AES (« Inductive Coupled Plasma - Atomic Emission Spectroscopy » ou « Spectrométrie d'émission atomique par plasma à couplage inductif ») avec différents types de détecteurs
- ICP-MS (« Inductively Coupled Plasma-Mass Spectrometry » ou « Spectrométrie de masse par Plasma à couplage inductif ») avec des spectromètres de masse quadripolaires ou à secteur magnétique
- CVAAS (« Cold-Vapor Atomic Absorption Spectroscopy » ou « Spectroscopie d'absorption atomique à vapeur froide »)
- Couplage ICP / MS / HPLC (« Inductively Coupled Plasma/Mass Spectrometry/High Performance Liquid Chromatography » ou « Chromatographie liquide Haute Performance / Spectrométrie de Masse par Plasma à Couplage Inductif »)
- Fluorescence X
- Traitements d'échantillons par voie humide, par voie sèche ou micro-ondes.

### a) MIL-100 (Fe) ou Fe₃O[C₆H₃-(CO₂)₃]₂.x.nH₂O (X=F, Cl, OH)

Le carboxylate de fer MIL-100(Fe) a été synthétisé selon deux conditions: avec et sans acide fluorhydrique.

### Conditions de synthèse avec de l'acide fluorhydrique :

56 mg de fer métallique en poudre (1 mmol, commercialisé par la société Riedel de Haen, 99%), 140 mg d'acide 1,3,5-benzenetricarboxylique (0,6 mmol, 1,3,5-BTC ; commercialisé par la société Aldrich, 99%) sont dispersés dans 5 mL d'eau distillée avec 0,6 mL d'acide nitrique 2M (commercialisé par la société vWR, 50%) et 0,4 mL d'acide fluorhydrique 5M (commercialisé par la société SDS, 50%). Le tout est placé dans un corps en Téflon de 23 ml mis dans une bombe métallique de la société PAAR et laissé pendant 6 jours à 150°C avec un palier de monté en température de 12 heures et un palier de descente en température de 24 heures. Le solide est récupéré par filtration.

Puis, le solide (200 mg) est suspendu dans 100 mL d'eau distillée à reflux sous agitation pendant 3h pour éliminer l'acide trimésique restant dans les pores. Le solide est ensuite récupéré par filtration à chaud.

### Conditions de synthèse sans acide fluorhydrique :

0,27 g de FeCl₃.6H₂O (1 mmol, commercialisé par la société Alfa Aesar, 98%), 140 mg (0,6 mmol) d'acide 1,3,5-benzenetricarboxylique (1,3,5-BTC ; commercialisé par la société Aldrich, 99%) sont dispersés dans 5 mL de l'eau distillée. Le tout est laissé dans un corps en Téflon de 23 mL mis dans une Bombe métallique PAAR pendant 3 jours à 130°C. Le solide est ensuite filtré et lavé avec de l'acétone

Le solide (200 mg) est ensuite suspendu dans 100 mL d'eau distillée à reflux sous agitation pendant 3h pour éliminer l'acide trimésique restant dans les pores. Le solide est ensuite récupéré par filtration à chaud.

### Données caractéristiques du solide carboxylate de fer MIL-100(Fe) :

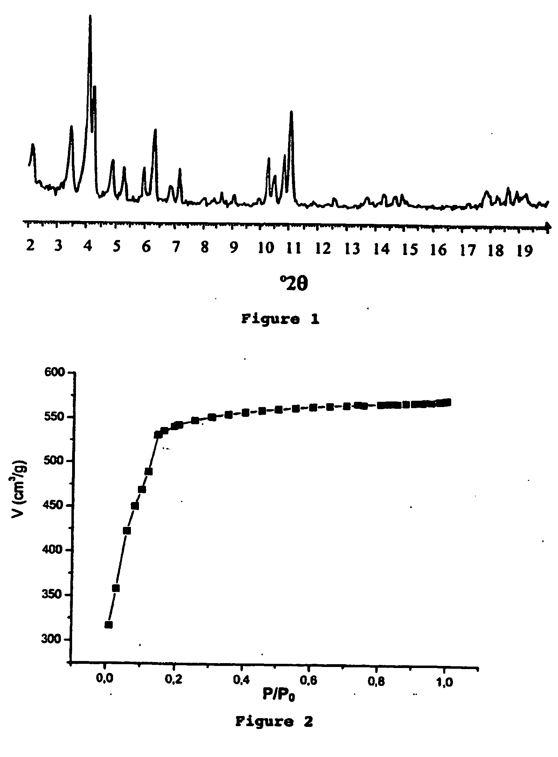

L'analyse de la structure cristalline du solide MIL-100(Fe) par diffraction des rayons X donne le diffractogramme RX représenté en figure 1.

Les caractéristiques de la structure cristalline sont les suivantes :
- le groupe d'espace est Fd-3m (n°227).
- les paramètres de maille sont : a=73,1 Å ; volume de la maille V=393000 Å³.

L'isotherme d'absorption d'azote à 77 K du solide MIL-100 (Fe) (à la pression P₀=1 atm) est donnée en figure 2. La surface (Langmuir) spécifique de ce solide est proche de 2900 m².g⁻¹.

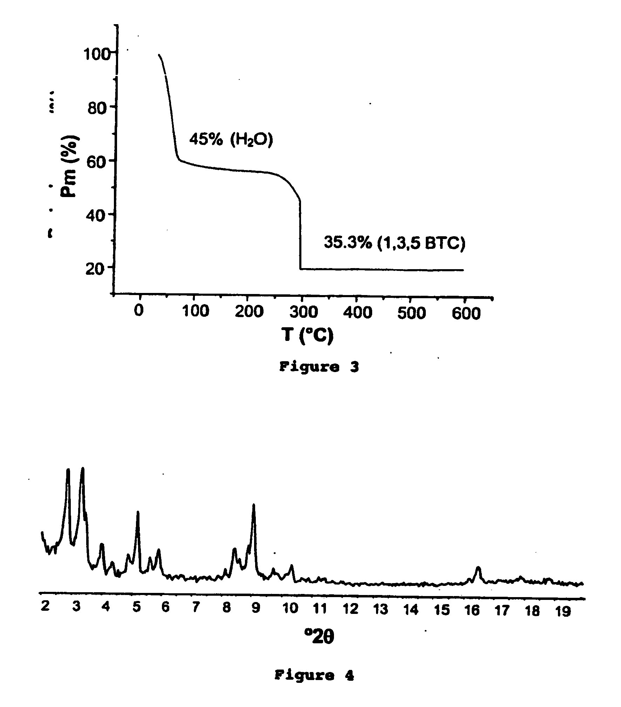

La courbe résultant de l'analyse thermogravimétrique du composé MIL-100(Fe) est donné en figure 3. Ce diagramme représente la perte de masse Pm (en %) en fonction de la température T (en °C).

Le tableau ci-dessous donne l'analyse élémentaire du solide MIL-100(Fe) ou Fe₃O[C₆H₃-(CO₂)₃]₂.X.nH₂O dans le cas où X=F.

**Tableau 1.**

| Élément (% massique) | % Fer | % Carbone | % Fluor |
|---|---|---|---|
| MIL-100(Fe) | 13,8 | 23,5 | 1,3 |

### b) MIL-101 (Fe) ou Fe₃O [C₆H₄-(CO₂)₂]₃.X.nH₂O (X=F, Cl, OH)

### Synthèse du solide MIL-101(Fe) :

0,27 g (1 mmol) de Fecl₃.6H₂O, 249 mg (1,5 mmol) d'acide 1,4-benzenedicarboxylique (1,4-BDC, commercialisé par la société Aldrich, 99%) sont dispersés dans 10 mL de diméthylformamide (DMF, commercialisé par la société Fluka, 98%). Le mélange est laissé 12 heures à 100°C dans un corps en Téflon de 23 ml mis dans une Bombe métallique PAAR. Le solide est ensuite filtré et lavé avec de l'acétone.

### Données caractéristiques du solide MIL-101(Fe) :

Le diffractogramme RX du solide MIL-101(Fe) est représenté dans la figure 4.

Les caractéristiques de la structure cristalline sont les suivantes :
- le groupe d'espace est Fd-3m (n°227).
- les paramètres de maille du solide MIL-101(Fe) à 298 K sont : a=89,0 Å ; volume de la maille V=707000 Å³.

La composition élémentaire théorique du solide sec (avec X=F) est la suivante : Fe 24,2 % ; C 41,4 % ; F 2,7 % ; H 1,7 %.

### c) MIL-88A (Fe) ou Fe₃O[C₂H₂-(CO₂)₂]₃.X.nH₂O (X=F, Cl, OH)

### Synthèse du solide MIL-88A(Fe) :

0,27 g (1 mmol) de FeCl₃.6H₂O (commercialisé par la société Alfa Aesar, 98%) et 116 mg (1 mmol) d'acide fumarique (Aldrich, 99%) sont dispersés dans 5 ml de dimethylformamide (DMF, Fluka, 98%) avec 0,4 mL NaOH 2M (Alfa Aesar, 98%). Le mélange est laissé dans un corps en Téflon de 23 ml mis dans une Bombe métallique PAAR pendant 12 heures à 100°C. Le solide est ensuite filtré et lavé avec de l'acétone.

Puis, le solide (200 mg) est mis en suspension dans 100 mL d'eau distillée sous agitation pendant 12h pour éliminer le solvant restant dans les pores. Le solide est ensuite récupéré par filtration.

### Données caractéristiques du solide MIL-88A(Fe) :

L'analyse de la structure cristalline du solide donne les caractéristiques répertoriées dans le tableau suivant :

**Tableau 2 : paramètres de maille du solide MIL-88A, sec et hydraté.**

| Phase | a (Å) | c (Å) | Volume de maille (Å³) | Taille de pore (Å) | Groupe d'espace |
|---|---|---|---|---|---|
| MIL-88A sec | 9,25 | 15,30 | 1135 | - | P-62c |
| MIL-88A hydrate (H₂O) | 13,9 | 12,66 | 2110 | 6-7 | P-62c |

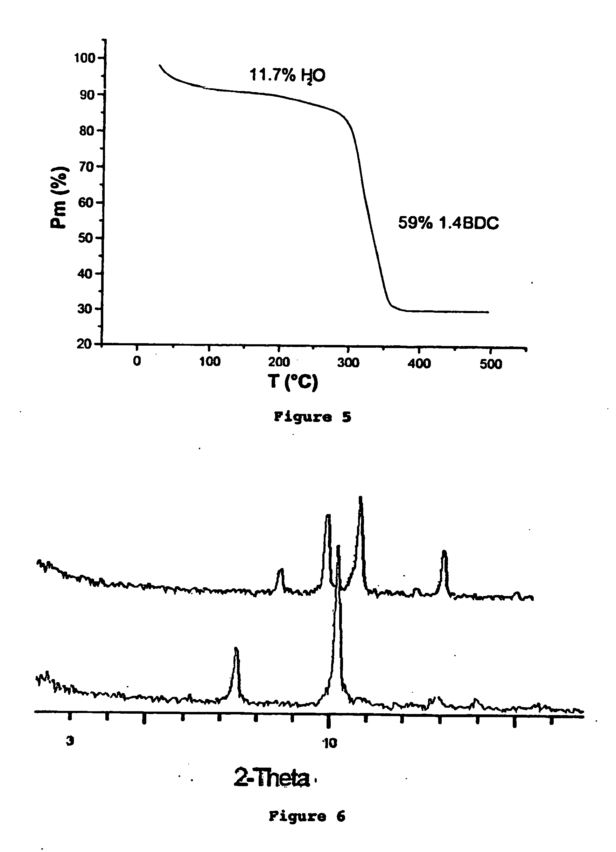

Le diffractogramme RX est donné en figure 6.

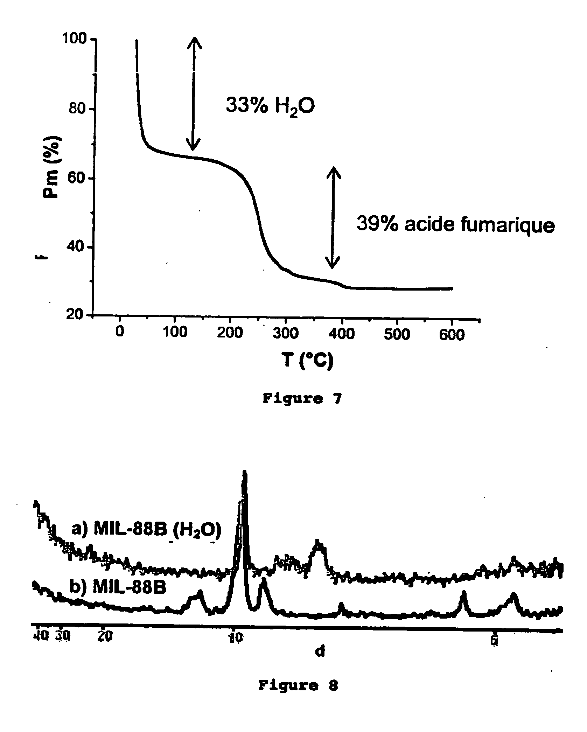

Les résultats de l'analyse thermogravimétrique du composé MIL-88A hydraté (sous air, à une vitesse de chauffe de 2°C/minute) sont représentés en figure 7. La perte de masse Pm (en %) est représentée en fonction de la température T (en °C).

Le composé MIL-88A ne présente pas de surface accessible (supérieure à 20 m²/g) à l'azote à 77 K, puisque la structure sèche possède une taille de pores trop faible pour incorporer de l'azote N₂.

L'analyse élémentaire est donnée dans le tableau ci-dessous:

**Tableau 3 :**

| **Élément (% massique)** | **% Fer** | **% Carbone** |
|---|---|---|
| MIL-88A (brut) | 21,8 | 24,0 |

### d) MIL-88B (Fe) ou Fe₃O[C₆H₄-(CO₂)₂]₃.X.nH₂O (X=F, Cl, OH)

### Synthèse du solide MIL-88B(Fe) :

0,27 g (1 mmol) de FeCl₃.6H₂O (Alfa Aesar, 98%) et 116 mg (1 mmol) d'acide 1,4-benzenedicarboxylique (Aldrich, 98%) sont dispersés dans 5 mL de diméthylformamide (DMF, Fluka, 98%) avec 0,4 mL de soude 2M (Alfa Aesar, 98%). Le mélange est laissé dans un corps en Téflon de 23 ml mis dans une Bombe métallique PAAR pendant 12 heures à 100°C. Le solide est ensuite filtré et lavé avec de l'acétone.

200 mg du solide sont mis en suspension dans 100mL d'eau distillée sous agitation pendant 12h pour éliminer le solvant restant dans les pores. Puis, le solide est récupéré par filtration.

### Données caractéristiques du solide MIL-88B(Fe) :

L'analyse de la structure cristalline du solide donne les caractéristiques répertoriées dans le tableau suivant :

**Tableau 4 : paramètres de maille du solide MIL-88B, sec et hydraté.**

| Phase | a (Å) | c (Å) | Volume de maille (Å³) | Taille de pore (Å) | Groupe d'espace |
|---|---|---|---|---|---|
| MIL-88B sec | 9,6 | 19,1 | 1500 | <3 | P-62c |
| MIL-88B hydrate (EtOH) | 15,7 | 14,0 | 3100 | 9 | P-62c |

La figure 8 représente les diffractogrammes RX du solide sec et du solide hydraté.

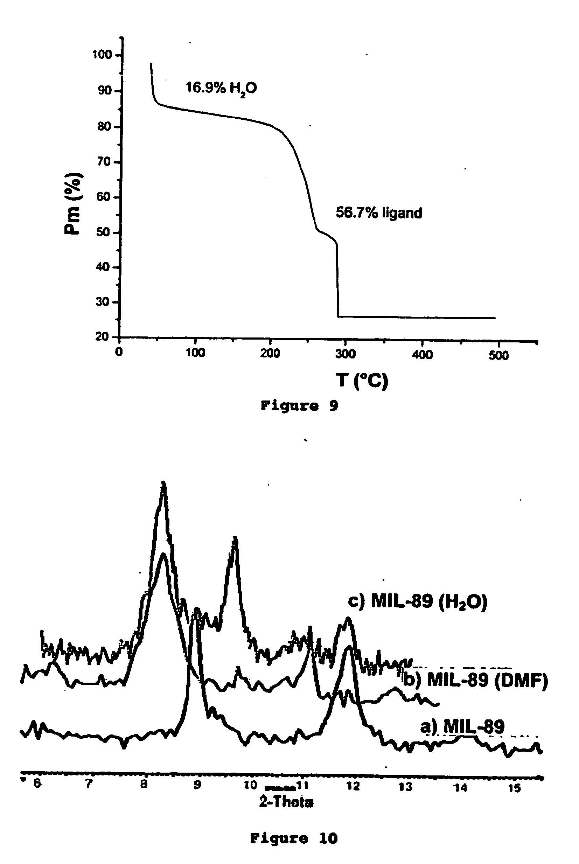

Les résultats de l'analyse thermogravimétrique du composé MIL-88B hydraté (sous air, à une vitesse de chauffe de 2°C/minute) sont représentés en figure 9. La perte de masse Pm (en %) est représentée en fonction de la température T (en °C).

Le composé MIL-88B ne présente pas de surface accessible (supérieure à 20 m²/g) à l'azote à 77 K, puisque la structure sèche possède une taille de pores trop faible pour incorporer de l'azote N₂.

### e) MIL-89 (Fe) ou Fe₃O[C₄H₄-(CO₂)₂]3.X.nH₂O (X=F, Cl, OH)

### Synthèse du solide MIL-89(Fe) :

172 mg (1 mmol) d'acétate de fer (préparé suivant la synthèse décrite par Dziobkowski et al., Inorg. Chem. 1982, 20, 671 [ref. 14]) et 150 mg (1 mmol) d'acide muconique (Fluka, 97%) sont dispersés dans 10 ml de méthanol (Fluka, 98%) avec 0,35 mL de soude 2M (Alfa Aesar, 98%). Le mélange est laissé dans un corps en Téflon de 23 ml mis dans une bombe métallique PAAR pendant 3 jours à 100°C. Le solide est ensuite filtré et lavé avec de l'acétone.

200 mg du solide sont mis en suspension dans 100mL d'eau distillée sous agitation pendant 12h pour éliminer le solvant restant dans les pores. Le solide est ensuite récupéré par filtration.

### Données caractéristiques du solide MIL-89 (Fe) :

La figure 10 représente les diffractogrammes RX a), b) et c) respectivement du solide MIL-89(Fe) sec, du solide MIL-89(Fe) solvaté avec le DMF et du solide MIL-89(Fe) hydraté.

Le composé MIL-89(Fe) ne présente pas de surface accessible (supérieure à 20 m²/g) à l'azote à 77 K, puisque la structure sèche possède une taille de pores trop faible pour incorporer de l'azote N₂.

### f) MIL-88C(Fe) ou Fe₃O [C₁₀H₆- (CO₂)₂]_{3·} X. nH₂O (X=F, Cl, OH)

### Synthèse du solide MIL-88C(Fe) :

172 mg (1 mmol) d'acétate de fer (synthétisé selon l'exemple 2) et 140 mg (1 mmol) d'acide 2,6-naphtalenedicarboxylique (Aldrich, 95%) sont dispersés dans 5 ml de diméthylformamide (DMF, Fluka, 98%). Le mélange est laissé dans un corps en Téflon de 23 ml mis dans une bombe métallique PAAR pendant 3 jours à 150°C avec un palier de monté de 12 heures et un palier de descente de 24 heures. Le solide est récupéré par filtration. Le solide est séché à 150°C sous air pendant 15 heures.

### Données caractéristiques du solide MIL-88C(Fe) :

L'analyse de la structure cristalline du solide donne les caractéristiques répertoriées dans le tableau suivant :

**Tableau 5 : paramètres de maille du solide MIL-88C, sec et solvaté.**

| Phase | a (Å) | c (Å) | Volume de maille (Å³) | Taille de pore (Å) | Groupe d'espace |
|---|---|---|---|---|---|
| MIL-88C sec | 9,9 | 23,8 | 2020 | 3 | P-62c |
| MIL-88C solvaté (Pyridine) | 18,7 | 18,8 | 5600 | 13 | P-62c |

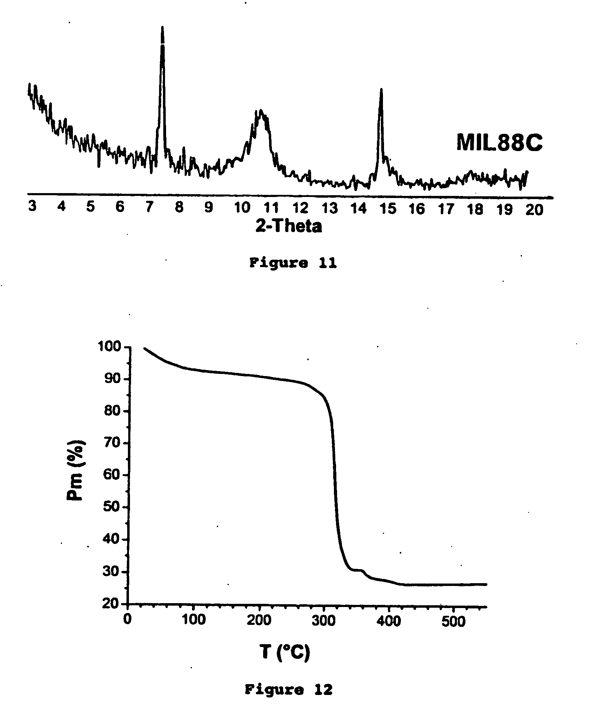

La figure 11 représente le diffractogramme RX du solide MIL-88C.

Les résultats de l'analyse thermogravimétrique du composé MIL-88C brut de synthèse (sous air, à une vitesse de chauffe de 2°C/minute) sont représentés en figure 12.

Ce composé ne présente pas de surface accessible (supérieure à 20 m²/g) à l'azote à 77 K, puisque la structure sèche possède une taille de pores trop faible pour incorporer de l'azote N₂.

### g) MIL-88D(Fe) ou Fe₃O [C₁₂H₈- (CO₂)₂]_{3·} X.nH₂O (X=F, Cl, OH)

### Synthèse du solide MIL-88D(Fe) :

270 mg (1 mmol) de FeCl₃.6H₂O (Alfa Aesar, 98%) et 140 mg (0,6 mmol) d'acide 4,4'-biphenyldicarboxylique (Fluka, 95%) sont dispersés dans 5 ml de Diméthylformamide (DMF, Aldrich, 99%). Le mélange est laissé dans un corps en Téflon de 23 ml mis dans une Bombé métallique PAAR pendant 12 heures à 100°C avec un palier de montée d'une heure et un palier de descente d'une heure. Le solide est récupéré par filtration.

Le solide est ensuite séché à 150°C sous air pendant 15 heures.

### Données caractéristiques du solide MIL-88D(Fe) :

L'analyse de la structure cristalline du solide donne les caractéristiques répertoriées dans le tableau suivant :

**Tableau 6 : paramètres de maille du solide MIL-88D, sec et solvaté (pyridine).**

| Phase | a (Å) | c (Å) | Volume de maille (Å³) | Taille de pore (Å) | Groupe d'espace |
|---|---|---|---|---|---|
| MIL-88D sec | 10,1 | 27,8 | 2480 | <3 | P-62c |
| MIL-88D solvaté (pyridine) | 20,5 | 22,4 | 8100 | 16 | P-62c |

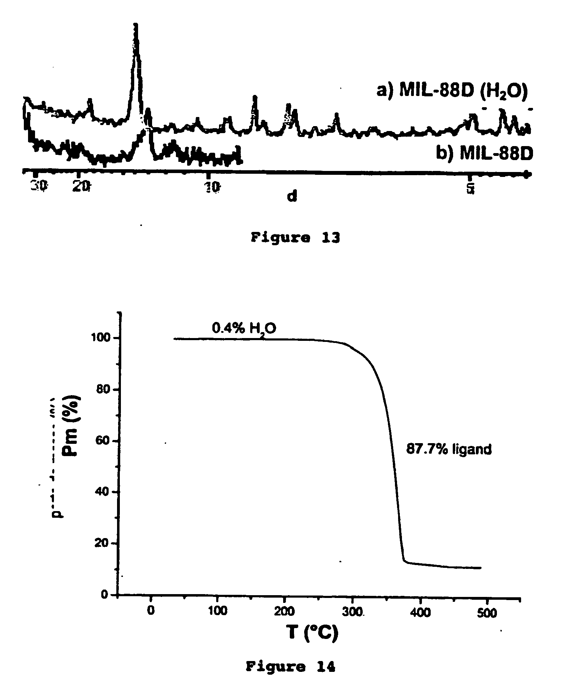

La figure 13 représente le diffractogramme RX du solide MIL-88D brut (courbe (b) du bas) et hydraté (courbe (a) du haut).

Les résultats de l'analyse thermograviatétrique du composé MIL-88D(Fe) hydraté (sous air, à une vitesse de chauffe de 2°C/minute) sont représentés en figure 14 (perte de masse Pm en fonction de la température T).

Ce composé ne présente pas de surface accessible (supérieure à 20 m²/g) à l'azote à 77 K, puisque la structure sèche possède une taille de pores trop faible pour incorporer de l'azote H₂.

### h) MIL-88B-NO₂ (Fe) ou Fe₃O[C₆H₃NO₂- (CO₂)₂]₃.X.nH₂O (X=F, C1, OH)

### Synthèse du solide MIL-88B-NO₂(Fe) :

0, 27 g (1 mmol) de FeCl₃.6H₂O (Alfa Aesar, 98%) et 211 mg (1 mmol) d'acide 2-nitrotéréphthalique (Acros, 99%) sont dispersés dans 5 ml d'eau distillée. Le mélange est laissé dans un corps en Téflon de 23 ml mis dans une bombe métallique PAAR pendant 12 heures à 100°C. Le solide est récupéré par filtration.

200 mg du solide sont mis en suspension dans 10mL d'éthanol absolu dans un corps en Téflon de 23 ml mis dans une bombe métallique PAAR pendant 12 heures à 100°C pour éliminer l'acide restant dans les pores. Le solide est ensuite récupéré par filtration et séché à 100°C.

### Données caractéristiques du solide MIL-88B-NO₂(Fe) :

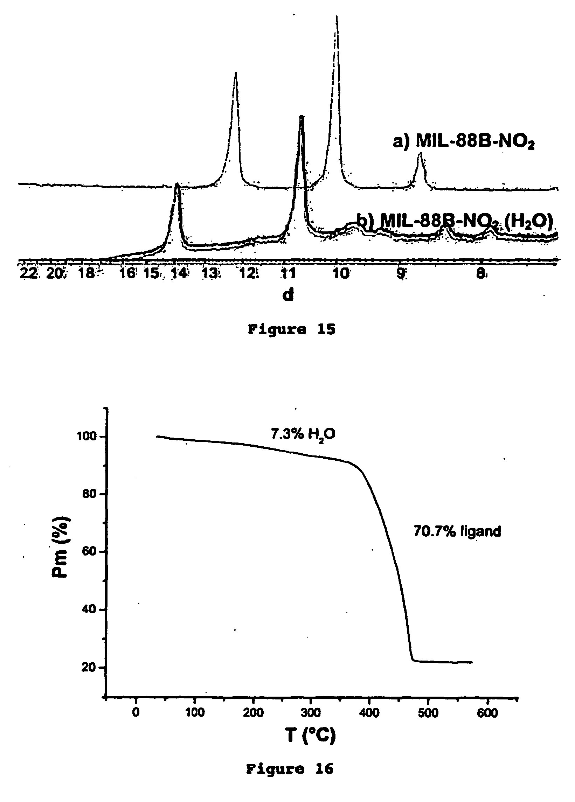

La figure 15 représente le diffractogramme RX du solide MIL-88B-NO₂ brut (courbe (a) du haut) et hydraté (courbe (b) du bas).

Les résultats de l'analyse thermogravimétrique (sous air, à une vitesse de chauffe de 2°C/minute) du composé MIL-88B-NO₂(Fe), après lavage et séchage, sont représentés en figure 16. La perte de masse Pm (en %) est représentée en fonction de la température T (en °C).

Ce composé ne présente pas de surface accessible (supérieure à 20 m²/g) à l'azote à 77 K, puisque la structure sèche possède une taille de pores trop faible pour incorporer de l'azote N₂.

L'analyse élémentaire est donnée dans le tableau ci-dessous :

**Tableau 7 :**

| Élément (% massique) | % Fer | % Carbone | % Azote |
|---|---|---|---|
| MIL-88B-NO₂ | 20,6 | 39,3 | 4,6 |

### i) MIL-88B-2OH (Fe) ou Fe₃O (C6H₂ (OH)₂- (CO₂)2]₃.X.nH₂O (X=F, Cl, OH)

### Synthèse du solide MIL-88B-20H(Fe) :

354 mg (1 mmol) de Fe(ClO₄)₃.xH₂O (Aldrich, 99%) et 198 mg (1 mmol) d'acide 2,5-dihydroxotéréphthalique (obtenu par hydrolyse de l'ester diéthylique correspondant, Aldrich 97%) sont dispersés dans 5 ml de DMF (Fluka, 98%). Le mélange est laissé dans un corps en Téflon de 23 ml mis dans une Bombe métallique PAAR pendant 12 heures à 85°C. Le solide est récupéré par filtration.

Pour éliminer l'acide restant dans les pores, le produit est calciné à 150°C sous vide pendant 15 heures.

### Données caractéristiques du solide MIL-88B-20H(Fe) :

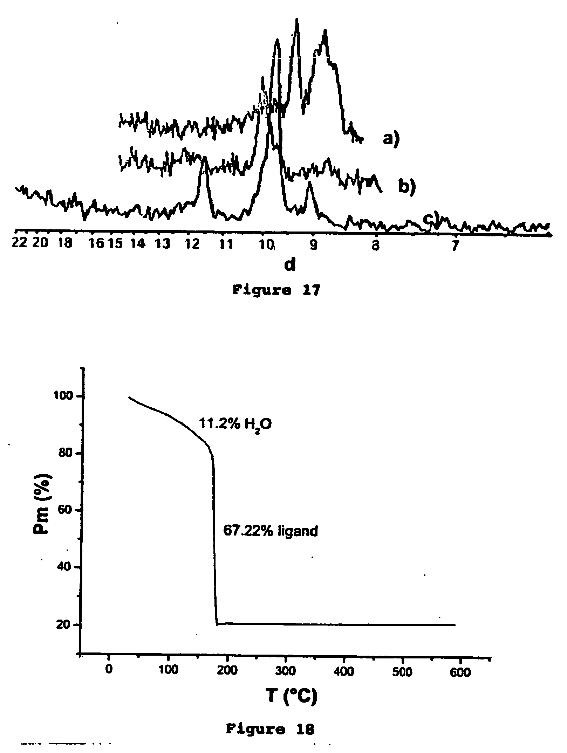

La figure 17 représente le diffractogramme RX du solide MIL-88B-20H brut (courbe (c) en bas), hydraté (courbe (b) du milieu) et séché sous vide (courbe (a) en haut).

Les résultats de l'analyse thermogravimétrique (sous air, à une vitesse de chauffe de 2°C/minute) du composé MIL-88B-20H(Fe), après lavage et séchage, sont représentés en figure 18. La perte de masse Pm (en %) est représentée en fonction de la température T (en °C).

Ce composé ne présente pas de surface accessible (supérieure à 20 m²/g) à l'azote à 77 K, puisque la structure sèche possède une taille de pores trop faible pour incorporer de l'azote N₂.

L'analyse élémentaire est donnée dans le tableau ci-dessous :

**Tableau 8 :**

| Élément (% massique) | % Fer | % Carbone |
|---|---|---|
| MIL-88B-20H | 15,4 | 36,5 |

### j) MIL-88B-NH₂ (Fe) ou Fe₃O(C₆H₃NH₂-(CO₂)₂]₃.X.nH₂O (X=F, Cl, OH)

### Synthèse du solide MIL-88B-NH₂(Fe) :.

0.27 g (1 mmol) de FeCl₃.6H₂O. (Alfa Aesar, 98%) et 180 mg (1 mmol) d'acide 2-aminotéréphthalique (Fluka, 98%) sont dispersés dans 5 ml de l'éthanol absolu. Le mélange est laissé dans un corps en Téflon de 23 ml mis dans une Bombe métallique PAAR pendant 3 jours à 100°C. Le solide est récupéré par filtration.

Pour éliminer l'acide restant dans les pores, le solide est calciné à 200°C pendant 2 jours.

### Données caractéristiques du solide MIL-88B-NH₂(Fe) :

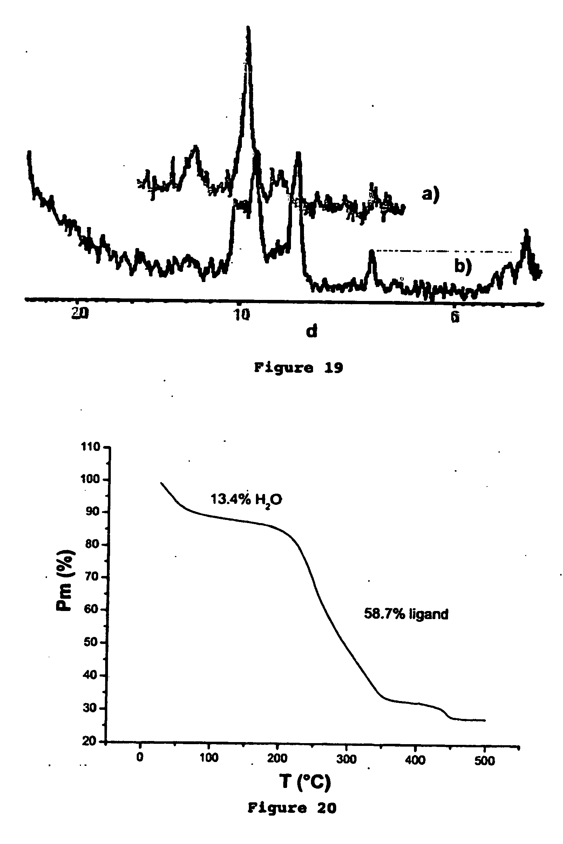

La figure 19 représente le diffractogramme RX du solide MIL-88B-NH₂ brut (courbe (b) en bas), et séché sous vide (courbe (a) en haut).

Les résultats de l'analyse thermogravimétrique (sous air, à une vitesse de chauffe de 2°C/minute) du solide MIL-88B-NH₂(Fe) hydraté sont représentés en figure 20.

Ce composé ne présente pas de surface accessible (supérieure à 20 m²/g) à l'azote à 77 K, puisque la structure sèche possède une taille de pores trop faible pour incorporer de l'azote N₂.

### k) MIL-88B-CH₃ (Fe) ou Fe₃O [C₆H₃CH₃ - (CO₂)₂]₃·X.nH₂O (X=F, Cl, OH)

### Synthèse du solide MIL-88B-CH₃(Fe) :

354 mg (1 mmol), de Fe(ClO₄)₃.xH₂O (Aldrich, 99%) et 180 mg (1 mmol) d'acide 2-méthyltéréphthalique (préparé suivant la synthèse décrite par Anzalone et al., J. Org. Chem. 1985, 50, 2128 [ref. 15]) sont dispersés dans 5 ml de méthanol (Fluka, 99%). Le mélange est laissé dans un corps en Téflon de 23 ml mis dans une Bombe métallique. PAAR pendant 3 jours à 100°C. Le solide est récupéré par filtration.

200 mg du solide sont mis en suspension dans 10mL de DMF sous agitation à température ambiante pour échanger l'acide présent dans les pores par le DMF, puis le DMF est éliminé par chauffage à 150°C sous vide pendant 12 heures.

### Données caractéristiques du solide MIL-88B-CH₃ (Fe) :

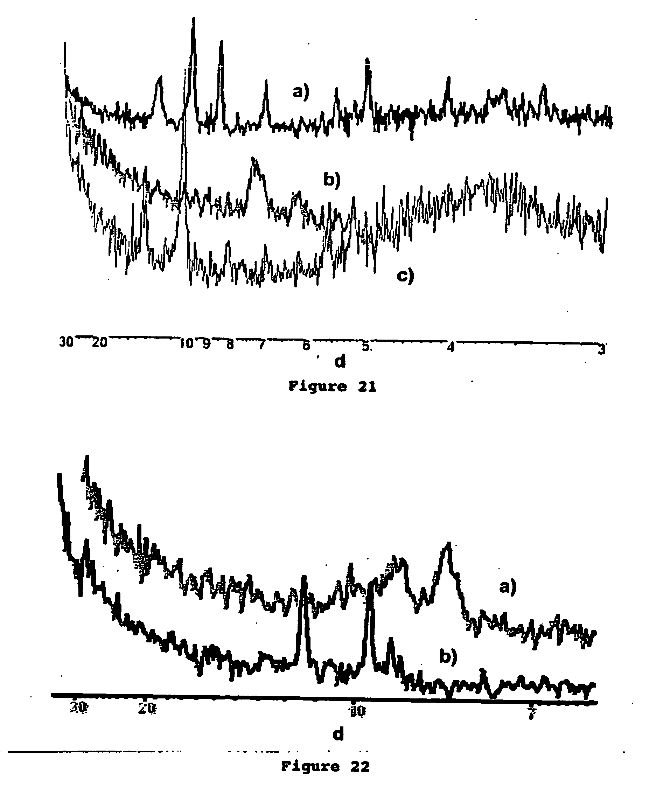

La figure 21 représente le diffractogramme RX du solide MIL-88B-CH₃ brut (courbe (a) en haut), hydraté (courbe (b) du milieu) et solvaté avec le DMF (courbe (c) en bas).

Ce composé ne présente pas de surface accessible (supérieure à 20 m²/g) à l'azote à 77 K, puisque la structure sèche possède une taille de pores trop faible pour incorporer de l'azote N₂.

### 1) MIL-88B-Cl (Fe) ou Fe₃O[C₆H₃Cl- (CO₂)₂]₃.X.nH₂O (X=F, Cl, OH)

### Synthèse du solide MIL-88B-Cl(Fe) :

354 mg (1 mmol) de Fe(ClO₄)₃.xH₂O (Aldrich, 99%) et 200 mg (1 mmol) d'acide 2-chlorotéréphthalique (synthétisé selon la synthèse A de l'exemple 3) sont dispersés dans 10 ml de DMF avec 0,1 mL de HF SM (SDS, 50%) et 0,1 mL de HCl 1M (Aldrich, 37%). Le mélange est laissé dans un corps en Téflon de 23 ml mis dans une Bombe métallique PAAR pendant 5 jours à 100°C. Le solide est récupéré par filtration.

Le solide obtenu est calciné à 150°C sous vide.

### Données caractéristiques du solide MIL-88B-Cl (Fe) :

La figure 21 représente le diffractogramme RX du solide MIL-88B-Cl brut (courbe (a) en haut), hydraté (courbe (b) du milieu) et solvaté avec le DMF (courbe (c) en bas).

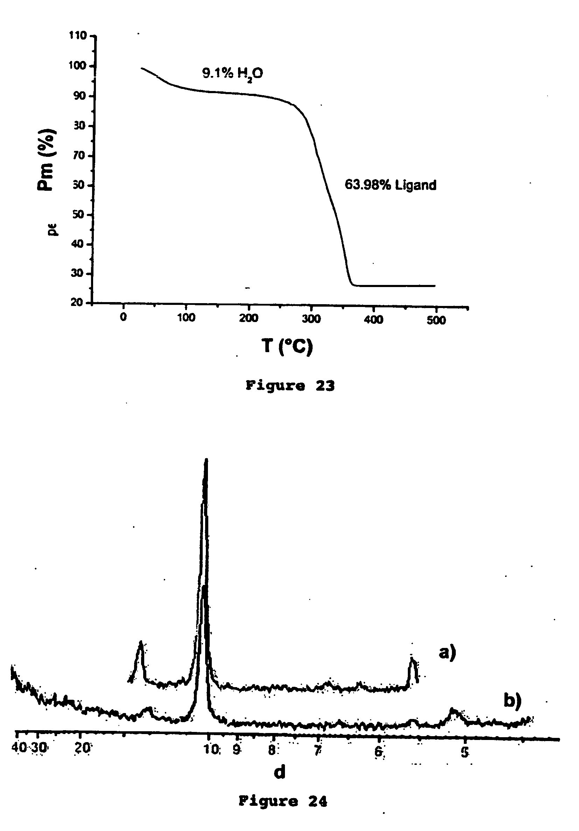

L'analyse thermogravimétrique (sous air, à une vitesse de chauffe de 2°C/minute) du solide MIL-88B-Cl(Fe) hydraté est représentée en figure 23.

Ce composé ne présente pas de surface accessible (supérieure à 20 m²/g) à l'azote à 77 K, puisque la structure sèche possède une taille de pores trop faible pour incorporer de l'azote N₂.

### m) MIL-88B-4CH₃ (Fe) ou Fe₃O [C₆ (CH₃)₄- (CO₂)₂]₃.X.nH₂O (X=F, Cl, OH)

### Synthèse du solide MIL-88B-4CH₃, (Fe) :

0,27 g (1 mmol) de FeCl₃.6H₂O (Alfa Aesar, 98%), 222 mg (1 mmol) d'acide 1,4-tétraméthyltéréphthalique (Chem Service, 95%) sont dispersés dans 10 ml de DMF (Fluka, 98%) avec 0,4 mL de soude 2M (Alfa Aesar, 98%). Le mélange est laissé dans un corps en Téflon de 23 ml ... mis dans une Bombe métallique PAAR pendant 12 heures à 100°C. Le solide est récupéré par filtration.

200 mg du solide sont mis en suspension dans 100mL d'eau sous agitation à température ambiante pendant 12 heures pour éliminer l'acide restant dans les pores. Le solide est ensuite récupéré par filtration.

### Données caractéristiques du solide MIL-88B-4CH₃ (Fe) :

La figure 24 représente le diffractogramme RX du solide brut (courbe (b) en bas) et du solide hydraté (courbe (a) en haut).

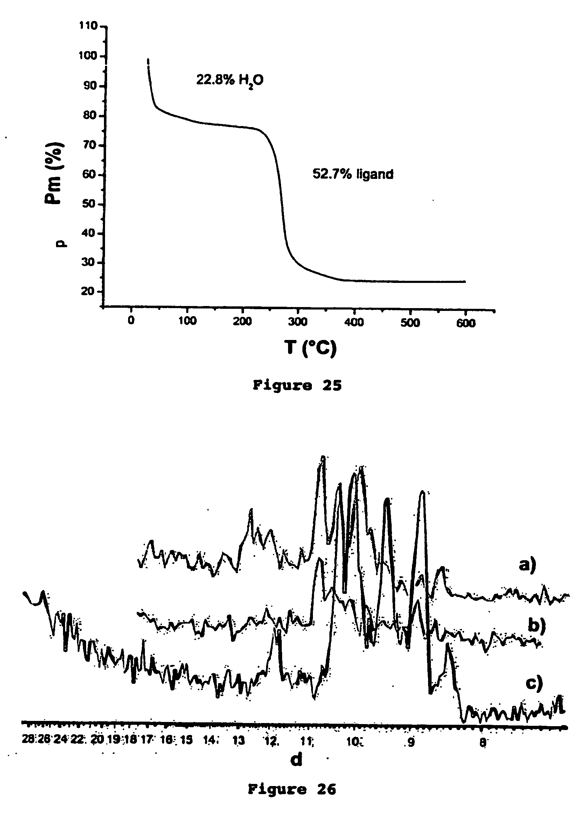

L'analyse thermogravimétrique (sous air, à une vitesse de chauffe de 2°C/minute) du solide MIL-88B-4CH₃(Fe) hydraté est représentée en figure 25.

Ce composé présente une surface accessible de l'ordre de 1200 m²/g (Langmuir) à l'azote à 77 K, puisque la structure sèche possède une taille de pores suffisante (6-7 Å) pour incorporer de l'azote N₂.

### n) MIL-88B-4F (Fe) ou Fe₃O [C₆F₄- (CO₂)₂]₃.X.nH₂O (X=F, Cl, OH)

### Synthèse du solide MIL-88B-4F (Fe) :

270 mg (1 mmol) de FeCl₃.6H₂O (Alfa Aesar, 98%) et 230 mg (1 mmol) d'acide tétrafluorotéréphthalique (Aldrich, 98%) sont dispersés dans 10 ml d'eau distillée. Le mélange est laissé dans un corps en Téflon de 23 ml mis dans une Bombe métallique PAAR pendant 12 heures à 85°C. Le solide est récupéré par filtration.

200 mg du solide sont mis en suspension dans 20mL de l'eau sous agitation à température ambiante pendant 2 heures pour éliminer l'acide restant dans les pores. Le solide est ensuite récupéré par filtration.

### Données caractéristiques du solide MIL-88B-4F (Fe) :

La figure 26 représente le diffractogramme RX du solide brut (courbe (c) en bas), du solide hydraté (courbe (b)) et du solide solvaté avec l'éthanol (courbe (a) en haut).

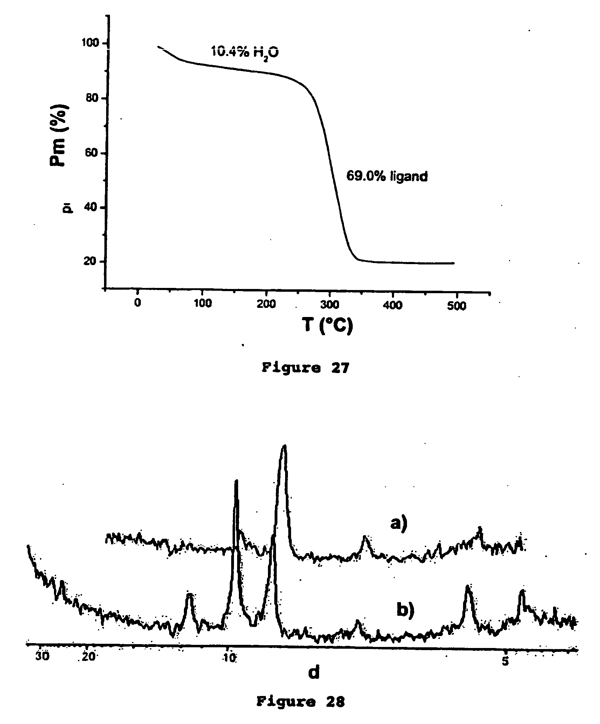

L'analyse thermogravimétrique (sous air, à une vitesse de chauffe de 2°C/minute) du solide MIL-88B-4F(Fe) hydraté est représentée en figure 27.

Ce composé ne présente pas de surface accessible (supérieure à 20 m²/g) à l'azote à 77 K, puisque la structure sèche possède une taille de pores trop faible pour incorporer de l'azote N₂.

### o) MIL-88B-Br (Fe) ou Fe₃O [C₆H₃Br- (CO₂)₂]₃.X.nH₂O (X=F, Cl, OH)

### Synthèse du solide MIL-88B-Br (Fe) :

270 mg (1 mmol) de FeCl₃.6H₂O (Alfa Aesar, 98%) et 250 mg (1 mmol) d'acide 2-bromotéréphthalique . (Fluka, 95%) sont dispersés dans 10 ml de DMF (Fluka, 98%) avec 0,2 mL d'acide fluorhydrique 5M (SDS, 50%). Le mélange est laissé dans un corps en Téflon de 23 ml mis dans une Bombe métallique PAAR pendant 12 heures à 150°C3. Le solide est récupéré par filtration.

Pour éliminer l'acide restant dans les pores, le solide est calciné à 150°C sous vide pendant 15 heures.

### Données caractéristiques du solide MIL-88B-Br (Fe) :

La figure 28 représente le diffractogramme RX du solide brut (courbe (b) en bas) et du solide hydraté (courbe (a) en haut).

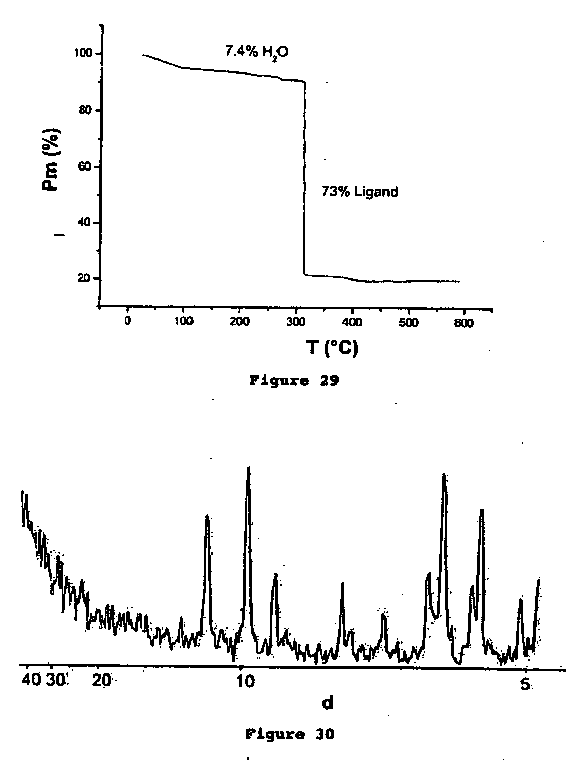

L'analyse thermogravimétrique (sous air, à une vitesse de chauffe de 2°C/minute) du solide MIL-88B-Br(Fe) hydraté est représentée en figure 29.

Ce composé ne présente pas de surface accessible (supérieure à 20 m²/g) à l'azote à 77 K, puisque la structure sèche possède une taille de pores trop faible pour incorporer de l'azote M₂.

### p) MIL-88E (Pyr) (Fe) ou Fe₃O[C₄H₃N₂-(CO₂)₂]₃.X.nH₂O (X=F, Cl, OH)

### Synthèse du solide MIL-88E (Fe) :

270 mg (1 mmol) de FeCl₃.6H₂O (Alfa. Aesar, 98%) et 204 mg (1 mmol) d'acide 2,5-pyrazinedicarboxylique (Aldrich, 98%) sont dispersés dans 5 ml de DMF (Fluka, 98%) avec 0,05 mL de HF 5M (SDS, 50%). Le mélange est laissé dans un corps en Téflon de 23 ml mis dans une Bombe métallique PAAR pendant 3, jours à 100°C. Le solide est récupéré par filtration.

### Données caractéristiques du solide MIL-88E (Fe) :

La figure 30 représente le diffractogramme RX du solide MIL-88E(Fe) brut de synthèse.

Ce composé ne présente pas de surface accessible (supérieure à 20 m²/g) à l'azote à 77 K, puisque la structure sèche possède une taille de pores trop faible pour incorporer de l'azote N₂.

### q) MIL-88F (Thio) (Fe) ou Fe₃O[C₄H₂S-(CO₂)₂]₃.X.nH₂O (X=F, Cl, OH)

### Synthèse du solide MIL-88F(Fe) :

354 mg (1 mmol) de Fe(ClO₄)₃.xH₂O (Aldrich, 99%) et 258 mg (1 mmol) d'acide 2,5-tiophenedicarboxylique (Aldrich, 99%)) sont dispersés dans 2,5 ml de DMF (Fluka, 98%) avec 0,1 mL de HF 5M (SDS, 50%). Le mélange est laissé dans un corps en Téflon de 23 ml mis dans une Bombe métallique PAAR pendant 3 jours à 100°C. Le solide est récupéré par filtration.

200 mg du solide sont mis en suspension dans 100mL d'eau sous agitation à température ambiante pendant 12 heures pour éliminer l'acide restant dans les pores. Le solide est ensuite récupéré par filtration.

### Données caractéristiques du solide MIL-88F(Fe)

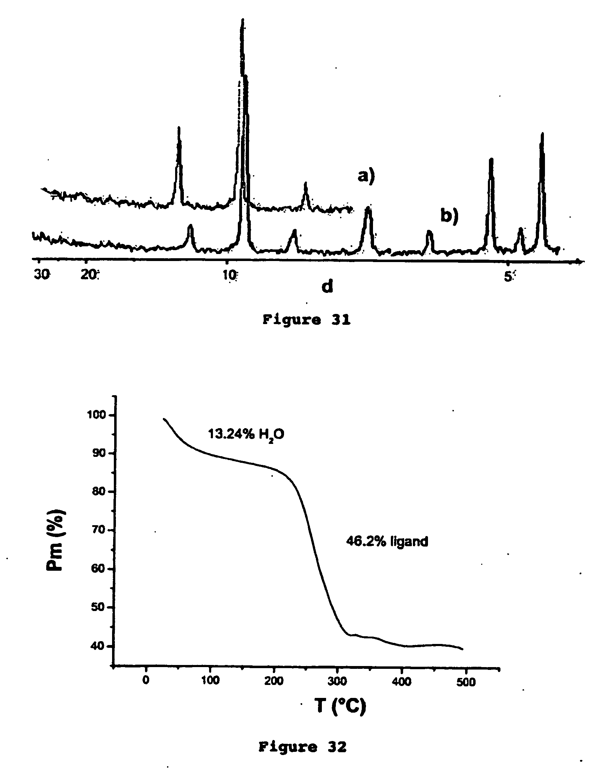

La figure 31 représente les diffractogrammes RX du solide - brut (courbe (b) en bas) et du solide hydraté (courbe (a) en haut).

L'analyse thermogravimétrique (sous air, à une vitesse de chauffe de 2°C/minute) du solide MIL-88F (Fe) hydraté est représentée en figure 32.

Ce composé ne présente pas de surface accessible (supérieure à 20 m²/g) à l'azote à 77 K, puisque la structure sèche possède une taille de pores trop faible pour incorporer de l'azote N₂.

### r) MIL-88D-4CH₃ (Fe) ou Fe₃O [C₁₂H₄ (CH₃)₄- (CO₂)₂]₃.X.nH₂O (X=F, Cl, OH)

### Synthèse du solide MIL-88D-4CH₃)(Fe) :

354 mg (1 mmol) de Fe(ClO₄)₃.xH₂O (Aldrich, 99%) et 298 mg (1 mmol) d'acide tétraméthylbiphényl-4,4'-dicarboxylique (synthétisé selon la synthèse B décrite dans l'exemple 3) sont dispersés dans 5 ml de DMF (Fluka, 98%) avec 0,2 mL de soude 2M (Alfa Aesar, 98%). Le mélange est laissé dans un corps en Téflon de 23 ml mis dans une Bombe métallique PAAR pendant 12 heures à 100°C. Le solide est récupéré par filtration.

200 mg du solide sont mis en suspension dans 10mL de DMF sous agitation à température ambiante pendant 2 heures pour échanger l'acide restant dans les pores. Le solide est ensuite récupéré par filtration, et le DMF présent dans les pores éliminé par calcination à 150°C sous vide pendant 15 heures.

### Données caractéristiques du solide MIL-88D-4CH₃(Fe)-:

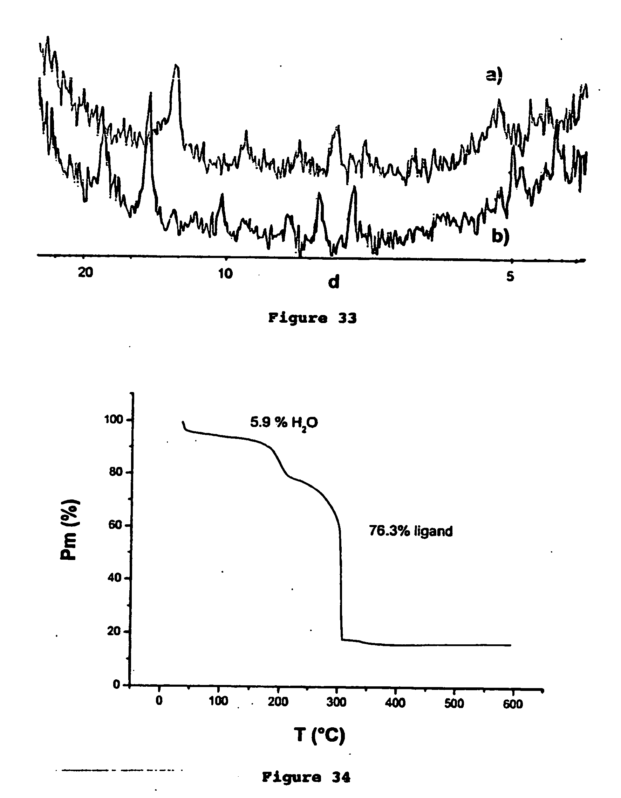

La figure 33 représente les diffractogrammes RX du solide brut (courbe, (b) en bas) et du solide hydraté (courbe (a) en haut).

L'analyse thermogravimétrique (sous air, à une vitesse de chauffe de 2°C/minute) du solide MIL-88D-4CH₃(Fe) hydraté est représentée en figure 34.

Ce composé ne présente pas de surface accessible (supérieure à 20 m²/g) à l'azote à 77 K, puisque la structure sèche possède une taille de pores trop faible pour incorporer de l'azote N₂.

### s ) MIL-88D-2CH₃ (Fe) ou Fe₃O (C₁₂H₆ (CH₃)₃- (CO₂)₂]₃.X.nH₂O (X=F, Cl, OH)

### Synthèse du solide MIL-88D-2CH₃(Fe) :

270 mg (1 mmol ) de FeCl₃.6H₂O (Alfa Aesar, 98%) et 268 mg (1 mmol) diacide diméthylbiphényl-4,4'-dicarboxylique (synthétisé selon la synthèse C décrite dans l'exemple 3) sont dispersés dans 5 ml de DMF (Fluka, 98%) avec 0,25 mL de HF 5M (SDS, 50%). Le mélange est laissé dans un corps en Téflon de 23 ml mis dans une Bombe métallique PAAR pendant 12 heures à 150°C. Le solide est récupéré par filtration.

Pour éliminer l'acide restant dans les pores, le solide est calciné à 150°C sous vide pendant 15 heures.

### Données caractéristiques du solide MIL 88D-2CH₃(Fe) :

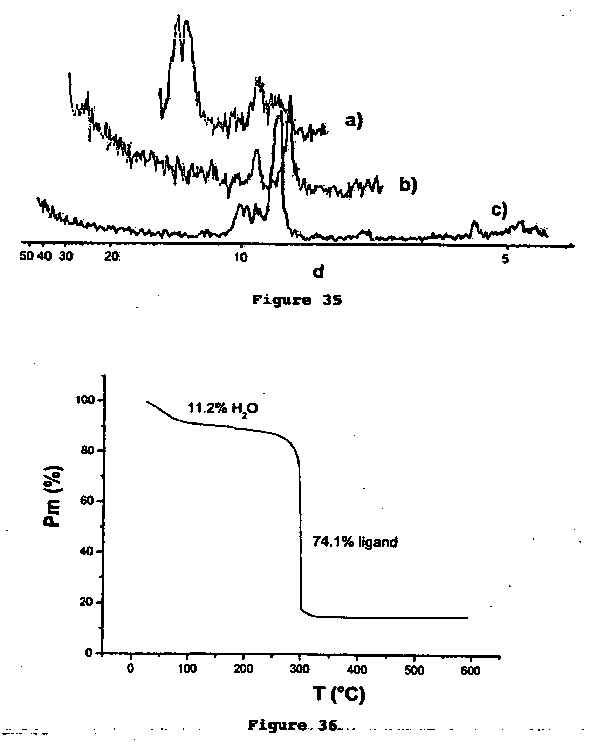

La figure 35 représente les diffractogrammes RX du solide brut (courbe (c) en bas), du solide hydraté MIL-88D-2CH₃(B₂O) (courbe (b) du milieu) et du solide en suspension dans l'eau MIL-88D-2CH₃ (goutte H₂O) (courbe (a) en haut).

L'analyse thermogravimétrique (sous air, à une vitesse de chauffe de 2°C/minute) du solide MIL-88D-2CH₃(Fe) brut est représentée en figure 36.

Ce composé ne présente pas de surface accessible (supérieure à 20 m²/g) à l'azote à 77 K, puisque la structure sèche possède une taille de pores trop faible pour incorporer de l'azote N₂.

### t) MIL-88G (AzBz) (Fe) ou Fe₃O[C₁₂H₈N₂-(CO₂)₂]₃.X.nH₂O (X=F, Cl, OH)

### Synthèse du solide MIL-88G(Fe) :

118 mg (0,33 mmol) de Fe(ClO₄)₃.xH₂O (Aldrich, 99%) et 90 mg (0,33 mmol) d'acide 4,4'-azobenzènedicarboxylique (synthétisé selon la méthode décrite par Ameerunisha et al., J. Chem. Soc. Perkin Trans.2 1995, 1679 [ref. 16]) sont dispersés dans 15 ml de DMF (Fluka, 98%). Le mélange est laissé dans un corps en Téflon de 23 ml mis dans une Bombe métallique PAAR pendant 3 jours à 150°C. Le solide est récupéré par filtration.

200 mg du solides sont mis en suspension dans 10 mL de DMF sous agitation à température ambiante pendant 2 heures pour échanger l'acide restant dans les pores. Le solide est ensuite récupéré par filtration et le DMF restant dans les pores éliminé par calcination à 150°C sous vide pendant 15 heures.

### Données caractéristiques du solide MIL-88G(Fe) :

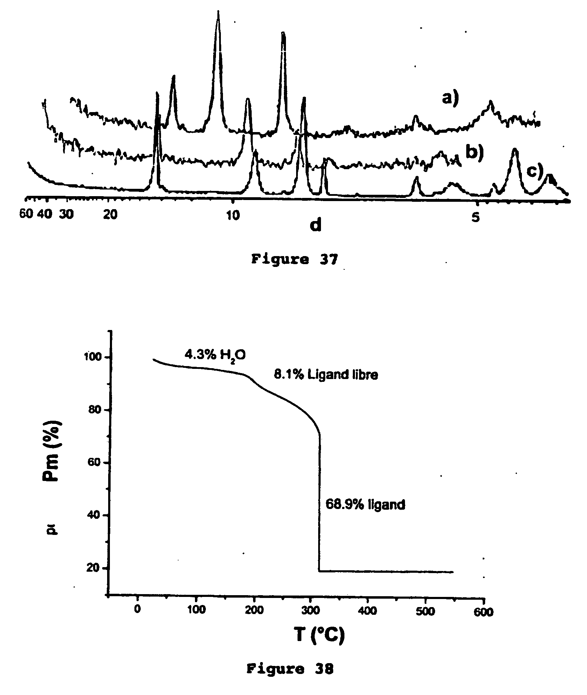

La figure 37 représente les diffractogrammes RX du solide MIL-88G brut (courbe (c) en bas), du solide solvaté avec le DMF (courbe (b) du milieu) et du solide solvaté avec la pyridine (courbe (a) en haut).

L'analyse thermogravimétrique (sous air, à une vitesse de chauffe de 2°C/minute) du solide MIL-88G(Fe) brut est représentée en figure 38.

Ce composé ne présente pas de surface accessible (supérieure à 20 m²/g) à l'azote à 77 K, puisque la structure sèche possède une taille de pores trop faible pour incorporer de l'azote N₂.

### u) MIL-88G-2Cl (AzBz-2Cl) (Fe) ou Fe₃O[C₁₂H₆N₂Cl₂-(CO₂₎₂]₃.X.nH₂O (X=F, Cl, OH)

### Synthèse du solide MIL-88G-2Cl(Fe) :

177 mg (0,5 mmol) de Fe(ClO₄)₃.xH₂O (Aldrich, 99%) et 169 mg (0,5 mmol) d'acide dichloro-4,4'-azobenzenedicarboxylique (synthétisé selon la synthèse D décrite dans l'exemple 3) sont dispersés dans 15 ml de DMF (Fluka, 98%). Le mélange est laissé dans un corps en Téflon de 23 ml mis dans une Bombe métallique PAAR pendant 12 heures à 150°C. Le solide est récupéré par filtration.

200 mg du solide sont mis en suspension dans 10mL de DMF sous agitation à température ambiante pendant 2 heures pour échanger l'acide restant dans les pores. Le solide est ensuite récupéré par filtration, et le DMF restant dans les pores est éliminé par calcination à 150°C sous vide pendant 15 heures.

### Données caractéristiques du solide MIL-88G-2Cl(Fe) :

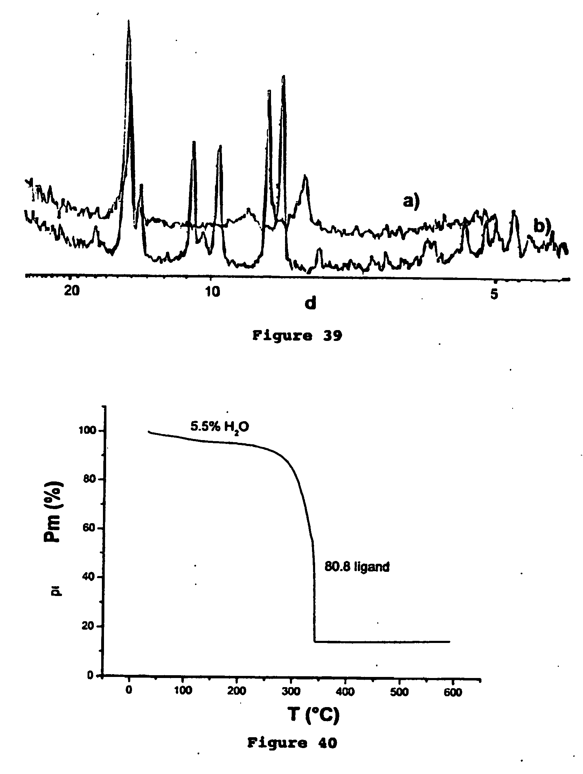

La figure 39 représente les diffractogrammes RX du solide MIL-88G-2Cl brut (courbe (b) en bas) et du solide MIL-88G-2Cl sec (courbe (a) en haut).

L'analyse thermogravimétrique (sous air, à une vitesse de chauffe de 2°C/minute) du solide MIL-88G-2Cl(Fe) brut est représentée en figure 40.

Ce composé ne présente pas de surface accessible (supérieure à 20 m²/g) à l'azote à 77 K, puisque la structure sèche possède une taille de pores trop faible pour incorporer de l'azote N₂.

### v) MIL-102(Fe) ou Fe₆O₂X₂(C₁₀H₂-(CO₂)₄]₃.nH₂O (X=F, Cl-)

### Synthèse du solide MIL-102(Fe) :

270 mg (1 mmol) de FeCl₃.6H₂O (Alfa Aesar, 98%) et 268 mg (1 mmol) d'acide 1,4,5,8-naphthalenetétracarboxylique sont dispersés dans 5 ml d'eau distillée. Le mélange est laissé dans un corps en Téflon de 23 ml mis dans une Bombe métallique PAAR pendant 15 heures à 100°C. Le solide est récupéré par filtration.

### Données caractéristiques du solide MIL-102(Fe) :

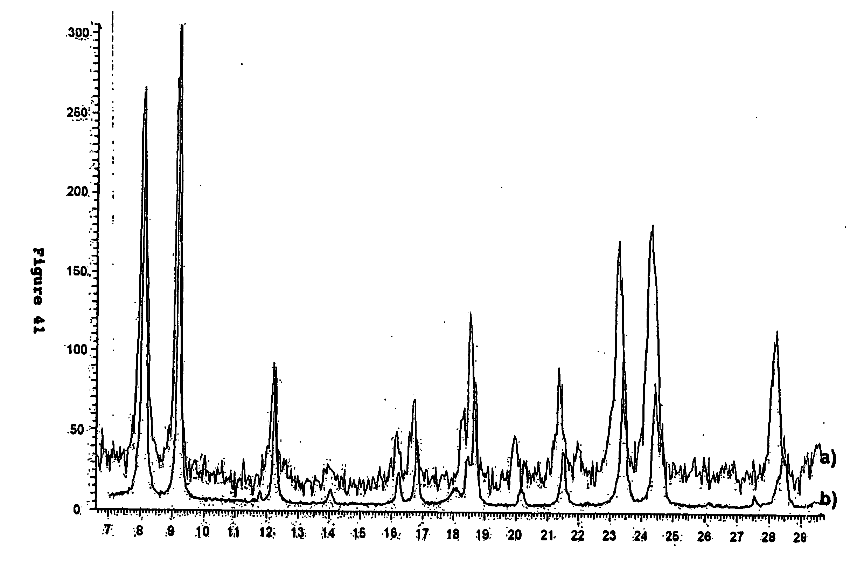

La figure 41 représente les diffractogrammes RX du solide MIL-102(Fe) brut (courbe (a)) et du solide MIL-102(Cr) (courbe (b)).

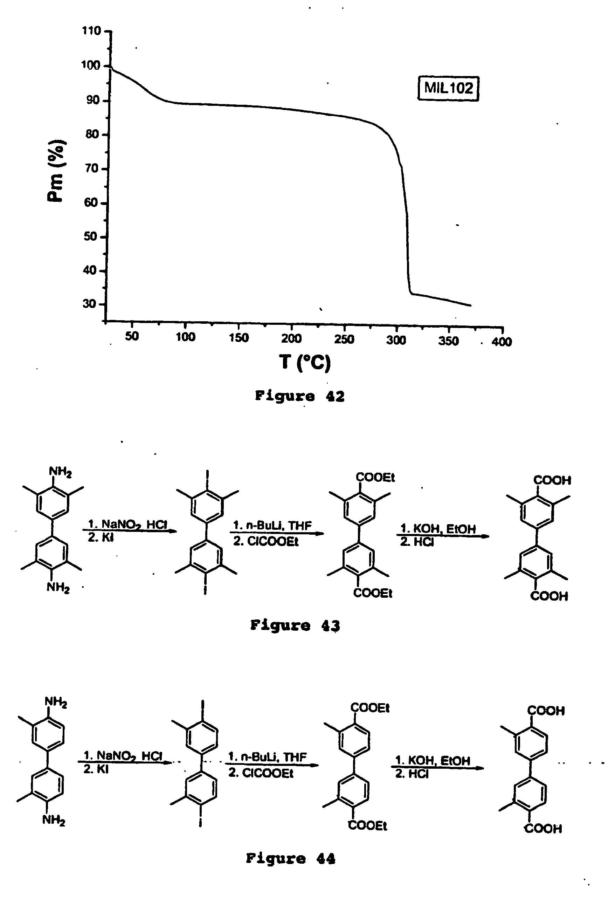

L'analyse thermogravimétrique (sous air, à une vitesse de chauffe de 2°C/min) du solide MIL-102(Fe) brut est représentée en figure 42.

Ce composé présente une surface spécifique faible (surface de Langmuir : 101 m²/g) à l'azote à 77 K.

### w) MIL-126(Fe) ou Fe₆O₂X₂[C₁₀H₂-(CO₂)₄]₃.nH₂O (X=F, Cl-) 4,4'-biphenyldicarboxylate de fer

### Synthèse du solide MIL-126(Fe) :

270 mg (1 mmol) de FeCl₃.6H₂O (Alfa Aesar, 98%) et 140 mg (0,6 mmol) d'acide 4,4'-biphenyldicarboxylique (Fluka, 95%) sont dispersés dans 5 mil de diméthylformamide (DMF, Aldrich, 99%). Le mélange est laissé dans un corps en Téflon de 23 ml mis dans une bombe métallique PAAR pendant 12 heures à 150°C avec un palier de montée de 1 heure et un palier de descende de 1 heure. Le solide est récupéré par filtration.

Le solide est ensuite séché à 150°C sous vide primaire pendant 15 heures.

### Données caractéristiques du solide MIL-126(Fe) :

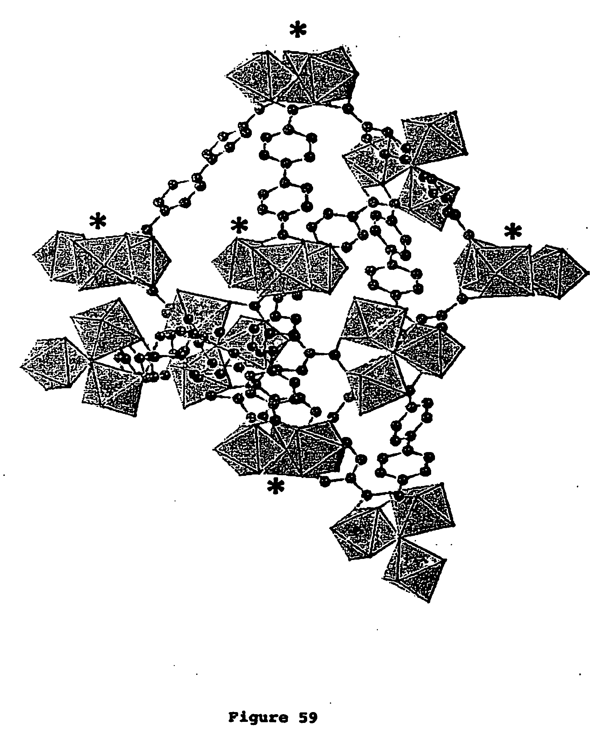

La structure cristallographique du solide MIL-126(Fe) est une forme interpénétrée de la structure MIL-88D(Fe), c'est-à-dire qu'elle possède deux sous-réseaux cristallins de type MIL-88D enchevêtrés (figure 59).

L'analyse de la structure cristalline du solide donne les caractéristiques répertoriées dans le tableau suivant.

**Tableau 9 : paramètres de maille du solide MIL-126, sec et solvaté (diméthylformamide).**

| Phase | a (Å) | C (Å) | Volume de maille (Å³) | Taille de pore (Å) | Groupe d'espace |
|---|---|---|---|---|---|
| MIL-126 sec | 19,5 | 35,3 | 13500 | 4 à 10 | P 41 21 2 |
| MIL-126 solvaté (DMF) | 21,8 | 36,1 | 17200 | 5 à 11 | P 41 21 2 |

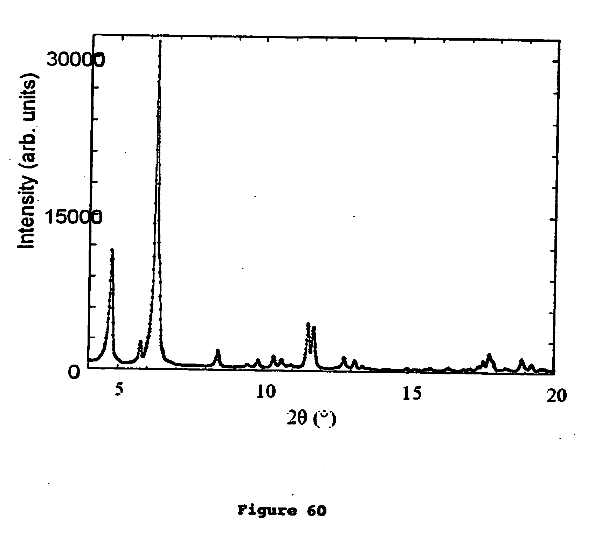

La figure 60 représente le diffractogramme RX du solide MIL-126 brut.

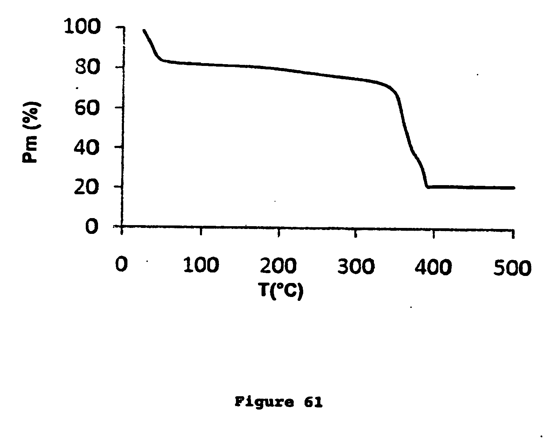

Les résultats de l'analyse thermogravimétrique du composé MIL-126(Fe) brut de synthèse (sous air, à une vitesse de chauffe de 2°C/minute) sont représentés en figure 61 (perte de masse Pm en fonction de la température T).

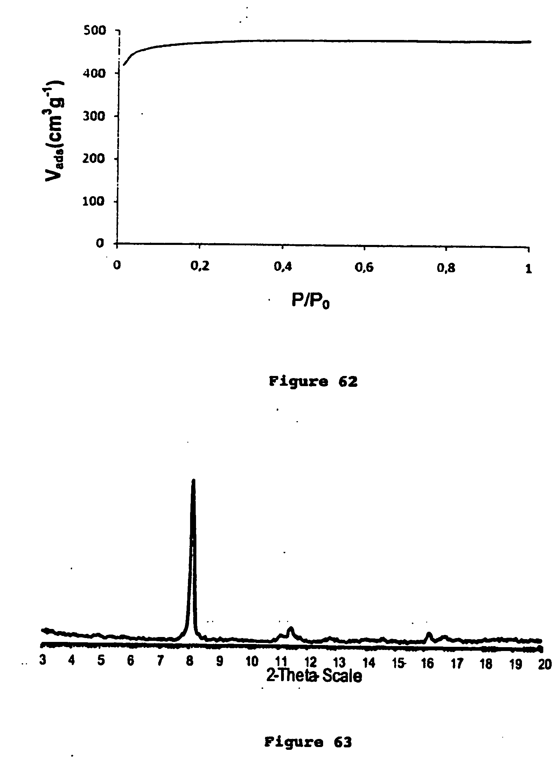

Ce composé présente une surface accessible importante(Langmuir) (supérieure à 2100 m²/g) à l'azote à 77 K (figure 62).

### y) 3,3',5,5'-azobenzènetétracarboxylate de fer ou Fe₆O₂[C₁₂H₆N₂-(CO₂)₄]_{3.}X₂.nH₂O (X=F, Cl, OH)

### Synthèse du solide 3,3',5,5'-azobenzènetétracarboxylate de fer:

118 mg (0.3 mmol) de Fe(ClO₄)₃.nH₂O (Aldrich, 98%) et 119 mg (0,6 mmol) d'acide 3,3',5,5'-azobenzènetetracarboxylique (préparé selon le mode opératoire indiqué ci-dessous dans « synthèse I ») sont dispersés dans 5 ml de diméthylformamide (DMF, Aldrich, 99%) avec l'addition de 0,1 mL d'acide fluorhydrique 5M (HF, SDS 50%). Le mélange est laissé dans un corps en Téflon de 23 ml mis dans une bombe métallique PAAR pendant 3 jours à 150°C avec un palier de montée de 1 heure. Le solide est récupéré par filtration.

Le solide est ensuite séché à 200°C sous vide primaire pendant 15 heures.

### Synthèse I: acide 3,3',5,5'-azobenzènetétracarboxylique

15g d'acide 2-nitroisophthalique (commercialisé par la société Aldrich, 98%) et 50g de soude sont placés dans 225 mL d'eau distillée, et chauffés à 50°C sous agitation. 100g de glucose (Aldrich, 96%) dissout dans 150 mL d'eau sont ajoutés. Le mélange est agité 15 minutes, puis un bullage d'air est effectué pendant 3 heures, à température ambiante (20°C). Le sel de disodium est récupéré par filtration, lavé à l'éthanol, puits dissout à nouveau dans 120 mL d'eau. De l'acide chlorhydrique (commercialisé par la société Aldrich VWR, 37%) est ajouté jusqu'à obtenir un pH égal à 1. Le solide est récupéré par filtration et séché sous vide à 90°C.

### Données caractéristiques du solide 3,3',5,5'-azobenzènetétracarboxylate de fer :

La figure 63 représente le diffractogramme RX du solide 3,3',5,5'-azobenzènetétracarboxylate de fer brut. La phase, de symétrie cubique, est un isotype de celle publie par le groupe du Professeur Eddaoudi à l'indium (groupe de espace *Pa*3) [51].

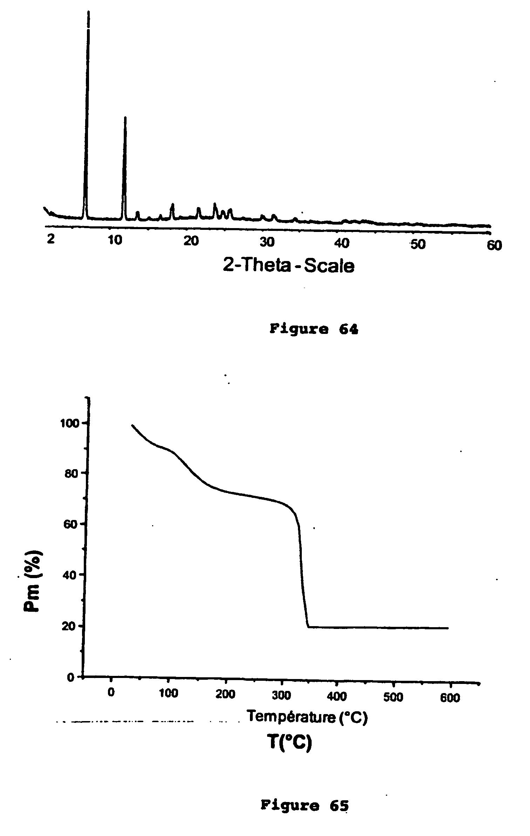

Les résultats de l'analyse thermogravimétrique du composé 3,3',5,5'-azobenzènetétracarboxylate de fer brut de synthèse (sous air, à une vitesse de chauffe de 2°C/minute) sont représentés en figure 65 (perte de masse Pm en fonction de la température T).

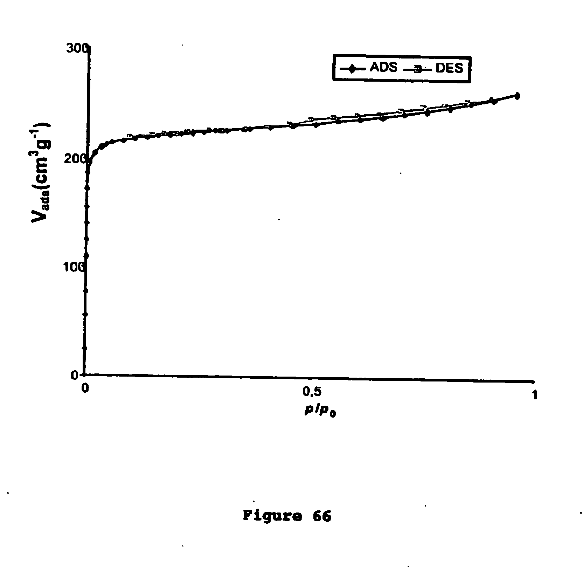

Ce composé présente une surface accessible importante (Langmuir) (supérieure à 1200 m²/g) à l'azote à 77 K (figure 66).

### z ) 2,5-dihydroxoterephthalate de fer ou Fe₂(₂OC-C₆H₂(OH)₂-CO₂) (H₂O). xH₂O

### Synthèse du solide 2,5-dihydroxotéréphthalate de fer:

270 mg (1 mmol) de FeClO₃.6H₂O (Alfa Aesar, 98%) et 200 mg (1 mmol) d'acide 2,5-dihydroxotéréphthalique (obtenu par hydrolyse de l'ester diéthylique correspondant, Aldrich 97%) sont dispersés dans 5 ml de diméthylformamide (DMF, Aldrich, 99%). Le mélange est laissé dans un corps en Téflon de 23 ml mis dans une bombe métallique PAAR pendant 3 jours à 150°C avec un palier de montée de 12 heures et un palier de refroidissement de 24 heures. Le solide est récupéré par filtration.

Le solide est ensuite séché à 150°C sous vide primaire pendant 15 heures.

### Données caractéristiques du solide 2,5-dihydroxoterephthalate de fer :

La figure 64 représente le diffractogramme RX du solide 2,5-dihydroxotéréphthalate de fer brut. La phase, de symétrie trigonal, est un isotype de celle publiée par Dietzel et al. au cobalt et nickel (groupe de espace *R3*) [61].

### Exemple 2 : Synthèse de l'acétate de fer(III)

L'acétate de fer(III), utilisé dans les exemples ci-dessous pour la synthèse des matériaux MOF selon l'invention, est synthétisé selon le protocole suivant. Cette synthèse peut se référer à la publication de Dziobkowski et al., Inorg. Chem., 1982, 21, 671 [ref. 14].

6,72 g de poudre de fer métallique (Riedel-de Haën, 99%), 64 mL d'eau déionisée et 33,6 mL d'acide perchlorique à 70 % dans l' eau (Riedel-de Haën) sont mélangés sous agitation magnétique et chauffés à 50°C pendant 3 heures. Après arrêt du chauffage, la solution est agitée pendant 12 heures. Le fer métallique résiduel est éliminé par décantation suivie d'un changement de récipient. 20,6 mL de solution de peroxyde d'hydrogène dans l'eau (commercialisé par la société Alfa Aesar, 35%) sont ajoutés goutte à goutte sous agitation, le tout étant maintenu dans un bain de glace à 0°C. On ajoute 19,7 g d'acétate de sodium (Aldrich, 99%) à la solution bleue sous agitation en maintenant la solution à 0-5°C. On laisse la solution s'évaporer pendant 3 jours sous la hotte dans un cristallisoir (Volume=0,5 1) en verre. Finalement, les cristaux rouges d'acétate de fer sont récupérés par filtration et lavés très rapidement avec de l'eau déionisée glacée. Les cristaux sont ensuite séchés sous atmosphère d'air.

### Exemple 3 : Synthèse des ligands

### a) Synthèse A : synthèse de l'acide chlorotéréphtalique

6 g (0,043 mol) de chloroxylene (commercialisé par la société Aldrich, > 99%), 16 mL d'acide nitrique (commercialisé par la société VWR, 70%) et 60 mL d'eau distillée sont introduits dans un corps en téflon de 120 mL. Celui-ci est placé dans une bombe métallique PAAR, chauffé à 170°C pendant 12h. Le produit est récupéré par filtration, puis lavé abondamment à l'eau distillée. Un rendement de 75% est obtenu.

RMN ¹H (300 MHz, d6-DMSO) : δ (ppm) : 7, 86 (d, J = 7,8 Hz), 7,93 (dd, J = 7,8; 1,2 Hz), 7,96 (d, J = 1,2 Hz)

### b) Synthèse B : synthèse de l'acide 3,5,3',5'-tétraméthylbiphényl-4,4'-dicarboxylique

Le schéma réactionnel de cette synthèse est représenté en figure 43.

### 1^{ère} étape :

10,2 g de tétraméthylbenzidine (98%, Alfa Aesar) sont mis en suspension dans 39 mL d'acide chlorhydrique concentré (37%, commercialisé par la société Aldrich) à 0°C. La diazotation a été effectuée par addition d'une solution du nitrite de sodium (6 g dans 50 mL d'eau). Après 15 min d'agitation à 0°C, une solution d'iodure de potassium (70 g dans 200 mL d'eau) a été ajoutée lentement à la solution violette résultante. Une fois l'addition terminée, le mélange est agité pendant 2 heures à température ambiante. La suspension noire résultante est filtrée pour récupérer un précipité noir, lavé à l'eau. Le solide est mis en suspension dans le dichlorométhane (DCM, 98%, commercialisé par la société SDS) et une solution saturée de thiosulfate de sodium est ajoutée, causant la décoloration. Après 1 heure d'agitation, la phase organique est décantée et la phase aqueuse est extraite au DCM. La phase organique est séchée sur sulfate de sodium, puis évaporée pour donner l'intermédiaire diiodé sous forme d'un solide grisâtre. L'élution avec du pentane pur sur colonne de silice (commercialisé par la société SDS) permet d'obtenir le mélangé des composés monoiodé et diiodé. Le mélange de ces derniers a été directement employé dans l'étape suivante. 2^{e} étape :

7,2 g du composé iodé brut est solubilisé dans 100 mL de tétrahydrofurane (THF, distillé sur sodium). Après refroidissement à -78°C, 35 mL de n-butyllithium dans le cyclohexane (2,5 M, commercialisé par la société Aldrich, sont ajoutés. La solution est laissée revenir à température ambiante, une suspension blanche apparaît après 2 heures. Elle est à nouveau refroidie à -78°C et 12 mL d'éthylchloroformate sont ajoutés. Le mélange est laissé à température ambiante, une solution jaune limpide est obtenu après 1 heure. Une partition entre l'eau et le dichlorométhane, suivie d'une extraction au dichlorométhane donne le diester brut. Celui-ci est purifié par chromatographie sur gel de silice, avec pour éluant un mélange Et₂O/Pentane : 1/9, (rapport frontal : R_{f} = 0,3). 6,3 g de diester sont obtenus sous la forme d'un solide incolore (rendement de 42 % à partir de la benzidine).

Caractérisation du diester obtenu : RMN ¹H (300 MHz, CDCl₃): δ (ppm) : 1,29 (t, *J* = 7,2 Hz, 6H), 2,29 (s, 139); 4,31 (q, *J* = 7,2 Hz, 4H); 7,12 (s, 4H). RMN ¹³C (75 MHz, CDCl₃): δ (ppm) : 14,3 (CH₃), 19,9 (CH₃), 61,0 (CH₂), 126,5 (CH), 133,2 (Cq), 135,5 (Cq), 141,4 (Cq), 169,8 (Cq).

### 3^{e} étape :

Finalement, le diester est saponifié avec 9,7 g d'hydroxyde de potassium (commercialisé par la société VWR) dans 100 mL d'éthanol 95% (commercialisé par la société SDS), au reflux pendant 5 jours. La solution est concentrée sous vide et le produit est solubilisé dans l'eau. De l'acide chlorhydrique concentré est ajouté jusqu'à pH 1, et un précipité blanc est formé. Il est récupéré par filtration, lavé à l'eau et séché. 5,3 g de diacide sont ainsi obtenus sous forme de solide blanc (rendement quantitatif).

### c) Synthèse C : synthèse de l'acide 3,3'-diméthylbiphényl 4,4'-dicarboxylique

Le schéma réactionnel de cette synthèse est représenté en figure 44.

La même procédure que celle décrite pour la synthèse B a été utilisée en partant de 12,1 g de diméthylbenzidine. A l'issue de la 1^{ère} étape, 18,4 g de 3,3'-diméthyl-4,4'-diiodo-biphényle sont obtenus (rendement: 74%).

Caractérisation du composé diiodé obtenu :

RMN ¹H (300 MHz, CDCl₃): δ (ppm) : 2,54 (s, 6H), 7,10 (dd, *J* = 2,2 et 8,1 Hz, 28), 7,46 (d, *J* = 2,2 Hz, 2H), 7,90 (d, *J* = 8,1 Hz, 2H). RMN ¹³C (75 MHz, CDCl₃): δ (ppm) : 28,3 (CH₃), 100,3 (Cq), 126,0 (CH), 128,3 (CH), 139,4 (CH), 140,4 (Cq), 141,9 (Cq).

A l'issue des 2° et 3° étapes, 6,9 g d'acide 3,3'-dimethyl-biphenyl-4,4'-dicarboxylique sont obtenus à partir des 18,4 g de composé diiodé.

### Caractérisation des composés obtenus :

Le diester obtenu l'issue de la 2^{e} étape et le diacide obtenu à l'issue de la 3^{e} étape ont des signatures spectroscopiques identiques à celles décrites dans la littérature (Shiotani Akinori, Z. Naturforsch. 1994, 49, 12, 1731-1736 [ref. 31]).

### d) Synthèse D : synthèse de l'acide 3,3'-dichloro4,4'-azobenzènedicarboxylique

15g d'acide *o*-chlorobenzoïque (commercialisé par la société Aldrich, 98%) et 50g de soude sont placés dans 225 mL d'eau distillée, et chauffés à 50°C sous agitation. 100g de glucose (Aldrich, 96%) dissout dans 150 mL d'eau sont ajoutés. Le mélange est agité 15 minutes, puis un bullage d'air est effectué pendant 3 heures, à température ambiante. Le sel de disodium est récupéré par filtration, lavé à l'éthanol, puis dissout à nouveau dans 120 mL d'eau. De l'acide chlorhydrique (commercialisé par la société Aldrich VWR, 37%) est ajouté jusqu'à obtenir un pH égal à 1. Le solide est récupéré par filtration et séché sous vide à 90°C.

### e) Synthèse E : acide 3,5,3',5'-azobenzènetétracarboxylique

19 g d'acide 5-nitroisophthalique (Aldrich, 98%) et 50 g de soude sont placés dans 250 mL d'eau distillée, et chauffés à 50°C sous agitation. Une solution de 100g de glucose (Aldrich, 96%) dissous dans 150 mL d'eau est ajoutée. Le mélange est agité 15 minutes, puis un bullage d'air est effectué pendant 3 h à température ambiante. Le sel de disodium résultant est récupéré par filtration, dissout dans 300 mL d'eau à température ambiante. De l'acide chlorhydrique (VWR, 37%) est ajouté jusqu'à obtenir un pH égal à 1. Le solide est récupéré par filtration et séché sous vide à 90°C.

### Exemple 4 : Synthése de nanoparticules de MOF selon l'invention

### a) MIL-89nano

La synthèse du MIL-89nano est effectuée à partir de l'acétate de fer (1 mmol; synthétisé selon la synthèse décrite dans l'exemple 2) et l'acide muconique (1 mmol; Fluka, 97%) en 5 mL d'éthanol (Riedel-de Haën, 99,8%) avec l'adition de 0,25 mL de hydroxyde de sodium 2M (Alfa Aesar, 98%) dans un autoclave (Paar bomb) à 100°C pendant 12h. Après refroidissement du container, le produit est récupéré par centrifugation à 5000 rpm (rotation par minute) pendant 10 minutes.

200 mg du solide sont mis en suspension dans 100 mL d'eau distillée sous agitation pendant 15h pour éliminer le solvant restant dans les pores. Puis, le solide est récupéré par centrifugation à 5000 rpm pendant 10 minutes.

La taille de particule mesurée par diffusion de lumière est de 400 nm (nanomètres).

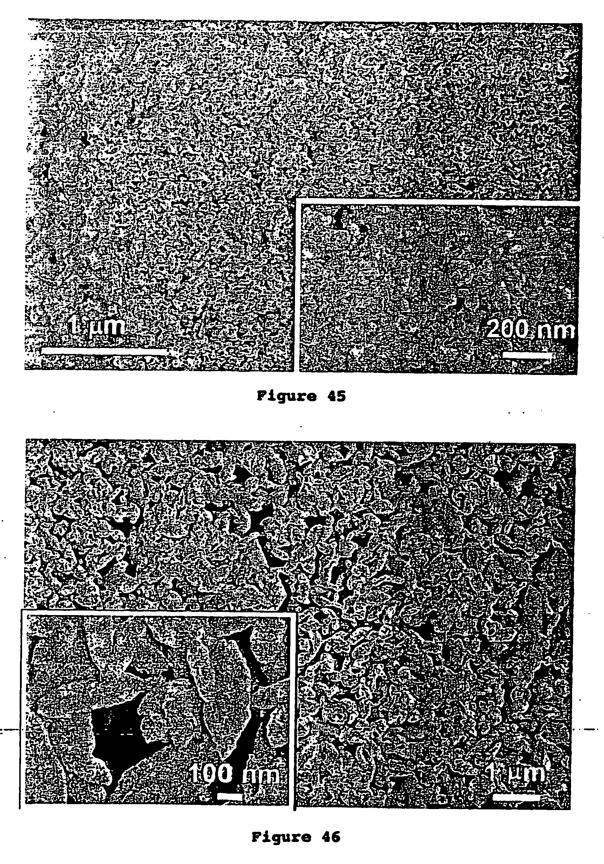

La figure 45 représente un cliché obtenu par microscopie électronique à balayage (MEB) du solide MIL-89nano.

Les nanoparticules montrent une morphologie arrondie et légèrement allongée, avec une taille de particules très homogène de 50-100 nm (Figure 45).

### b) MIL-88Anano

Pour l'obtention du matériau MIL-88Anano, du FeCl₃.6H₂O (1 mmol; Alfa Aesar, 98%) et de l'acide fumarique (1 mmol; Acros, 99%) sont dispersés dans 15 mL d'éthanol (Riedel-de Haën, 99,8%). Puis, à cette solution est ajouté 1 mL d'acide acétique (Aldrich, 99.7%). La solution est placée dans un flacon en verre et chauffée à 65°C pendant 2 heures. Le solide est récupéré par centrifugation à 5000rpm pendant 10 minutes.

200 mg du solide sont mis en suspension dans 100mL d'eau distillée sous agitation pendant 15h pour éliminer le solvant restant dans les pores. Puis, le solide est récupéré par centrifugation à 5000 rpm pendant 10 minutes.

La taille de particule mesurée par diffusion de lumière est de 250 nm.

La microscopie électronique à balayage (MEB) du solide MIL-88Anano est représentée en figure 46.

Les images de MEB (Figure 46) montrent des particules allongées avec des arêtes. Il existe deux tailles de particules : environ 500 nm et 150 nm.

### c) MIL-100nano

La synthèse du MIL-100nano est effectuée en mélangeant du Fecl₃.6H₂O (1 mmol; Alfa Aesar, 98%) et de l'acide 1,3,5-benzenetricarboxylique (1,3,5-BTC ; 1 mmol; Aldrich, 95%) dans 3 mL d'eau distillée. Le mélange est placé dans une bombe PAAR à 100°C pendant 12h. Le produit est récupéré par centrifugation à 5000rpm (10 minutes).

200 mg du solide sont mis en suspension dans 100 mL d'eau distillée sous agitation et reflux pendant 3h pour éliminer l'acide restant dans les pores. Puis, le solide est récupéré par centrifugation à 5000rpm (10 minutes). La taille de particule mesurée par diffusion de lumière est 536 nm.

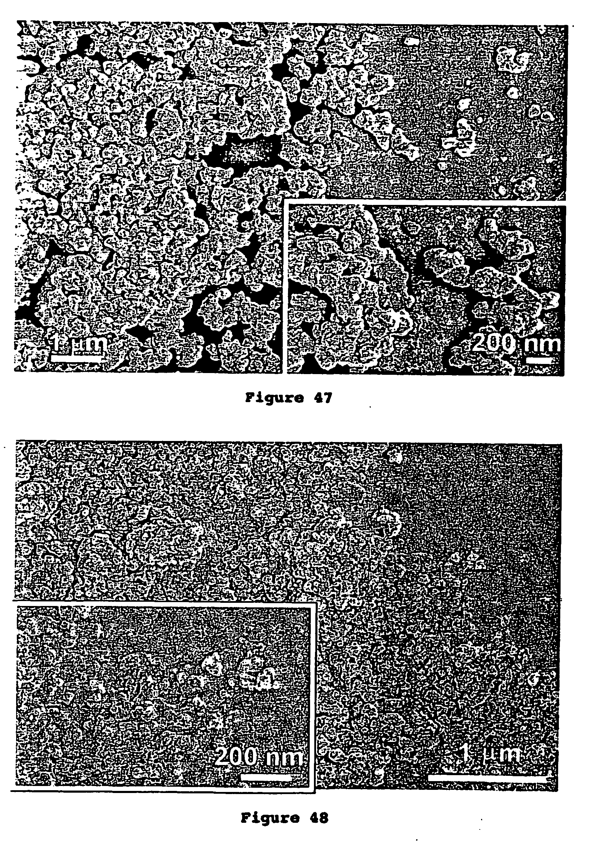

La microscopie électronique à balayage (MEB) du solide MIL-100nano est représentée en figure 47.

On peut observer sur les images de MEB une grande agrégation particulaire (Figure 47). Les nanoparticules sont plutôt sphériques, mais la taille reste difficile à déterminer compte tenu de la grande agrégation. On peut estimer une taille de 40-600 nm.

### d) MIL-101nano

Pour l'obtention du solide MIL-101nano une solution de FeCl₃.6H₂O (1 mmol; Alfa Aesar, 98%) et d'acide 1,4-benzenedicarboxylique (1,5 mmol; 1,4-BDC Aldrich, 98%) dans 10 mL de diméthylformamide (Fluka, 98%) est placée dans une bombe Paar et chauffé à 100°C pendant 15 heures. Le solide est récupéré par centrifugation à 5000rpm (10 minutes).

Pour éliminer l'acide qui reste dans les pores, le produit est chauffé à 200°C sous vide pendant 1 jour. Le produit est conservé sous vide ou atmosphère inerte compte tenu de sa faible stabilité à l'air.

La taille de particule est mesurée par diffusion de lumière est 310 nm.

### e) MIL-88Btnano

Le solide MIL-88Btnano est synthétisé à partir d'une solution de Fecl₃.6H₂O (1 mmol; Alfa Aesar, 98%) et d'acide 1,4-benzenetetramethyldicarboxylique (1 mmol; Chem Service) dans 10 mL de diméthylformamide (Fluka, 98%) avec 0,4 mL de NaOH 2M. Cette solution est placée dans une bombe Paar et chauffée à 100°C pendant 2 heures. Puis, le container est refroidi avec de l'eau froide, le produit est récupéré par centrifugation à 5000rpm (10 minutes).

200 mg du solide sont mis suspension dans 100mL d'eau distillée sous agitation pendant 15h pour éliminer le solvant restant dans les pores. Le solide est ensuite récupéré par centrifugation à 5000rpm (10 minutes).

La mesure de la taille de particule par diffusion de lumière montre deux populations de nanoparticules de 50 et 140 nm.

Les nanoparticules du solide MIL-88Btnano ont une morphologie sphérique avec une taille d'environ 50 nm. Seulement une fraction minoritaire possède une taille d'environ 200 nm. On peut y observer aussi des agglomérats de petites particules.

La microscopie électronique à balayage (MEB) du solide MIL-88Btnano est représentée en figure 48.

### f) MIL-88Bnano

Le solide MIL-88Bnano est synthétisé à partir d'une solution d'acétate de fer (1 mmol; synthétisé selon la synthèse décrite dans l'exemple 2) et d'acide 1,4-benzenedicarboxylique (1 mmol; 1,4-BDC Aldrich, 98%) dans 5 mL de méthanol (Aldrich, 99%). Cette solution est placée dans une bombe Paar et chauffé à 100°C pendant 2 heures. Le container est ensuite refroidit avec de l'eau froide, le produit est récupéré par centrifugation à 5000rpm (10 minutes).

200 mg du solide sont mis en suspension dans 100 mL d'eau distillée sous agitation à reflux pendant 15h pour échanger le solvant qui reste dans les pores. Puis, le solide est récupéré par centrifugation à 5000rpm (10 minutes).

La mesure de la taille de particule par diffusion de lumière montre une distribution bimodale de nanoparticules de 156 et 498 nm.

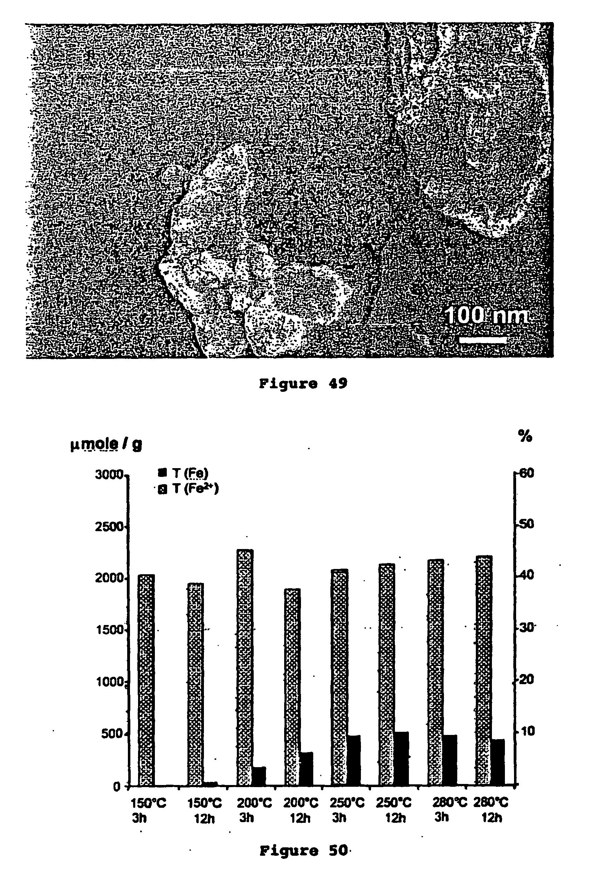

La microscopie électronique à balayage (MEB) du solide MIL-88Bnano est représentée en figure 49.

La morphologie des particules est très irrégulière, avec une taille de 300 nm.

La détermination de la taille de particule par diffusion de lumière a été effectué sur un appareil Malvern Zetasizer Nano série - Nano-ZS (modèle Zen 3600 ; serial N° 500180 ; UK).

La microscopie électronique à balayage a été réalisé en utilisant un microscope Topcon (Akashi) EM 002B ultra-haute résolution 200kV.

Les différences entre les valeurs apportées par les deux techniques sont dues d'une part à la coloration orange des particules de carboxylates de fer, le faisceau laser de l'appareil de diffusion de lumière étant rouge, et d'autre part à des phénomènes d'agrégation particulaire.

### Exemple 5 : Synthèse de carboxylates de fer (III) à surface modifiée par le chitosane

La modification de surface des nanoparticules avec le chitosane permet d'envisager différentes voies d'administration des nanoparticules grâce aux propriétés de bio-adhésion spécifiques à ce polymère.

Dans cet exemple la modification superficielle est réalisée pendant la synthèse du matériau MIL-88A.

### a) Préparation des nanoparticules à surface modifiée

A une solution de FeCl₃.6H₂O (1 mmol, 270 mg; Alfa Aesar, 98%) et d'acide fumarique (1 mmol, 116 mg; Acros, 99%) dans 5 mL d'eau distillée, dans un bombe en téflon de 23 mL, ont été ajoutés 7 mg de l'agent modifiant de surface, le chitosane modifié. Deux types de chitosane modifiés avec des chaînes alkyle (C12, lauryle) ont été utilisés ; l'un avec un modification de 2% de chaînes alkyles (Q25) et l'autre modifie avec 7% (Q100).

Pour la dissolution complète du chitosane, la solution est placée sous agitation pendant 45 minutes.

La bombe en téflon est placée dans un corps métallique fermé hermétiquement et chauffé dans un étuve à 80ºC pendant 12 heures.

Le solide obtenu est récupéré par centrifugation à 5000 rpm pendant 10 minutes et lavé avec de l'eau distillée et l'acétone.

### b) Analyse et caractérisation

La taille de particules obtenues est mesurée avec un appareil potentiel Z Malvern Zetasizer Nano serie - Nano-ZS; model Zen 3600 ; serial N° 500180 ; UK, en observant une taille de 2,64 et 0,91 microns pour MIL88A-Q 25 et MIL88A-Q 100, respectivement.

Les diagrammes de diffraction de rayon X (DRX) sont collectés avec un diffractomètre Siemens D5000 X Pert MDP (λ_{Cu} Kα₁, Kα₂) de 3 à 20º (2θ) avec un taille de pas de 0,04º et 2 s par pas.

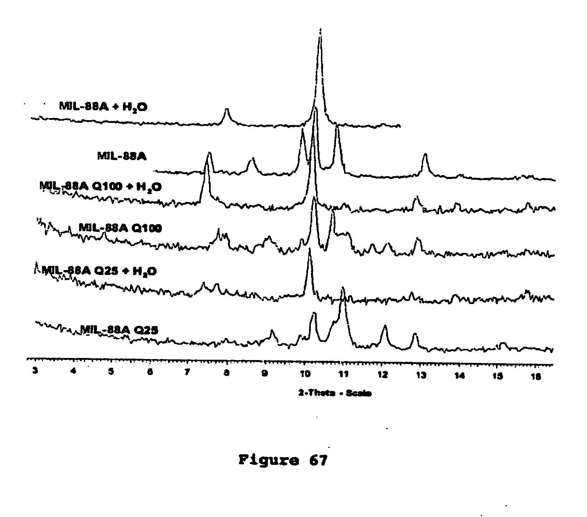

Les diagrammes de DRX présentés en figure 67 ont permit de vérifier que la phase obtenue est bien le MIL-88A. La flexibilité du matériau est également vérifiée par DRX en ajoutant une goutte d'eau sur le solide.

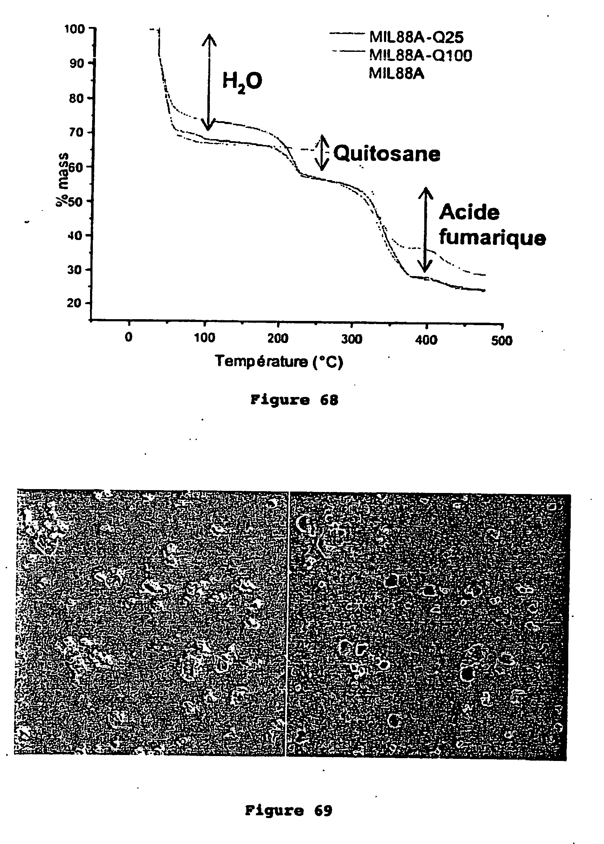

La quantité de chitosane incorporée au matériau est estimée par analyse thermogravimétrique (ATG) présentée en figure 68. L'appareil utilisé est un appareil TGA2050 TA de 25 à 500°C avec un rampe de chauffage de 2°C/min sous flux d'oxygène (100mL/min). Dans les matériaux, la quantité d'acide fumarique est bien autour du 45% (par rapport au produit déshydraté). Les matériaux MIL88A-Q25 et MIL-88A-Q100 contiennent une quantité de chitosane de l'ordre de 16% et de 22% (poilas) par rapport au produit déshydraté, respectivement.

### Exemple 6 : Synthèse de carboxylates de fer (III) à surface modifiée par le dextrane fluoresceine biotin

Dans cet exemple, le dextrane utilisé est greffé d'une part avec la fluorescéine et d'autre part avec de la biotine (Dex B FITC 10000 g/mol, anionique, lysine fixable, Molecular Probes, catalog D7178).

Les caractéristiques du dextrane sont les suivantes : Dextrane fluorescein and biotin, poids moléculaire 10 000 g/mole, anionique, capable de fixer la lysine (« mini-emerald ») lot 36031A, D7178, « Molecular probes », Technologie de détection in vitro, 1 mole fluorescéine/mole, 2 moles biotine/mole.

### a) Préparation des nanoparticules à surface modifiée

Les particules de 1,3,5-benzenetricarboxylate de fer MIL-100 (diamètre de particule 1,79 microns) ont été lavées avec de l'eau MilliQ.

Cinq milligrammes de particules ont été dispersées dans 0,5 mL eau MilliQ. Nous avons rajouté à cette suspension 0,5 mL de solution aqueuse de Dex B FITC (5 mg/mL). Elles ont été incubées a température ambiante pendant 24 h, puis récupérées par centrifugation (3800 rpm, 10 minutes). Le surnageant a été prélevé, puis le culot (particules) a été resuspendu dans 0,5 ml d'eau MilliQ. Après une nouvelle centrifugation, les particules ainsi lavées du Dex B FITC en excès ont été placées sur une lamelle afin d'être observées dans le microscope confocal (excitation 488 nm, émission 515 nm).

### b) Analyse et caractérisation

La fluorescéine permet la détection des particules à l'aide d'un microscope confocal à balayage laser, alors que la biotine, hydrophobe, permet :
1. l'ancrage dans le coeur des particules
2. la fonctionnalisation avec des ligands biotinylés.

La figure 69 présente les coupes optiques ainsi obtenues. On distingue une auréole autour des particules, indiquant la présence de dextrane (seul composé fluorescent) uniquement à la surface. En effet, les longues chaînes de polymère n'ont pas pu pénétrer au coeur des particules.

Cette méthode de modification de surface présente l'avantage de ne pas perturber le coeur des particules (contenant des principes actifs) et de se faire post-synthèse, donc d'offrir une variété de recouvrements possibles.

### Exemple 7: Synthèse de carboxylates de fer (III) à surface modifiée par le polyéthylène glycol (PEG)

Pour réduire au minimum la toxicité du Busulfan dans le foie, il faut empêcher les nanoparticules d'être dirigées vers le foie; la meilleure méthode consiste à greffer en surface des nanoparticules hybrides des chaînes hydrophiles de type polyéthylène glycol (PEG), afin de diminuer leur accumulation dans cet organe. Nous envisageons une étude complète de la dégradation *in vitro* des particules recouvertes ou non de PEG, dans des milieux différents.

Les chaînes du PEG pourront avoir différent groupes terminaux pour se greffer à la surface des matériaux. Ainsi, l'interaction du PEG avec la surface de particule peut être modifié en utilisant différents types de PEG.
- PEG-NH₂ (alpha-t-butyloxycarbonylamino-omega-amino poly(ethylenglycol) (PEG ; Boc-NH-PEG-NH2, 5000 MW, Iris Biotech)
- PEG-COOB (poly(ethylenglycol) carboxylic acid, Iris Biotech)
- PEG-PO₄ synthétisé au laboratoire selon le procédé suivant :

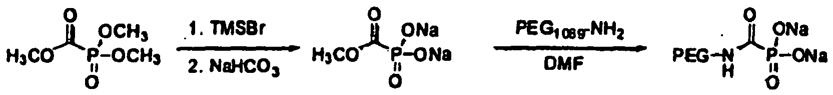

Le groupe phosphonate est attaché au PEG-NH₂ par une condensation amide avec un ester lié à un groupe phosphonate. Le sel de sodium du phosphonate a été utilisé. Ensuite, le couplage a été réalisé a partir du triméthyl-phosphonoformate [CAS 31142-23-1] selon la procédure décrite par Robert A. Moss, Hugo Morales-Rojas, Saketh Vijayaraghavan and Jingzhi Tian, J. Am. Chem. Soc., 2004, 126,(35), 10923 - 10936 [52].

Une dissolution du PEG-NH2 (87,6 mg, M = 5400, Iris Biotech GmbH, PEG1069) dans 2 mL de DMF (Fluka, 97%) avec un excès de disodium méthylphosphonoformate (50 mg, M = 183,99) a été chauffé a 100°C pendant 15h sous agitation. Après, le solvant est éliminé sous vide et le résidu suspendu dans éthanol absolu. L'excès de phosphonoformate est insoluble, donc il peut être éliminé par filtration. Le filtrat est concentré pour obtenir le produit (85 mg). NMR ³¹P, (D₂O), d = 1,3 ppm.

La modification par polyéthylène glycol pourra se réaliser pendant ou après la synthèse comme indiqué ci-dessous.

### a) Modification superficielle avec du PEG-COOH pendant la synthèse des nanoparticules

Les synthèses des NOP₉ se font directement en présence de monométhoxy PEG monoacide (MeO-PEG-COOH) de formule générale CH₃-O-(CH₂-CH₂-O)ₙ-CH₂-CH₂-COOH (Sigma, masse molaire 5000 g/mole).

Le monométhoxy PEG monoacide est introduit à 3 ; 8,5 ou 13 % en masse par rapport au poids total de solide utilisé en synthèse.

### Procédé de préparation :

L'acétate de fer (1 mmol; synthétisé selon la synthèse A décrite dans l'exemple 2) et l'acide muconique (1 mmol; Fluka 97%) sont mélangés dans 10 mL de méthanol (Aldrich, 99,9%). Le tout est introduit dans un corps en téflon de 23 mL. Le monoacide PEG est ensuite introduit à hauteur de 3 ; 8,5 ou 13 % en masse par rapport au poids total de solide. 0,35 mL de soude 2 M est éventuellement ajoutée. La solution est placée sous agitation pendant 20 minutes.

La bombe en téflon est placée dans un corps métallique fermé hermétiquement et chauffé dans une étuve à 100°C pendant 12 heures.

Le solide obtenu est récupéré par centrifugation à 5000 rpm pendant 10 minutes et lavé avec de l'eau distillée et l'acétone.

Le dosage du PEG dans les carboxylates de fer est effectué comme suit : les particules sont dégradées totalement en milieu acide (HCl 5M) afin de libérer le PEG associé. Après neutralisation des solutions obtenues (à pH=7) et destruction des nanoparticules avec de la soude, le dosage du PEG a été effectué par spectrophotométrie UV (à la longueur d'onde de 500 nm), d'après la méthode décrite dans B. Baleux et al. C.R. Acad. Sciences Paris, série_C, 274 (1972) pages 1617-1620 [53]. Les principaux résultats sont répertoriés dans le tableau suivant.

**Tableau 10. Modification du matériau MIL88A avec le PEG 5000 g/mole.**

| Ajout de solution aqueuse de NaOH | % massique de PEG introduit en début de synthèse | % massique de PEG dans la composition des nanoparticules | Diamètre des nanoparticules (nm) (mesuré par diffusion de lumière) |
|---|---|---|---|
| - | 3 | 3,8 | 570 |
| oui | 3 | 4,8 | 230 |
| - | 8,5 | 13,4 | 590 |
| oui | 8,5 | 13 | 230 |
| - | 13 | 18,5 | 565 |
| oui | 13 | 18 | 310 |

Nous pouvons constater que :
- l'ajout de soude permet de diminuer la taille des nanoparticules
- le % massique de PEG dans les nanoparticules est supérieur au % massique de PEG introduit en début de synthèse
- de manière remarquable, il est possible d'obtenir des particules d'environ 230 nm contenant 13 % en poids de PEG, ce qui est intéressant pour des applications médicales (« furtivité »)

En effet, les nanoparticules « furtives » décrites dans la littérature contiennent généralement moins de 10 % mass de PEG, comme décrit dans R. Gref et al. Colloids and Surfaces B: Biointerfaces, Volume 18, Issues 3-4, October 2000, pages 301-313 [54].

### b) Modification superficielle des nanoparticules MIL-100 avec du PEG après la synthèse desdites nanoparticules

Les nanoparticules du MIL-100 sont synthétisées par voie microondes (Microondes CEM) en partant d'une solution de 9,7 g nitrate de fer hexahydrate (Aldrich, 97%), 3,38g d'acide 1,3,5-benzenetricarboxylique (1,3,5-BTC, Aldrich, 99%) et 40 g d'eau distillée à 180°C pendant 30min (puissance 600W). La taille de particule mesurée par diffusion de lumière est de 400nm.

Les nanoparticules pegylées du MIL-100 modifiées par polyéthylène glycol sont obtenues par la modification de surface des particules citées antérieurement. 30 mg du MIL-100 sont suspendus dans 3mL d'une solution aqueuse de 10mg du polyéthylène glycol aminoteminal (PEG-NH2 5000 g/mole, Aldrich, 97%) à 30°C pendant 3 heures sous agitation. Ces nanoparticules sont récupérées par centrifugation (10000 rpm/10 min) et lavées avec de l'eau distillée.

La quantité du PEG en surface est déterminée par la méthode de Baleux et Champertier, basée dans la formation d'un complexe coloré par l'iodine-iodide sur le PEG, lequel est sélectivement mesuré par spectrophotométrie à 500nm). La quantité du PEG est de 19% en masse et la taille de particules après la modification par le polyéthylène glycol augmente à 800nm. Par contre, l'observation de nanoparticules modifiées et non modifiées par le PEG par microscopie électronique à balayage (SEM) montre de nanoparticules de 150nm dans les deux cas. Cette différence peut être dûe a des phénomènes d'agrégation particulaires.

### Exemple 8 : Synthèse par voie ultrasons de carboxylates de fer (III) à surface modifiée par le polyéthylène glycol (PEG)

La synthèse par voie ultrasons de nanoparticules de solide MIL-88A à surface modifiée par le PEG a été réalisée à différents temps de reaction (30, 60, 90 et 120 minutes).

Dans les exemples qui suivent deux modes opératoire ont été effectués :
a) dans le 1^{er} mode opératoire, le PEG est ajouté 15 minutes avant la fin de la synthèse,
b) dans le second mode opératoire, le PEG est ajouté dès le début de la synthèse (t = 0 min).

Pour chacune des synthèses ci-dessous, des solutions aqueuses de chlorure de fer III (2,7 mg/ml ; FeCl₃ 6H₂O commercialisé par ACROS 97%) et d'acide fumarique (1,16 mg/ml ; C₄H₄O₄ commercialisé par ACROS 99%) sont préparées. Les deux réactifs solides sont pesés et dissous séparément dans l'eau dans les proportions données dans les exemples ci-dessous. La solution d'acide fumarique est portée à 70°C sous agitation pendant 120 min pour solubiliser le produit. Le chlorure de fer est agité à l'aide d'un agitateur magnétique pendant 30 min.

### a) Synthèse n° 1 :

Au total 8 fioles sont préparées. Dans chacune des 8 fioles de 20 mL, sont ajoutés 5 mL de solution de chlorure de fer III (2,7 mg/ml) et 5 mL de solution d'acide fumarique (1,16 mg/ml) :
- 4 fioles servent de témoin où les réactions sont réalisées pour les 4 temps de synthèse : 30, 60, 90 et 120 min,
- dans les 4 autres fioles, 5 mg de PEG sont ajoutés 15 min avant la fin de chacune des synthèses de durée 30, 60, 90 et 120 min (la fin d'une synthèse correspond à la sortie du bain à ultrasons).

Les 8 fioles sont placés en même temps dans un bain de sonication à 0°C, pendant les durées t correspondantes (30, 60, 90 et 120 min).

Après la synthèse, un volume de 0,1 ml de solution est prélevé dans chaque fiole afin de déterminer la taille des particules par diffusion de lumière grâce à un appareil de Dynamic Light Scattering (DLS, Nanosizer). Le reste de solution est ensuite passé à la centrifugeuse à 10 000 rpm à 0°C pendant 15 min afin de séparer le surnageant du solide formé. Le surnageant est éliminé à l'aide d'une pipette pasteur et le culot récupéré est placé sous la hotte à température ambiante (environ 20°C).

Appareillage utilisé :
- Bain de sonication : Labo-moderne TK 52H n° de sérié : 164046192 SONOCLEAU
- Centrifugeuse : JOUAN MR 1812
- Nanosizer : Coulter N4 PLUS USA ; Malvern

### b) Synthèse n° 2 :

Au total 8 fioles sont préparées. Dans chacune des 8 fioles de 20 mL, sont ajoutés 5 mL de solution de chlorure de fer III (2,7 mg/ml) et 5 mL de solution d'acide fumarique (1,16 mg/ml) :
- 4 fioles servent de témoin où les réactions sont réalisées pour les 4 temps de synthèse : 30, 60, 90 et 120 min,
- dans les 4 autres fioles, 5 mg de PEG sont ajoutés dès le début de chacune des synthèses de durée 30, 60, 90 et 120 min.

Les 8 fioles sont placés en même temps dans un bain de sonication à 0°C, pendant les durées t correspondantes (30, 60, 90 et 120 min).

Après la synthèse, un volume de 0,1 ml de solution est prélevé dans chaque fiole afin de déterminer la taille des particules par diffusion de lumière grâce à un appareil de Dynamic Light Scattering (DLS, Nanosizer). Le reste de solution est ensuite passé à la centrifugeuse à 10 000 rpm à 0°C pendant 15 min afin de séparer le surnageant du solide formé.

Le surnageant est éliminé à l'aide d'une pipette pasteur et le culot récupéré est placé sous la hotte à température ambiante (environ 20°C).

Appareillage utilisé :
- Bain de sonication : Labo-moderne TK 52H n° de série : 164046192 SONOCLEAN
- Centrifugeuse JOUAN MR 1812
- Nanosizer : Coulter N4 PLUS USA ; Malvern

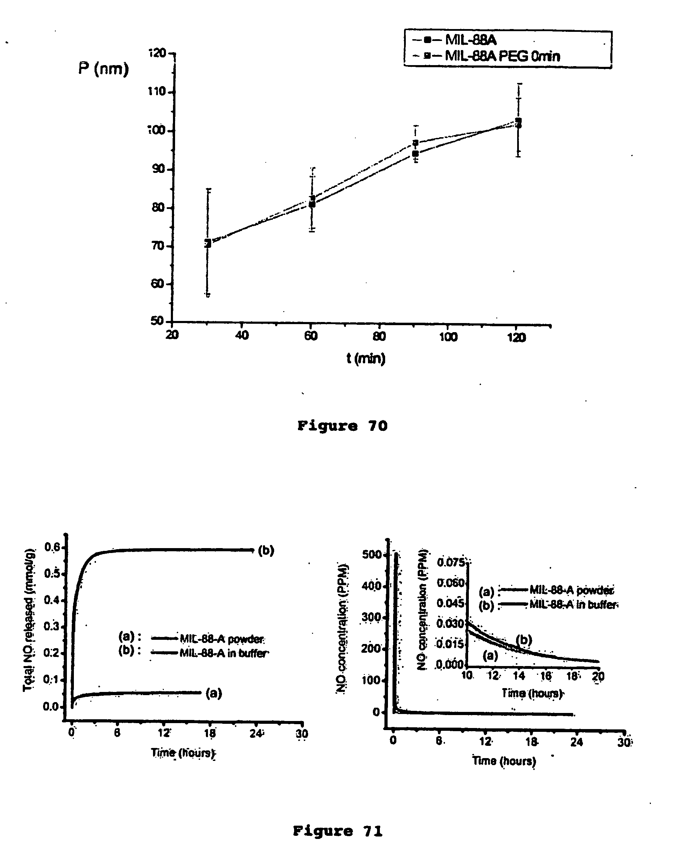

L'évolution de la taille de particule (P en nm) en fonction du temps (t en min) est représenté dans la figure 70. Celle-ci montre qu' il n'y a pas de variation significative après l'ajoute du PEG au temps initial de synthèse.

Que ce soit en présence ou non de PEG au temps initial de la synthèse, il est possible d'observer en DRX un epaulement à 11°, caractéristique de la phase MIL-88A qui semble augmenter en intensité avec le temps de synthese.

### c) conclusion de l'étude:

Le but de cette étude est d'optimiser la taille des particules qui doivent être inférieures à 200 nm afin de pouvoir les rendre compatible avec une administration par voie intraveineuse. Les résultats obtenus sont satisfaisants puisque les diamètres de particules obtenus sont inférieurs à 200 nm (avec vérification des structures cristallines type MIL-88A dans la plupart de solides). De plus, même si les rendements sont inférieurs à ceux obtenus par voie solvothermale ou voie microondes, on peut les considérer comme acceptables (tableau ci-dessous).

**Tableau 11. Rendements de synthèse par voie ultrasons**

| **Temps (min)** | **Rendement (%)** | | | |
|---|---|---|---|---|
| | **Témoin** | **AcH** | **PEG t=0** | **PEG t=f-15min** |
| **30** | 24 | 13,4 | 31,4 | 20,1 |
| **60** | 27,2 | 15 | 29,4 | Non mesuré |
| **90** | 35,6 | 14 | 24 | 28,3 |
| **120** | 35,1 | 19,1 | 32 | 41,2 |

Il est possible d'observer que la taille des particules augmente en fonction du temps de synthèse.

De même, la modification par le PEG à t=0 min aboutit à des diamètres de particules plus petits que la modification par PEG à t=f-15min, due probablement au fait que la croissance cristalline soit arrêtée plus tôt.

### Exemple 9 : Synthèse de solides MOF à surface modifiée par le polyéthylène glycol (PEG) et l'acide folique (FA)

### Acide folique :

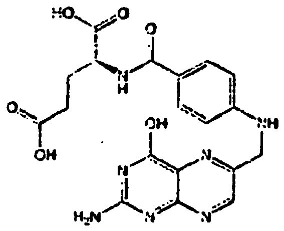

### a) Synthèse n° 1 : modification de surface après synthèse des nanoparticules

### Modification de surface avec du PEG :

100 mg de nanoparticules de MIL100, MIL88, MIL53 ou MIL101 (préalablement deshydraté à 100°C/nuit) sont dispersées sous sonication dans 100 mL de solution à 17,9 mM de 2-(méthoxy(polyéthylèneoxy)-propyl)triméthoxysilane dans le toluène anhydre. Le mélange est soumis à des ultrasons à 60°C pendant 4 h, sous débit de gaz inerte (azote). La suspension colloïdale résultante, contenant les nanoparticules à surface modifiée avec du PEG, est lavée deux fois avec de l'éthanol et deux fois dans une solution de citrate de sodium 20 m*M* (pH 8,0) et resuspendue finalement dans l'eau.

### Modification de surface avec du PEG et du FA :

Le FA a été attaché aux nanoparticules grâce à un espaceur bifonctionnel, le silane-PEG-trifluoroéthylester (TFEE) synthétisé selon une méthode décrite dans la littérature par Kohler N. et al., J Am Chem Soc 2004; 126 ; 7206-7211 [55].

100 mg de nanoparticules sont recouvertes par du PEG-TFEE selon la même méthode que décrit ci-dessus, en utilisant le silane-PEG-TFEE à la place du 2-méthoxy (polyéthylèneoxy)-propyltriméthoxysilane.

Les nanoparticules résultantes, recouvertes de PEG-TFEE, sont lavées deux fois puis resuspendues dans 100 mL de toluène sec. Une amine primaire a été greffée aux groupements terminaux des chaines de PEG en rajoutant 1 mL de éthylènediamine (Sigma) aux nanoparticules maintenues sous débit d'azote. Le mélange a été soumis aux ultrasons (4h, 60°C). Les nanoparticules résultantes, recouvertes par l'amine, ont été lavées trois fois avec de l'éthanol, et trois fois avec du diméthyl sulfoxide (DMSO). Les nanoparticules ont été finalement resuspendues dans 50 mL de DMSO anhydre. Le FA a été couplé aux groupements amine terminaux des chaines de PEG en rajoutant 50 mL de solution de FA (23 mM à dans du DMSO) avec des quantités équimolaires de dicyclohexylcarbodiimide (DCC) (Sigma) et 10 pL de pyridine. Le mélange a été protégé de la lumière et laissé réagir pendant une nuit sous agitation bidimensionnelle (180 rpm). Les nanoparticules conjuguées avec du PEGH et du FA (NP-PEG-FA) ont été lavées deux fois avec de l'éthanol, et deux fois avec une solution de citrate de sodium 20 mM (pH 8,0) et resuspendues finalement dans cette même solution de citrate de sodium.

### b) Synthèse n°2 : modification de surface pendant la synthèse des nanoparticules

La modification de la surface des solides MOF peut également se faire pendant la synthèse.

Dans l'exemple qui suit, la modification de surface est réalisée avec du chitosane préalablement greffé avec de l'acide folique (FA).

Un exemple de synthèse de chitosane greffé avec de l'acide folique via un espaceur PEG est décrite dans la publication de Peggy Chan et al., Biomaterials, Volume 28, Issue 3, 2007, pp 540-549 [56].

Les réactifs suivants ont été utilisés pour la réalisation de cet exemple :
- chitosane (masse molaire Mn de 255 kDa, viscosité: 200-800 cps dans 1% d'acide acétique, commercialisé par la société Sigma-Aldrich)
- le *N*-hydroxylsuccinimide-PEG-Maleimide (NBS-PEG-MAL, Mn 3400 Da, commercialisé par la société Nektar, NOF Corporation, Tokyo, Japan)
- l'ester succinimidyl de monométhoxy-PEG (mPEG-SPA, Min 5000 Da, commercialisé par la société Nektar, NOF Corporation, Tokyo, Japan).

Le chitosane est préalablement déacétylé pour obtenir un degré d'acétylation de 82% (determiné par ¹H-NMR) selon le procédé décrit par Wang LS (Thesis, National University of Singapore, Singapore, 2001).

100 mg de chitosane ont été dissous dans 50 mL de solution d'acide acétique (20%). Le pH de la solution a été ajusté à 6 par addition d'hydroxyde de sodium et le mPEG-SPA a été introduit dans le mélange réactionnel. Le mélange est laissé réagir pendant 24 h à température ambiante sous agitation. Le produit obtenu est dialysé pendant 24 h contre de l'eau de-ionisée en utilisant une membrane avec un seuil de coupure de 12,000 Da (Spectrum Laboratories, USA) et finalement, lyophilisé.

Pour synthétiser le chitosane greffé avec du PEG et du FA, l'ester de N-hydroxysuccinimide de FA a été préparé selon la méthode décrite par J.H. Van Steenis et al., J Control Release 87 (2003), pp. 167-176 [57].

Brièvement, 1 g de FA a été ajouté à un mélange de DMSO anhydre (40 mL) et triéthylamine (TEA, 0,5 mL). Le mélange a été agité à l'abri de la lumière pendant la nuit, dans des conditions anhydres. Les autres réactifs, dicyclohexylcarbodiimide (DCC, 0,5 g) et N-hydroxysuccinimide (NHS, 0,52 g) ont été ajoutés et le mélange est laissé réagir pendant 18h à l'abri de la lumière et dans des conditions anhydres. Le produit secondaire, la dicyclohexylurée (DCU) ayant précipité, a été enlevé par filtration. Le DMSO et le TFA ont été évaporés sous vide. Le produit de la réaction a été séché sous vide, puis dissout dans 1,5 mL de mélange 2:1 (v:v) de DMSO et TEA. Une quantité équimolaire de 2-aminoéthanethiol (Wako) a été rajoutée et la reaction a été laissée se poursuivre pendant la nuit dans des conditions anhydres. Ainsi, un groupement thiol a pu être introduit à l'acide folique et le produit résultats est dénommé

100 mg de chitosane sont dissous dans 50 mL de solution d'acide acétique (20%). Le pH de la solution est ajusté à 6 par addition d'hydroxyde de sodium et 100 mg de NHS-PEG-Mal sont introduits dans le mélange réactionnel. La mélange est laissé réagir pendant 3 h à température ambiante (environ 20°C) sous agitation, puis le pH est ajusté à 7. Le mélange est laissé réagir pendant la nuit, dans des conditions anhydres. Le FA-SH synthétisé comme précédemment a été rajouté de manière graduée sous agitation et le pH a été ajusté à 6,5-7,5 avec de la soude.

Le conjugué obtenu dénommé FA-PEG chi porte du FA couplé au chitosane via un bras espaceur de PEG, ce qui est un avantage pour atteindre le récepteur de l'acide folique (comme il a été décrit dans la littérature, voir par exemple : A. Gabizon, H. Shmeeda, A.T. Horowitz and S. Zalipsky, Tumor cell targeting of liposome-entrapped drugs with phospholipids-anchored folic acid-PEG conjugates, Adv Drug Deliv Rev 56 (2004), pp. 1177-1192 [58].

Le degré de substitution peut être ajusté en variant le rapport massique PEG : chitosane utilisé dans la réaction. Ce polymère a été dialysé pendant 48 h contre de l'eau de-ionisée en utilisant une membrane avec un seuil de coupure de 12,000 Da (Spectrum Laboratories, USA) et finalement, lyophilisé.

### c) Synthèse n°3 :

Les solides hybrides peuvent être modifiés en surface par adsorption de polysaccharides tels que le dextrane greffé avec la biotine.

Nous pouvons donc envisager d' adsorber, à la place du dextrane greffé avec la biotine, du chitosane greffé avec l'acide folique (synthétisé comme décrit dans la publication citée plus haut), et, éventuellement, si nécessaire, greffé aussi avec d'autres composés hydrophobes comme le cholestérol ou des chaînons aliphatiques, afin d'assurer une meilleure adhésion au niveau de la surface des nanoparticules.

Une fonctionnalisation de surface pourrait également se faire *via* l'adsorption d'autres polysaccharides greffés avec de le FA.

### d) Synthèse n°4 :

Les solides hybrides peuvent être modifiés en surface avec du PEG lors de leur synthèse. Le monométhoxy PEG monoacide utilisé lors de cette synthèse est substitué par du PEG monoacide comportant une fonction réactive bloquée en bout de chaine comme le produit commercial :

Boc-PEG-carbonateNHS, MW 5000, Boc = tert-butoxycarbonyle (Reference SUNBRIGHT^{®} BO-050TS, NOF Corporation).

Après réaction, comme indiqué dans l'exemple, des mélanges Me0-PEG-COOH et Boc-PBG-carbonateNHS (rapports massiques 1:0.05 à 1:0,5) sont utilisés à la place de Me0-PEG-COOH. La déprotection se fera par ajout d'acide trifluoroacétique (TFA).

### Protocole opératoire:

On ajoute 0,6 mL de TFA à une suspension de 300 mg de nanoparticules dans 2 mL d'eau. On laisse réagir pendant 1h à température ambiante (environ 20°C) sous agitation magnétique. On isole les particules par centrifugation et on les lave trois fois avec de l'eau distillée.

On fonctionnalise les groupements réactifs en surface avec des ligands tels le FA, par exemple comme dans la synthèse n°1 indiqué dans le paragraphe a).

### e) Caractérisation des nanoparticules :

La quantité d'acide folique effectivement couplée aux nanoparticules pourra être déterminée après dégradation de celles-ci dans un milieu acide, neutralisation à pH 7, puis redissolution dans un solvant approprié, tel le dichlorométhane, le DMSO, ou un mélange de ces deux solvants. L'acide folique pourra alors être quantifié par une mesure de l'absorbance UV (à 358 nm, le coefficient d'extinction molaire E de l'acide folique est 15,76 M⁻¹ cm⁻¹).

Pour vérifier que l'acide folique se trouve bien en surface des nanoparticules, la technique de la résonance de surface du plasmon (BIAcore) est utilisé. La protéine qui se lie à l'acide folique (« folate binding protein ») est immobilisée au niveau de la surface du détecteur, sur un mince film de dextrane activé (procédure classique recommandée par le constructeur BIAcore). La quantité de nanoparticules effectivement attachée à ce support est évaluée par rapport à celle de nanoparticules non recouvertes d'acide folique.

### Exemple 10 : Synthèse de matériaux MOF à base de ligands bioactifs

L'utilisation des ligands avec une activité biologique présente un intérêt pour :
- la libération de composé actif par dégradation du matériau MOF
- l'encapsulation d'autres molécules actives pour des thérapies combinées

Des tests de l'activité antimicrobienne, ainsi que la dégradation dans des milieux physiologiques et l'activité sur des cellules seront réalisés sur des carboxylates de fer poreux a structure flexible de type MIL-88 utilisant les acides 4,4'azobenzene dicarboxylique et 3,3'-dichlore 4,4'-azobenzene dicarboxylique, entre autres.

Dans les synthèses qui suivent, divers molécules bioactives sont utilisées pour préparer les matériaux MOF de la présente invention, et notamment : l'azobenzène, l'acide azélaïque et l'acide nicotinique.

L'azobenzène (AzBz), de formule C₆H₅-N=H-C₆H₅, peut être incorporé aux matrices de polymère comme stabilisateur. De plus, la structure rigide des molécules azoïques leur permet de se comporter en tant que mésogènes à cristal liquide dans de nombreux matériaux. Par ailleurs, l'azobenzène peut être photoisomérisé (isomère *cis* ou *trans*) d'où son utilisation pour photomoduler l'affinité d'un ligand (par exemple un médicament) pour une protéine. En effet, l'azobenzène peut jouer le rôle de photo-interrupteur entre un ligand et une protéine en permettant ou en empêchant la liaison protéine-médicament suivant l'isomère cis ou trans de l'azobenzène (une extrémité de l'azobenzene peut par exemple être substituée par un groupement se liant à la protéine cible tandis que l'autre extrémité est reliée à un ligand (médicament) de la protéine).

L'acide azélaïque (HO₂C-(CH₂)₇-CO₂H) est un acide dicarboxylique saturé qui a des propriétés antribactérienne, kératolytique et comédolytique. Il est utilisé notamment dans le traitement de l'acné et la rosacea.

L'acide nicotinique (C₅H₄N-CO₂H) est l'une des deux forme de la vitamine B3 avec la nicotinamide. La vitamine B3 est notamment nécessaire au métabolisme des glucides, lipides et protéines.

### a) MIL-88G(AzBz)(Fe) ou Fe₃O[C₁₂H₈N₂-(CO₂)₂]₃.X.nH₂O (X=F, Cl, OH)

118 mg de Fe(ClO₄)₃.xH₂O (0.33 mmol, Aldrich, 99%) et 90 mg (0,33 mmol) d'acide 4,4'-azobenzenedicarboxylique (synthétisé selon la méthode décrite par Ameerunisha et al., J. Chem. Soc. Perkin Trans. 2, 1679, 1995 [59]) sont dispersés dans 15 ml de DMF (Fluka, 98%). Le tout est laissé dans un corps en Téflon de 23 ml mis dans une bombe métallique PAAR pendant 3 jours à 150°C. Le solide est récupéré par filtration.

200 mg du solide sont suspendus dans 10mL de DMF sous agitation à température ambiante pendant 2 h pour échanger l'acide restant dans les pores. Le solide est ensuite récupéré par filtration puis calciné à 150°C sous vide pendant 15 heures pour éliminer le DMF restant dans les pores.

La taille de particule mesurée par diffusion de lumière est > 1 micron.

### b) MIL-88G-2Cl(AzBz-2Cl)(Fe) ou Fe₃O[C₁₂H₆N₂=Cl₂-(CO₂)₂]₃.X.nH₂O (X=F Cl, OH)

177 mg de Fe(ClO₄)₃.xH₂O (0,5 mmol, Aldrich, 99%) et 169 mg (0,5 mmol) d'acide dichloro-4,4'-azobenzenedicarboxylique (préparé selon la synthèse D décrite dans l'exemple 3) sont dispersés dans 15 ml de DMF (Fluka, 98%). Le tout est laissé dans un corps en Téflon de 23 ml mis dans une bombe métallique PAAR pendant 12 h à 150°C. Le solide est récupéré par filtration.

200 mg du solide sont suspendus dans 10mL de DMF sous agitation à température ambiante pendant 2 h pour échanger l'acide restant dans les pores. Le solide est ensuite récupéré par filtration puis calciné à 150°C sous vide pendant 15 h pour éliminer le DMF restant dans les pores.

La taille de particule mesurée par diffusion de lumière est > 1 micron.

### c) Azobenzène 3,3',5,5'-tétracarboxylate de fer 1

118 mg de Fe(ClO₄)₃.xH₂O (0,3 mmol, Aldrich, 99%) et 119 mg (0,3 mmol) d'acide 3,3',5,5'-azobenzènetétracarboxylique (préparé selon la synthèse E décrite dans l'exemple 3) sont dispersés dans 15 ml de DMF (Fluka, 98%) avec 0,1 mL de HF 5M (SDS, 50%). Le tout est laissé dans un corps en Téflon de 23 ml mis dans une bombe métallique PAAR pendant 3 jours à 150°C. Le solide est récupéré par filtration et lavé avec de l'acétone.

Le solide obtenu a une structure cubique rigide.

La taille de particule mesurée par diffusion de lumière est > 1 micron.

### d) Azobenzene 3,3',5,5'-tétracarboxylate de fer 2

118 mg de Fe(ClO₄)₃.xH₂O (0,3 mmol, Aldrich, 99%) et 119 mg (0,3 mmol) d'acide 3,3',5,5'-azobenzènetétracarboxylique (préparé selon la synthèse E décrite dans l'exemple 3) sont dispersés dans 15 ml de l'eau distillée avec 0,1 mL de HF 5M (SDS, 50%). Le tout est laissé dans un corps en Téflon de 23 ml mis dans une Bombe métallique PAAR pendant 3 jours à 150°C. Le solide est récupéré par filtration et lavé avec l'acétone.

La taille de particule mesurée par diffusion de lumière est 498 nm, avec une deuxième population minoritaire de 1100 nm.

### e) Azélate de fer 1

270 mg de FeCl₃.6H₂O (1 mmol, Aldrich, 99%) et 188 mg (1 mmol) d'acide azelaique (Aldrich, 99%) sont dispersés dans 5 ml d'eau distillée. Le tout est laissé dans un corps en Téflon de 23 ml mis dans une Bombe métallique PAAR pendant 3 jours à 100°C. Le solide est récupéré par filtration et lavé à l'acétone.

200 mg de solide sont suspendus dans 50 mL d'éthanol absolu sous agitation pendant 5 h pour l'activer. Le solide est récupéré par filtration.

La taille de particule mesurée par diffusion de lumière est > 1 micron (1500 nm).

### f) Nicotinate de fer 1

Les conditions de synthèse dans l'eau sont les suivantes:
135 mg de FeCl₃.6H₂O (1 mmol, Aldrich, 99%) et 62 mg (1 mmol) d'acide nicotinique (Aldrich, 99%) sont dispersés dans 5 ml d'eau distillée avec 0,1 mL de NaOH 2M. Le tout est laissé dans un corps en Téflon de 23 ml mis dans une Bombe métallique PAAR pendant 16 heures à 100°C. Le solide est récupéré par filtration et lavée avec l'acétone.

Les conditions de synthèse dans le DMF sont les suivantes :
135 mg de FeCl₃.6H₂O (1 mmol, Aldrich, 99%) et 62 mg (1 mmol) d'acide nicotinique (Aldrich, 99%) sont dispersés dans 5 ml de DMF (Fluka, 98%). Le tout est laissé dans un corps en Téflon de 23 ml mis dans une Bombe métallique PAAR pendant 16 h à 100°C. Le solide est récupéré par filtration et lavée avec l'acétone.

La taille de particule monodisperse (PDI=0,241) mesurée par diffusion de lumière est 662 nm.

### g) Nicotinate de fer 2

Les conditions de syntheses du nicotinate de fer 2 sont les suivantes:

71 mg d'acetate de fer (III) (0,12 mmol, selon la procède decrit anterieurment) et 73,8 mg (0,6 mmol) d'acide nicotinique (Aldrich, 99%) sont dispersés dans 5 ml de DMF (Fluka, 98%). Le tout est laissé dans un corps en Téflon de 23 ml mis dans une bombe métallique PAAR pendant 24 heures à 140°C. Le solide est récupéré par filtration et lavée avec l'acétone.

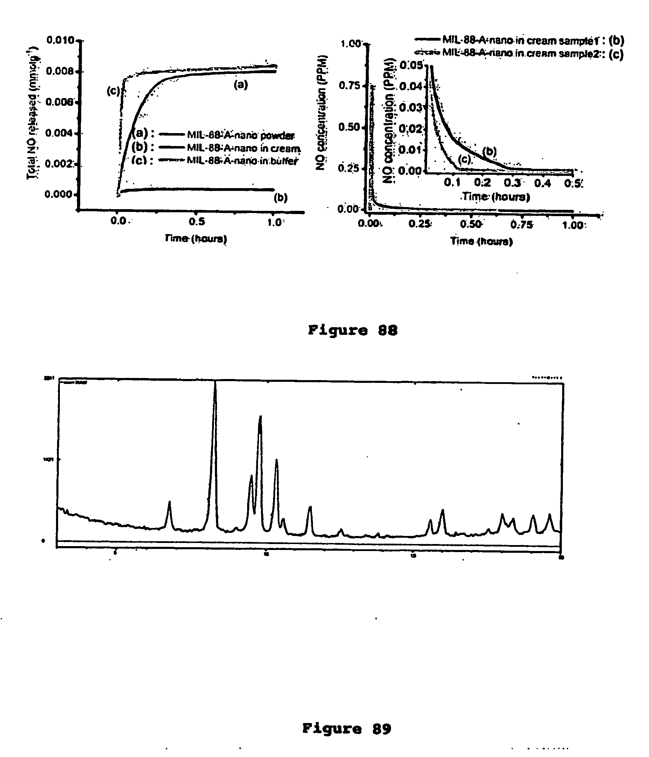

Les donnes cristallographiques de cette phase sont dans le figure 89:
Groupe de space P 21/n
   a = 16.422899 b = 21.423401 c = 11.048300 a = 16.422899 b = 21.423401 c = 11.048300 beta = 91.806999

### Exemple 11 : Détermination de la teneur en fer dans le solide MIL-100(Fe)

### Activation :

Afin de vider les pores du matériau (solvants, acides résiduels) et de libérer les sites de coordination du métal, l'activation du matériau MIL-100(Fe) a été effectuée par chauffage à 150°C sous vide primaire pendant 15 heures. Le solide résultant ne possède que du fer au degré d'oxydation +III.

### Réduction Fe³⁺/Fe²⁺ :

La réduction partielle du matériau MIL-100(Fe) a été effectuée par chauffage a 250°C sous vide primaire pendant 15 heures. La spectroscopie infrarouge a permis de quantifier la teneur relative en fer(II)/fer(III) autour de 20/80 % (Figure 50).

La figure 50 représente la quantité de sites Fer coordinativement insaturés présent dans le solide MIL-100(Fe) activé en fonction du traitement thermique effectué. Le solide MIL-100(Fe) est activé sous vide résiduel (environ 10⁻⁵ Torr) à différentes températures et pendant différentes durées. T(Fe) représente la teneur en sites Fer coordinativement instaurés et T(Fe²⁺) représente la teneur en sites Fe²⁺ coordinativement instaurés (en µmole de sites insaturés par gramme de solide activé ou en % de sites Fer insaturés).

Les quantités de sites Fer insaturés sont déterminées par adsorption de CO à 100K suivie par spectroscopie infrarouge. L'incertitude sur les valeurs est estimée à +/-10 %.

### Exemple 12: Mise en évidence de la flexibilité des solides

La catégorie de solides hybrides flexibles à base de trimères de métaux de transition trivalents est dénommée MIL-88. Ces composés sont typiquement construits à partir de trimères d'octaèdres de fer, c'est-à-dire trois atomes de fer connectés par un oxygène central et par six fonctions carboxylates connectant deux à deux les atomes de fer ; une molécule d'eau terminale, coordinée à chaque atome de fer vient ensuite compléter la coordinence octaédrique du métal. Ces trimères sont ensuite reliés entre eux par des acides dicarboxyliques aliphatiques ou aromatiques pour former les solides MIL-88A, B, C, D et MIL-89 (-A pour acide fumarique, -B pour acide téréphthalique, -C pour acide 2,6-naphtalenedicarboxylique, -D pour acide 4,4'-biphényldicarboxylique et MIL-89 pour l'acide trans, trans muconique), comme décrit dans le document de Serre et al., Angew. Chem. Int. Ed. 2004, 43, 6286 [17]. D'autres analogues avec d'autres diacides carboxyliques ont également été synthétisés et sont nommés MIL-88E, F, G...

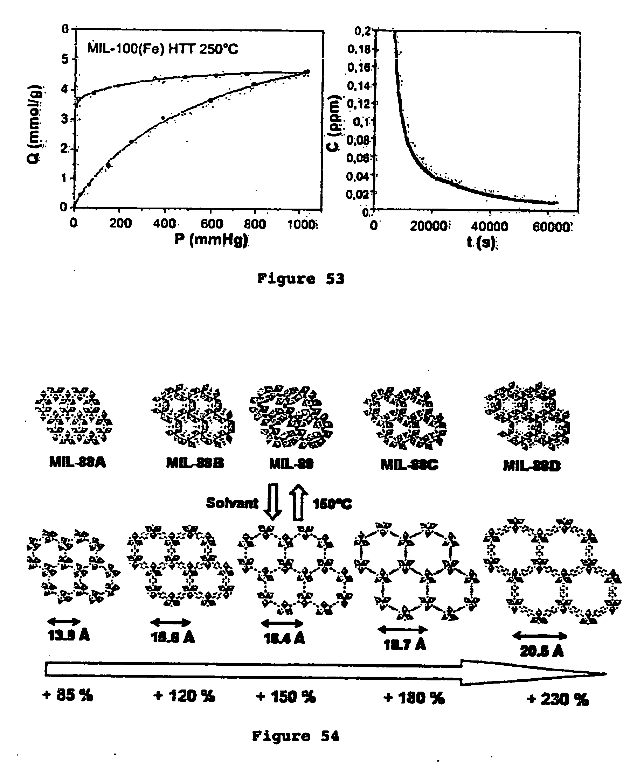

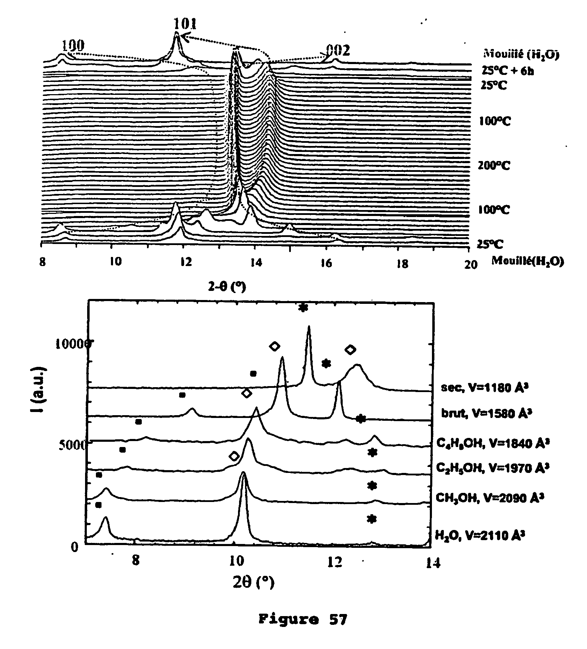

L'étude du comportement de ces solides par diffraction des RX, a permis d'établir que ces composés sont flexibles avec des amplitudes de « respiration » (c'est-à-dire de gonflement ou de contraction) considérables entre leur forme sèche et leur forme solvatée. Il en résulte des variations de volume de maille entre 85 et 230 % selon la nature de l'espaceur organique (figure 54), comme décrit dans le document Serre et al., Science, 2007, 315, 1828 [18]. Les inventeurs ont noté que les formes sèches ne sont pas poreuses avec une taille de pores (tunnels) à peu près identique quel que soit le ligand carboxylique utilisé. Par contre, le gonflement du solide hybride en phase liquide est fonction de la longueur de l'espaceur organique. Ainsi, la distance entre trimères dans la forme gonflée passe de 13,8 Å avec l'acide fumarique (MIL-88A) à 20,5 Å avec le ligand biphényl (MIL-88D). La taille des pores des formes gonflées varie ainsi entre 7 Å (MIL-88A) à 16 Å (MIL-88D). Le gonflement est réversible, comme le montre l'exemple du solide MIL-88A en présence d'eau dans la figure 57 et dépend également de la nature du solvant utilisé comme décrit dans le document Serre et al. J. Am. Chem. Soc., 2005, 127, 16273-16278 [19]. La « respiration » s'effectue de manière continue, sans rupture apparente de liaisons durant la respiration. Par ailleurs, de retour à température ambiante, le solide gonfle à nouveau par resolvatation, confirmant le caractère réversible de la respiration.

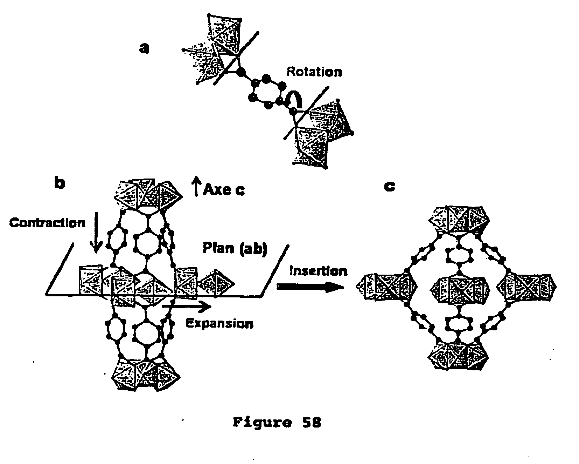

Si l'on regarde de près l'arrangement entre les trimères constitutifs de la structure, chaque trimère est relié à six autres trimères, trois en dessous et trois au dessus, par les dicarboxylates ce qui conduit à la formation de cages bipyramidales de trimères. Au sein de ces cages, la connexion entre trimères s'effectue uniquement selon l'axe c et l'absence de tout lien dans le plan (ab) est à l'origine de la flexibilité (figure 58).

**Tableau 12 :**

| Solide | Condition | a(Å) | c(Å) | V(Å³) | Expansion de la maille | Taille de pore estimée | Solvant |
|---|---|---|---|---|---|---|---|
| MIL-88A | 100°C | 9,6 | 14,8 | 1180 | > 80% | environ 6 Å | Eau |
| | 25°C | 11,1 | 14,5 | 1480 | | | |
| | Forme ouverte | 13,8 | 12,5 | 2100 | | | |
| MIL-88B | 100°C | 9,6 | 19,1 | 1500 | > 100% | environ 9Å | Ethanol |
| | 25°C | 11,0 | 19,0 | 2000 | | | |
| | Forme ouverte | 15,7 | 14,0 | 3100 | | | |
| MIL-88C | 100°C | 9,9 | 23,8 | 2020 | > 170% | environ 13 Å | Pyridine |
| | 25°C | 10,2 | 23,6 | 2100 | | | |
| | Forme ouverte | 18,7 | 18,8 | 5600 | | | |
| MIL-88D | 100°C | 10,1 | 27,8 | 2480 | >220% | environ 16 Å | Ethanol |
| | 25°C | 12,1 | 27,5 | 3500 | | | |
| | Forme ouverte | 20,5 | 22,4 | 8100 | | | |

En effet, lorsque l'on insère un solvant dans le matériau, la cage se déforme avec un rapprochement des trimères selon l'axe c et un éloignement dans les directions a et b, ce qui provoque une augmentation globale du volume de la cage (figure 58). Finalement, la flexibilité de ces solides hybrides est remarquable, mais cependant comparable à celle de certains polymères. La principale différence concerne la cristallinité des solides hybrides, les polymères étant amorphes. Enfin, contrairement aux polymères, le gonflement se produit dans les solides hybrides de manière anisotrope.

**Tableau 13 : Structures « MIL » de quelques carboxylates de fer(III) selon l'invention.**

| **Nanosolide MIL-n** | **Fraction organique** | **Formule** |
|---|---|---|
| **MIL-88A** | Acide fumarique | Fe₃OX[O₂C-C₂H₂-CO₂]₃.nH₂O |
| **MIL-88B** | Acide téréphthalique | Fe₃OX[O₂C-C₆H₄-CO₂]₃.nH₂O |
| **MIL-89** | Acide muconique | Fe₃OCl[O₂C-C₄-H₄-CO₂]₃.nH₂O |
| **MIL-100** | Acide 1,3,5-Benzene tricarboxylique (Acide 1,4-BTC) | Fe₃OX[C₆H₃-[CO₂]₃.nH₂O |
| **MIL-101** | Acide téréphthalique | Fe₃OX[O₂C-C₆H₄-CO₂]₃.nH₂O |

**Tableau 14 : caractéristique des structures « MIL » de carboxylates de fer (III).**

| **MIL-n** | **% de Fer*** | **Diamètre des pores (Å)**** | **Flexibilité** | **Base métallique** |
|---|---|---|---|---|
| **MIL-88A** | 30,8% | 6 | oui | Trimère d'octaèdre |
| **MIL-88B** | 24,2% | 9 | oui | Trimère d'octaèdre |
| **MIL-89** | 26,2% | 11 | oui | Trimère d'octaèdre |
| **MIL-100** | 27,3% | 25-29 | non | Trimère d'octaèdre |
| **MIL-101** | 24,2% | 29-34 | non | Trimère d'octaèdre |

| | | | | |
|---|---|---|---|---|
| * % théorique de fer en phase sèche ** taille de pore calculé à partir des structures cristallographiques | | | | |

### Example 13 : absorption et relarge de NO par des MOFs à base de fer

Des tests d'encapsulation et de relargage ont été realisés avec quelques MOFs ayant des caracteristiques diverses : rigide de grande capacité (MIL100-Fe), présentant une activité redox (MIL100-Fe), flexibles (MIL88), flexibles et substitués (sur le ligand) (MIL88-FeNO₂).

En, effet, si la délivrance de NO en quantité importante sur une courte période est facile à produire avec les zéolithes, les solides hybrides poreux de type MIL-n à base de métaux à haut degré d'oxydation (+3) semblent avoir le profil idéal pour le relargage prolongé de NO. En effet, les inventeurs ont montré précédemment que le MOF à larges pores dénoté MIL-100 constitué de trimères d'octaèdres de chrome ou de fer relié par des acides trimésiques, est stable, même après le départ de l'eau coordinée sur les centres métalliques. Cette dernière est facilement évacuée par chauffage sous vide et laisse place à des centres métalliques insaturés et accessibles (métal en coordinence cinq). La plupart des solides MIL au fer possèdent ce type de trimères et donc peuvent tous potentiellement adsorber sur leurs centres métalliques des molécules organiques possédant un caractère de donneur d'électrons (base de Lewis), tel que NO via le doublet libre situé dans les orbitales 5σ.

### Chargement en oxyde d'azote (NO) :

Les matériaux MOFs préalablement activés sont exposés à environ 2 bars de NO (99,5%, commercialisé par la société Air Liquide) pendant 30 minutes. Ils sont ensuite évacués sous vide (pour éviter la libération du NO physisorbé et donc le phénomène dit de « initial burst » correspondant à une très grande quantité de NO libéré dès les premières minutes de relargage) et placés sous atmosphère d'argon sec. Cette dernière opération (évacuation/argon) est répétée 3 fois pour s'assurer que tout le NO physisorbé a été éliminé.

### Mesure des isothermes d'adsorption/désorption de NO

Les mesures d'adsorption/désorption de NO sont effectuées à l'aide d'un appareil de type gravimétrique thermostaté pour éliminer tout effet de l'environnement extérieur. Une microbalance CI, commercialisée par la société CI Electronics Ltd) est utilisée (sensibilité : 0,1 µg, reproductibilité de la mesure de la masse : 0,01%). La pression est mesurée par deux sondes BOC Edwards Active (domaine de mesure : 1x10⁻⁸-1x10⁻² et 1x10⁻⁴-1x10³ mbar). L'échantillon de MOF (-50mg) est préalablement activé à la température requise (voir plus haut) à 2x10⁻³ mbar jusqu'à ce que plus aucune perte de masse ne soit observée. L'échantillon est ensuite refroidi à la température de mesure et conservé a cette température soit par un bain d'eau thermostaté (température précise à ±0,02 K), soit par trempage dans l'azote liquide. La contrebalance est maintenue à la même température que l'échantillon pour minimiser les effets de différence de température sur la lecture de la température, elle-même mesurée à l'aide d'un thermocouple de type K placé proche de l'échantillon (< 5 mm). La variation de température de l'échantillon durant la mesure est inférieure à 0,1 K.Le gaz NO sec (Air Liquide, 99,5 %) est introduit dans le système jusqu'à ce que la pression désirée soit atteinte, et la prise de masse mesurée en fonction du temps jusqu'à stabilisation.

De cette façon, une isotherme d'adsorption est obtenue en incrémentant la pression et en relevant le gain de masse de l'échantillon à l'équilibre. La désorption de NO est effectuée en diminuant graduellement la pression jusqu'à la valeur désirée. (2 x 10⁻³ mbar).

### Quantification du relargage de NO par chimiluminecsence

Les mesures de NO sont réalisées à l'aide d'un analyseur « Sievers NOA 280i chemiluminescence Nitric Oxide Analyzer ». L'instrument est calibré en passant de l'air dans un filtre zéro (Sievers, < 1 ppb NO) et 89,48 ppm de NO gazeux (Air Products, balance azote). Le flux gazeux est fixé à 180 mL/min avec une pression dans la cellule de 8,5 torr et une pression d'oxygène de 6,1 psi.

Pour mesurer le relargage de NO par un échantillon sous forme de poudre, de l'azote gazeux d'humidité connue (11% d'eau par passage du flux gazeux sur une solution aqueuse de LiCl) est passé sur la poudre et le flux gazeux résultant envoyé vers l'analyseur, et la quantité de NO (en ppm et ppb) enregistrée. Cette approche est valable par exemple pour des applications cutanées, où le solide est en contact avec la peau et la libération du NO se réalise en présence de l'humidité de la peau.

Pour des applications où le solide est en contact avec le sang (tubes, cathéters, etc.), la présence d'un milieu aqueux est nécessaire. Pour de telles applications, le relargage de NO par un échantillon sous forme de poudre a été étudiée dans un fluide physiologique simulé. Ainsi, le solide chargé en NO (50mg + NO) est suspendu dans 4 mL d'un tampon phosphate saline (pH -5,5 ; PBS) à 22°C sous agitation. La quantité de NO libéré à différents temps est analysée par l'analyseur « Sievers NOA 280i chemiluminescence Nitric Oxide Analyzer ».

### 13.1. Solide MIL10(Fe)

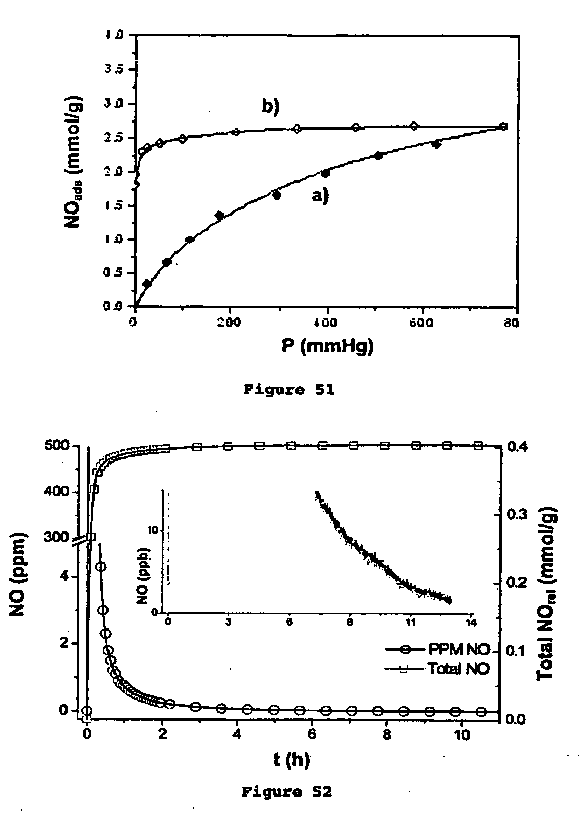

Le solide MIL100(Fe) adsorbe donc de fortes quantités de NO (2-4 mmol.g⁻¹) à température ambiante (environ 20°C)(figure 51) et le relargue lentement et très partiellement (sous flux d'humidité de 11%) comme le montrent les résultats préliminaires obtenus avec les solides MIL-100(Fe) (figure 52). Les profils de relargage de NO sous pression de vapeur d'eau sont, en outre, très intéressants.

La figure 51 représente l'isotherme d'adsorption de NO (NO_{ads} en mmol/g) à la température de 298 K pour le carboxylate de fer MIL-100(Fe) activé à 120°C pendant une nuit. Cette figure représente la quantité de NO adsorbé (courbe (a)) et désorbé (courbe (b)) en fonction de la pression P (en mmHg).

La figure 52 représente le profil de relargage de NO (NOᵣₑₗ en mmol/g) sous pression de vapeur du solide MIL-100(Fe). Le NO est représenté en ppm (ou « partie par million ») ou en ppb (ou « partie par milliard ») en fonction du temps t en heure.

On considère que le relargage de NO à un niveau utile sur le plan biologique est fini lorsque le taux de libération est inférieur à quelques ppb/minutes. Notons aussi que tout le gaz NO n'est que très partiellement relargué dans ces conditions (11% d'eau dans un flux de gaz neutre), et qu'il reste à peu près 75 % du NO encore adsorbé. Il restera donc à voir avec quelle vitesse ce dernier va se désorber lors de tests réels, i.e. lors de la mise en contact du solide avec le milieu physiologique (sur la peau ou en contact avec le sang).

Dans un second temps, les inventeurs ont réduit partiellement le fer(III) du composé MIL-100(Fe) par activation sous vide primaire (12 heures à 250°C). La spectroscopie infrarouge a montré que dans ces conditions, cela conduisait à la réduction d'environ 15-20 % du fer(III) en fer(II). Les métaux de transition de faibles degrés d'oxydation, tel que le fer(II), possèdent des électrons supplémentaires dans les orbitales d. Il est connu que le transfert électronique des orbitales d du métal vers les orbitales 2π* de molécules telles que NO et CO renforce la liaison métal-adsorbat (phénomène de rétro-donation) et stabilise ainsi les espèces coordinées sur le centre métallique. La quantité de NO augmente considérablement avec l'introduction de fer(II) (4,5 mmol.g⁻¹ à 1 bar au lieu de 2,5 mmol.g⁻¹ pour le solide pur fer(III)), car celui-ci est susceptible d'interagir avec plus d'une molécule de NO par centre métallique (figure 53). De plus, le relargage est alors plus lent, la libération de NO étant toujours présente après 17 heures au lieu de 12 heures avec l'analogue non réduit. Notons ici que la quantité totale de NO relarguée (<1 mmol.g⁻¹) est encore une fois largement inférieure à celle adsorbée (2,5-4,5 mmol.g⁻¹). Cela provient de la présence de sites très forts d'adsorption, que l'eau sous forme de vapeur (11% d'humidité dans le gaz neutre) ne parvient pas à désorber. Des tests en milieu aqueux permettront de connaître les performances réelles de ces solides.

La figure 53 représente, à gauche : l'isotherme d'adsorption de NO à 298 K pour le MIL-100(Fe) activé à 250°C sous vide pendant une nuit ; à droite : le profil de relargage de NO sous pression de vapeur du solide MIL-100(Fe) activé à 250°C sous vide pendant une nuit.

### 13.2 Solides MIL88A et MIL88B

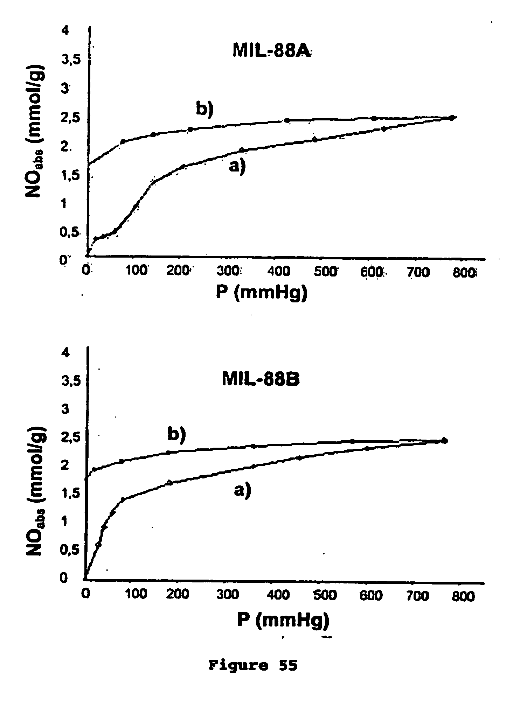

Finalement, les inventeurs ont testé l'adsorption et le relargage de NO à partir des phases flexibles MIL-88A et MIL-88B. Ces solides possèdent les mêmes trimères d'octaèdres que le composé MIL-100 mais avec une structure flexible. On peut noter ici que les inventeurs ont prouvé précédemment que ces deux composés gonflent en présence de liquides, mais ne sont pas poreux sous leur forme sèche (figure 54). Il n'était donc pas du tout évident de penser que ces solides allaient adsorber le gaz NO. Les inventeurs ont observé une adsorption de près de 2,5 mmol.g⁻¹ de NO à 298 K et à des pressions inférieures à 1 atmosphère (figure 55).

La figure 54 représente la vue schématique du phénomène de respiration dans les solides MIL-88 (-A, -B, -C et -D).

La figure 55 représente les isothermes d'adsorption de NO à 298 K pour les carboxylates de fer MIL-88A(Fe) et MIL-88B(Fe) activés à 150°C sous vide pendant une nuit. La quantité de NO (NO_{abs} en mmol/g) adsorbé (courbe (a)) et désorbé (courbe (b)) est représentée en fonction de la pression P (en mmHg).

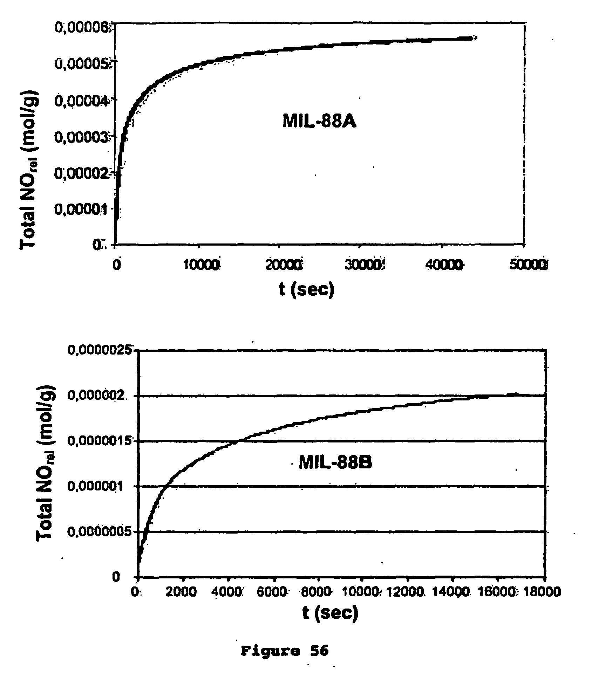

Le relargage de NO est ensuite testé sous pression de vapeur d'eau (11% d'humidité dans un gaz neutre). Dans les deux cas, la quantité de NO relarguée est encore plus faible qu'avec le solide MIL-100 (<0,055 et 0,002 mmol.g⁻¹) en comparaison avec les quantités adsorbées (2,5 mmol.g⁻¹) (figure 56), ce qui laisserait penser, à première vue, que le gaz est beaucoup plus fortement adsorbé que sur le composé MIL-100(Fe). C'est la première fois que l'on observe une si faible proportion de NO relargué avec un solide poreux ou un polymère.

La figure 56 représente les profils de relargage de NO sous pression de vapeur d'eau des solides MIL-88A(Fe) (en haut) et MIL-88B(Fe) (en bas) activés à 150°C pendant une nuit. La quantité de NO relargué (NOᵣₑₗ en mol/g) est exprimée en fonction du temps t (en secondes).

Pour des applications ou le solide est en contact avec le sang (tubes, cathéters, etc.), et donc en présence d'un milieu aqueux, cela va sans doute se traduire par un relargage extrêmement lent (quelques jours), ce qui, associé à la composition *a priori* biocompatible de ces solides (fer, acides carboxyliques) rend ces solides hautement intéressants pour les applications recherchées. Une explication possible à ce comportement singulier pourrait être la suivante : les phases flexibles MIL-88 sont « fermées » après vidage des pores par chauffage et donc sans porosité accessible pour les gaz habituels (H₂, CO₂, CH₄, N₂, etc.). Les mesures d'adsorption de N₂ à 77 K ont montré précédemment la quasi-absence d'adsorption dans ces solides. Si le gaz NO est tout de même adsorbé en quantité importante, sans doute sur Les centres métallique insaturés, c'est que l'interaction entre ce gaz et le fer est beaucoup forte qu'avec les autres types de molécules gazeuses, en tout cas suffisante pour « ouvrir » un peu le matériau. À ce stade, NO est rentré dans le solide chimisorbé sur le centre métallique mais ces derniers n'ayant que très peu ouverts leurs pores, vont laisser très difficilement l'eau diffuser dans les pores et donc cette dernière connaît les plus grandes difficultés à chasser le NO du solide. En milieu aqueux, lors de tests réels, la libération de NO va sans doute être conditionnée par l'hydrophobicité et la stabilité de ces phases flexibles. La possibilité de changer presque à souhait l'espaceur organique des phases MIL-88 va donc en théorie nous permettre de moduler la cinétique de relargage du NO en milieu physiologique.

La libération a été aussi étudiée dans des conditions plus proches de conditions réelles dans le plasma. Ainsi, le solide chargé en NO a été place dans 4mL d'un tampon phosphate (pH -5.5) a 22°C sous agitation.

La figure 71 représente les profils de relargage de NO sous pression de vapeur d'eau (courbe (a)) et dans le tampon phosphate (courbe (b)) de solide MIL-88A(Fe). La quantité de NO relarguée (NOᵣₑₗ en mmol. g⁻¹, à gauche, et ppm NO, à droite) est exprimée en fonction du temps t (en heures).

La quantité libérée est beaucoup plus importante quand la libération se produit dans le PBS que sous flux de vapeur d'eau ; ce qui est raisonnable en considérant que le contact avec le milieu liquide est plus important (figure 71). De cette façon, bien l'eau bien les phosphates présents dans le milieu pourront déplacer le gaz adsorbé/coordonné a métal.

Il y a une forte libération de NO dans les premières 2 heures et après la libération est maintenue dans des concentrations biologiquement actives (>10 ppb) jusqu'à 20 heures. Ca peut être intéressant pour avoir un effet de choque dans un premier temps (anticoagulant par exemple) et le maintenir sur quelques heures.

Concernant le solide MIL-88B(Fe), la quantité libérée est plus faible que celle libérée pour le MIL-88A(Fe). La quantité de NO libéré est très faible (0,002 mmol.g⁻¹), ca soit dans le flux gazeux ou dans le PBS (figure 58). La libération à des concentrations actives est très rapide (1 heure dans le PBS et 4 heures dans le flux gazeux).

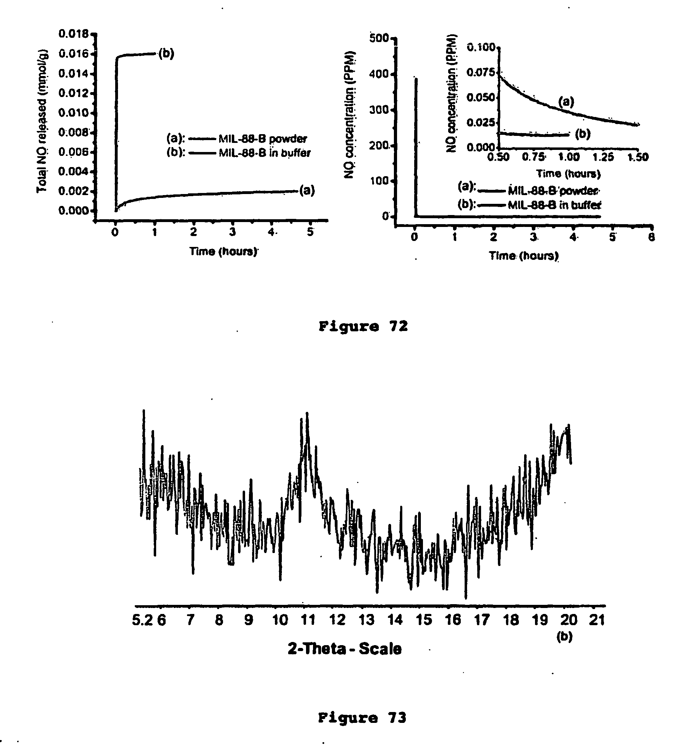

La figure 72 représente les profils de relargage de NO sous pression de vapeur d'eau (courbe (a)) et dans le tampon phosphate (courbe (b)) de solide MIL-88B(Fe). La quantité de NO relargué (NOᵣₑₗ en mmol. g⁻¹, a gauche, et ppm NO, a droite) est exprimée en fonction du temps t (en heures).

### 13.3. Solide MIL-88A-nano Fe₃O[(C₄H₂-(CO₂₎₂]₃.X.nH₂O (X=F, Cl, OH)

### Synthèse

Les conditions de synthèse microondes sont les suivantes :
270 mg (1 mmol) de FeCl₃.6H₂O, 116 mg d'acide fumarique (1,0 mmol, Acros 99%) dispersés dans 30 mL de l'eau distillée, le tout laissé dans un corps en téflon 2 min à 100°C avec une rampe de chauffage de 1 min (puissance 1600W).

Le solide est récupéré par centrifugation à 10000 rpm pendant 10 min.

200 mg du produit sont suspendus dans 100 mL de l'eau distille pour échanger l'acide fumarique qui reste libre. Le solide hydraté est récupéré par centrifugation à 10000rpm pendant 10min.

La figure 73 représente le diffractogramme des rayons X du solide MIL-88A-nano obtenu par synthèse microondes.

La taille de particule monodisperse mesurée par diffusion de lumière est 120 nm.

### Adsorption NO

50. mg de nanoparticules du MIL-88A-nano préalablement activés, sous vide a température ambiante (environ 20°C)pendant 5h et sous vide a 150°C pendant la 15 heures, sont exposés à environ 2 bars de NO 99,5%, commercialisé par la société Air Liquide) pendant 30 minutes (voir exemple 13).

La quantité de NO adsorbée, 2,5 mmol.g⁻¹, est considérable et tout a fait comparable avec celle obtenu pour la même structure avec une taille de cristallite plus grande (exemple précédente, taille de particule - 5 microns).

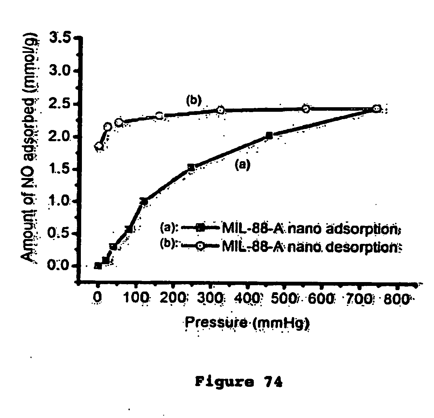

La figure 74 représente les isothermes d'adsorption de NO à 298 K pour les carboxylates de fer MIL-88A(Fe)-nano activé à 150°C sous vide pendant une nuit. La quantité de NO (NO_{abs} en mmol.g⁻¹) adsorbé (courbe (a)) et désorbé (courbe (b)) est représentée en fonction de la pression P (en mmHg).

### Libération de NO

Le relargage de NO est ensuite testé sous pression de vapeur d'eau (11% d'humidité dans un gaz neutre).

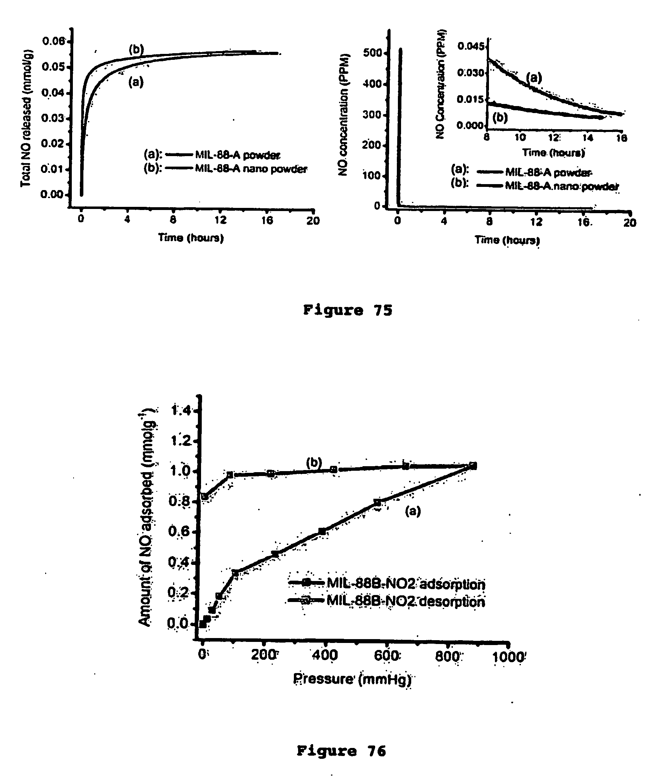

La figure 75 représente les profils de relargage de NO sous pression de vapeur d'eau des solides MIL-88A(Fe)-nano (120 nm, courbe (b)) et MIL-88A(Fe) (5 microns, courbe (a)). La quantité de NO relargué (NOᵣₑₗ en mmol.g⁻¹, à gauche, et ppm NO, à droite) est exprimée en fonction du temps t (en heures)

Dans les deux cas, matériaux nano et micrométrique, la quantité de NO relarguée est comparable (0,055 mmol.g⁻¹, Figure 75). MIL-88A(Fe) micrométrique semble avoir une libération plus lente que MIL-88A(Fe)-nano dans les premières 10 heures. Cet effet est probablement dû à une distance caractéristique de diffusion du NO plus petite dans les nanoparticules de MIL88A par rapport au MIL88A micrométrique.

### 13.4. Solides MIL88B modifiés avec différentes groupes fonctionnels

La structure cristalline flexible MIL-88B présente des isotypes par la modification de l'espaceur organique avec différents groupes fonctionnels. Ainsi, ces groupes fonctionnels vont substituer un ou plusieurs hydrogènes de l'espaceur organique (acide téréphtalique), en modulant ainsi l'hydrophobicité et la stabilité de ces phases flexibles, par conséquence l'adsorption et libération de gazes biologiques. De plus, les groupes accepteurs d'électrons pourront peut être de créer des nouvelles interactions avec les gazes biologiques (bases de Lewis).

Les inventeurs ont testé l'adsorption et le relargage de NO à partir des phases flexibles du type MIL-88B, à base des ligands organiques : nitrotéréphthalate (MIL88B-NO2) et 2,5-dihydroxotéréphthalate (MIL88B-20H).

### Adsorption de NO

Les deux matériaux ont montré une capacité d'adsorption similaire (1 mmol.g⁻¹), diminuée en comparaison avec le solide MIL-88B non modifié. Cet effet peut être expliqué par l'élimination incomplète de l'eau coordonnée.

La figure 76 représente les isothermes d'adsorption de NO à 298 K pour les carboxylates de fer MIL-88B(Fe)-NO2 activé à 150°C sous vide pendant une nuit. La quantité de NO (NO_{abs} en mmol.g⁻¹) adsorbé (courbe (a)) et désorbé (courbe (b)) est représentée en fonction de la pression P (en mmHg).

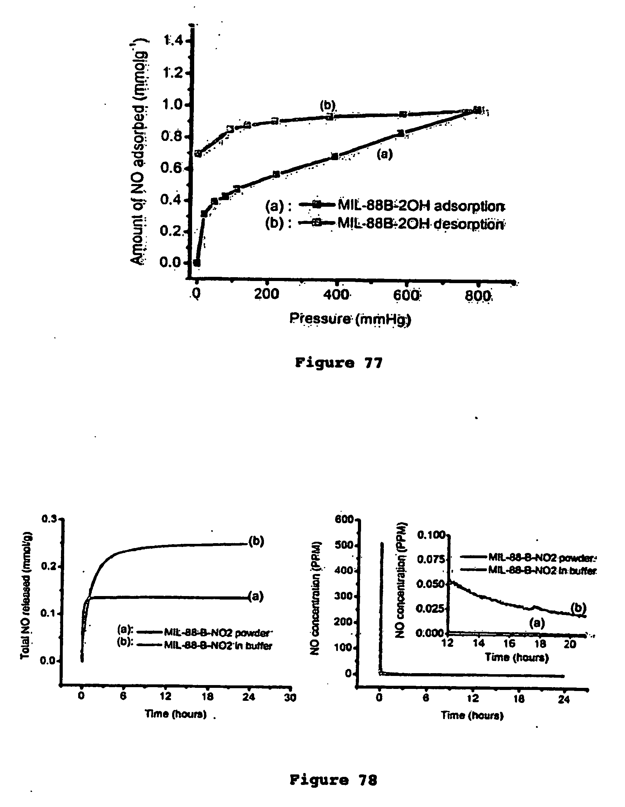

La figure 77 représente les isothermes d'adsorption de NO à 298 K pour les carboxylates de fer MIL-88B(Fe)-20H activé à 80°C sous vide pendant une nuit. La quantité de NO (NO_{abs} en mmol.g⁻¹) adsorbé (courbe (a)) et désorbé (courbe (b)) est représentée en fonction de la pression P (en mmHg).

### Libération de NO

La fonctionnalisation avec de groupes nitro ou dihydroxo dans le solide du type MIL-88B, permet un important ralentissement de la libération de NO dans le milieu PBS.

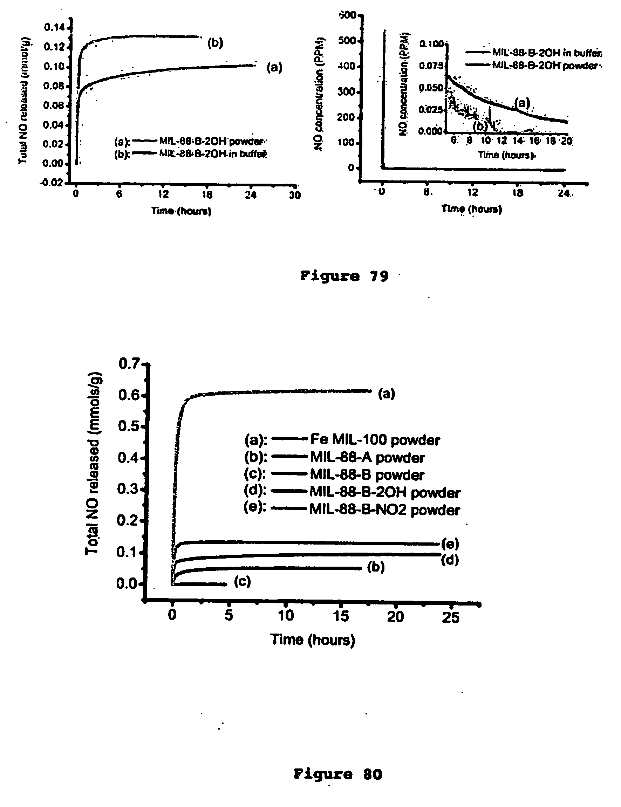

Ainsi, on observe la libération du NO à des concentrations biologiquement actives (>10 ppm) jusqu'à 11 et 24 heures avec MIL-88B-20H et MIL-88B-NO2 respectivement (figures 78 et 79), en comparaison avec le solide non modifié MIL-88B qui libère sa charge en 1 heure. De même, la quantité de NO libérée est aussi plus importante dans le MIL-88B-20H et MIL-88B-NO2 (0,13 et 0,25 mmol.g⁻¹, respectivement) par rapport avec le MIL-88B non modifié(0,01 mmol.g⁻¹).

On observe que quand la libération du NO est menée à bien dans la solution PBS, la quantité libérée est beaucoup plus importante que sous conditions de flux gazeux (Tableau 15).

La figure 78 représente les profils de relargage de NO sous pression de vapeur d'eau (courbe (a)) et dans le tampon phosphate (courbe (b)) de solide MIL-88B(Fe)-NO2. La quantité de NO relargué (NOᵣₑₗ en mmol.g⁻¹, à gauche, et en ppm NO, a droite) est exprimée en fonction du temps t (en heures).

La figure 79 représente les profils de relargage de NO sous pression de vapeur d'eau (courbe (a)) et dans le tampon phosphate (courbe (b)) de solide MIL-88B(Fe)-20H. La quantité de NO relargué (NOᵣₑₗ en mmol.g⁻¹, à gauche, et en ppm NO, à droite) est exprimée en fonction du temps t (en heures).

### Comparaison des MILs

On peut conclure au vu des résultats (Tableau 15 et Figure 80), que l'on peut moduler la capacité et la cinétique d'adsorption et de libération des carboxylates de fer en fonction de leur caractère rigide, flexible ou fonctionnalisé. Si le solide mésoporeux adsorbe et libère les plus grandes quantités de NO, les phases flexibles adsorbent moins mais libèrent avec des cinétiques très différentes en fonction du ligand choisi. Ainsi, les solides non modifiés libèrent très rapidement (<1h) une très faible quantité tandis que l'introduction d'une fonctionnalité permet non seulement d'augmenter la quantité libérée mais sur des temps caractéristiques beaucoup plus grands (11 et 24h). Cela vient sans doute de la plus grande facilité qu'a l'eau à diffuser dans ces phases à cause d'une ouverture de pores plus importantes et d'un caractère hydrophile des substituants (OH, NO₂).

**Tableau 15: Capacité et cinétique d'adsorption et de libération des carboxylates de fer en solution PBS et sous conditions de flux gazeux.**

| | NO ads (mmol/g) | NO libéré flux gazeux (mmol/g) | temps de libération (h) flux gazeux (>10ppb) | NO libéré PBS (mmol/g) | temps de libération (h) PBS (> 10ppb) |
|---|---|---|---|---|---|
| MIL88A | 2,5 | 0,055 | 18 | 0.5 | 20 |
| MIL88A -nano | 2,5 | 0,06 | 15 | 0.01 | 1 |
| MIL88B | 2,5 | 0.002 | 4 | 0.016 | 1 |
| MIL88B -NO₂ | 1,05 | 0,14 | 7 | 0.25 | 24 |
| MIL88B -2OH | 1 | 0,10 | 20 | 0.14 | 11 |
| MIL100 Fe | 2,5 | 0,4 | 10 | 0.4 | 24 |
| MIL100 -250°C | 4,5 | 0,6 | 15 | N/A | N/A |
| MIL22 | 0,18 | 0,0016 | 1 | N/A | N/A |

La figure 80 représente les profils de relargage de NO sous pression de vapeur d'eau des solides MIL-100Fe (courbe (a)), MIL-88A (courbe (b)), MIL-88B (courbe (c)), MIL-88-2OH (courbe (d)) et MIL-88B-NO2 (courbe (e)). La quantité de NO relargué (NOᵣₑₗ en mmol.g⁻¹) est exprimée en fonction du temps t (en heures).

### Exemple 14 : adsorption et libération de NO à partir du diphosphonate de titane MIL-22 (Ti₃O₂(H₂O)₂(O₃P-(CH₂)-PO₃)₂.(H₂O)₂)

Le diphosphonate de titane MIL-22 a été obtenu suivant la méthode rapporté par C. Serre, G. Férey, Inorg. Chem. 1999, 38, 5370-5373 [60].

50 mg du MIL-22 préalablement activés, sous vide a 300°C pendant 15 heures, sont exposés à environ 2 bars de NO 99,5%, commercialisé par la société Air Liquide) pendant 30 minutes (voir exemple 13).

La quantité de NO adsorbé théorique est de -4 mmol.g⁻¹. Par contre, la capacité d'adsorption de NO expérimentale n'est que de 0,18 mmol.g⁻¹. Cette différence peut être expliquée car les conditions d'activation sont insuffisantes pour éliminer la totalité de l'eau coordonnée. Ainsi, capacités plus importantes pourront être obtenues avec des conditionnes d'activation du solides tels que 500°C sous vide pendant 16 heures.

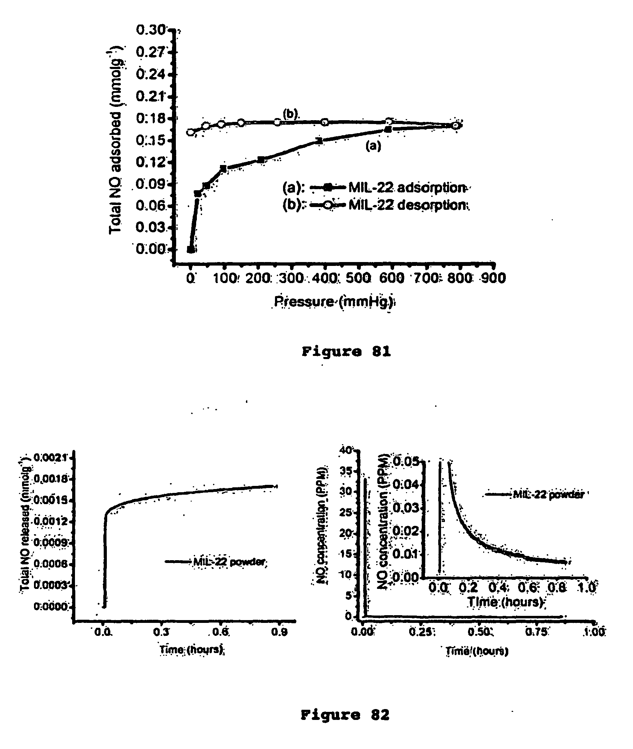

La figure 81 représente les isothermes d'adsorption de NO à 298 K pour le solide MIL-22 activé à 350°C sous vide pendant une nuit. La quantité de NO (NO_{abs} en mmol.g⁻¹) adsorbé (courbe (a)) et désorbé (courbe (b)) est représentée en fonction de la pression P (en mm Hg).

La figure 82 représente les profils de relargage de NO par lee solide MIL-22 sous pression de vapeur d'eau. La quantité de NO relargué (NOᵣₑₗ en mmol.g⁻¹ à gauche et en ppm NO à droite) est exprimée en fonction du temps t (en heures).

La libération partielle du NO (0.0016 mmol/g) est réalisé dans des concentrations biologiquement actives pendant 0,8 heures.

### Exemple 15 : Mesure des isothermes d'adsorption de CO pour le solide MIL-100(Fe)

Les mesures d'adsorption de CO sont effectuées à 303K jusqu'à 2 bars dans un système de dosage de gazes mis au point au laboratoire et connecté à un appareil de type gravimétrique thermostaté (Rubotherm Präzisionsmeβtechnik GmbH). L'échantillon du MIL-100(Fe) (500mg) est préalablement activé à la température requise (100°C ou 250°C) sous vide (à 2x10⁻³ mbar) pendant 12 ou 20 heures. Le gaz CO sec (Air Liquide, 99,9 %) est introduit dans le système jusqu'à ce que la pression désirée soit atteinte, et la prise de masse mesurée en fonction du temps jusqu'à stabilisation.

De cette façon, une isotherme d'adsorption est obtenue en incrémentant la pression et en relevant le gain de masse de l'échantillon à l'équilibre.

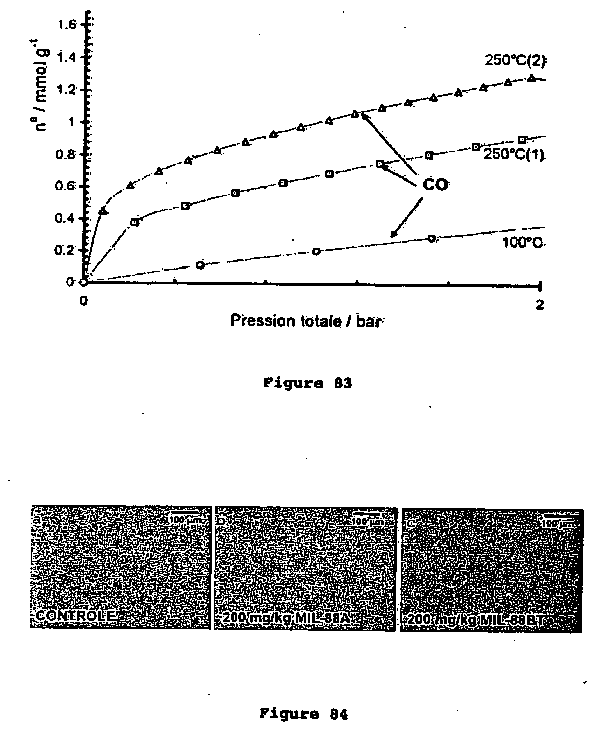

La figure 83 représente l'isotherme d'adsorption de CO (CO_{ads} en mmol/g) à la température de 303 K en fonction de la pression P (en bar) pour le carboxylate de fer MIL-100(Fe) activé à 100°C pendant une 12h (courbe 100°C), 250°C pendant 12h (courbe 205°C (1)) et 250°C pendant 20h (courbe 250°C (2)).

Le solide MIL-100(Fe) adsorbe de quantités considérables de CO à température ambiante (0,4-1,3 mmol.g⁻¹) et à base pression (jusqu'à 2 bars) (Figure 83). La capacité d'adsorption du CO dans le MIL-100(Fe) augmente drastiquement quand le fer(III) du composé MIL-100(Fe) est réduit partialement par activation a 250°C sous vide primaire (12 et 20 heures à 250°C). La spectroscopie Infra-Rouge a montré que dans ces conditions, cela conduisait à la réduction d'environ 15-20 % du fer(III) en fer(II). Les métaux de transition de faibles degrés d'oxydation, tel que le fer(II), possèdent des électrons supplémentaires dans les orbitales d. Il est connu que le transfert électronique des orbitales d du métal vers les orbitales 2π* de molécules telles que NO et CO renforce la liaison métal-adsorbat (phénomène de rétro-donation) et stabilise ainsi les espèces coordinnées sur le centre métallique. La quantité de CO augmente considérablement avec l'introduction de fer(II) (1,3 mmol.g⁻¹ à 2 bars au lieu de 0,4 mmol.g⁻¹ pour le solide pur fer (III)), car celui-ci est susceptible d'interagir avec plus d'une molécule de CO par centre métallique.

### Exemple 16 : Essais in vivo de toxicité des carboxylates de fer (III)

### a) Carboxylates de fer testés

Les deux solides carboxylates de fer suivants (synthétisés selon les modes opératoires de l'exemple 1) sont respectivement testés :
3. MIL-88A(Fe) de composition Fe₃O[O₂C-C₂H₂-CO₂]₃·OH·*n*H₂O
4. MIL-88Btnano(Fe) de composition Fe₃O[O₂C-C₆(CH₃)₄-CO₂]₃·OH·*n*H₂O

### b) Tests de toxicité

L'étude de toxicité aiguë *in vivo* est effectuée sur des rats Wistar femelles de 4 semaines (125 g) en injectant par voie intraveineuse aux rats des doses croissantes (50, 100 et 200 mg/kg) de nanoparticules MIL-88A (de 210 nm) et MIL-88Bt (de 100 nm) suspendus dans 0,5 mL d'une solution de glucose 5%.

Les nanoparticules sont stables dans ce milieu.

Le temps de stabilité de ces suspensions est réduit à quelques minutes quand la concentration de particules est maximale (200 mg/kg, 25 mg/0,5mL). Pour cette raison, les prélèvements sont réalisés sous agitation douce des suspensions de nanoparticules. Il n'a pas été possible d'administrer des doses supérieures à 200 mg/kg, puisque le volume maximal injectable à des rats est 0,5 ml.

Les résultats sont prometteurs étant donné qu'aucun signe de toxicité majeur n'est observé après 7 jours d'essai. Les valeurs sériques de l'albumine, cholestérol et transaminases (ASAT/ALAT) ne présentent pas de variation significative après 7 jours d'essai et le poids des organes par rapport au poids corporel ne varie pas significativement (Tableau 16).

**Tableau 16 : paramètres sériques mesurées 7 jours après l'introduction intraveineuses descarboxylates de fer MIL-88A(Pe) et MIL-88Bt(Pe).**

| | **Dosage (mg/kg)** | **Albumine (g/L)** | **CHOL (mmol /L)** | **ASAT/ ALAT** | **Poids organes/poids totale** | | |
|---|---|---|---|---|---|---|---|
| | | | | | **foie** | **reins** | **rate** |
| **Contrôle** | - | 44,2 | 2,5 | 2,5 | 0,041 | 0,009 | 0,004 |
| **MIL-88A** | 50 | 37,6 | 3 | - | 0,044 | 0,012 | 0,004 |
| **MIL-88A** | 100 | 46,0 | 2,2 | 2,5 | 0,041 | 0,009 | 0,004 |
| **MIL-88A** | 200 | 40,2 | 2,9 | 2,6 | 0,048 | 0,008 | 0,004 |
| **MIL-88Bt** | 50 | 39,5 | 2,5 | - | 0,048 | 0,010 | 0,003 |
| **MIL-88Bt** | 100 | 42,1 | 2,6 | 2,4 | 0,046 | 0,008 | 0,003 |
| **MIL-88Bt** | 200 | 38,5 | 2,6 | 2,5 | 0,044 | 0,008 | 0,004 |

Les coupes histologiques du foie sont observées par coloration du Proust (fer en bleu) et présentées en figure 84. elles montrent une accumulation de fer dans le foie. Bien qu'il soit nécessaire d'effectuer une étude plus approfondie sur les effets de ces solides dans l'organisme à long terme, ces résultats sont très prometteurs, et permettent d'envisager des applications biomédicales pour ces matériaux.

Des études de toxicité aiguë et subaiguë plus approfondies ont été réalisées.

Les animaux utilisés pour l'expérimentation sont des rats Wistar femelles de quatre semaines avec un poids de 161,36 ± 16,1 g.

Tous les essais ont été réalisés dans l'animalerie de l'Université de Pharmacie dans des conditions de température et humidité, et après 3 jours d'adaptation des animaux a l'animalerie (3 jours).

Pour les tests de toxicité aiguë, une injection intrajugulaire unique des matériaux MIL-88A (150 et 500 nm), MIL-88Bt (50 et 140 nm) ou de glucose 5% (groupe témoin) est effectuée à 4 groupes (à respectivement 1 jour, 1 semaine, 1 mois et 3 mois) de 8 rats choisies au hasard et anesthésiés sous isoflurane.

L'évolution du poids des animaux ainsi que leur comportement ont été suivis.

Des prélèvements de sang dans la jugulaire sous anesthésie par isoflurane ont également été réalisés à différents temps : 1 et 3 jours, 1 et 2 semaines, 1, 2 et 3 mois. Le sérum a été isolé pour mesurer des paramètres sériques tels que IL-6 (interleukine 6), albumine, Fe sérique, PAS, GGT, bilirubine, cholestérol et transaminases.

Par ailleurs, chaque groupe d'animaux a été sacrifié après respectivement 1 jour, 1 semaine, 1 et 3 mois. Les animaux ont été anesthésiés à l'isoflurane puis la rate, les reins, le foie et le coeur ont été prélevés et conservés pour des études histologiques. Quatre fois ont également été utilisés pour réaliser une extraction microsomale afin de mesurer l'activation du cytochrome P450.

Pour les tests de toxicité subaiguë, une injection intrajugulaire par jour est effectuée pendant 4 jours consécutifs sur 26 rats répartis au hasard dans différents groupes où les animaux sont sacrifiés au bout de 5 ou 10 jours.

L'évolution du poids des animaux isolés ainsi que leur comportement alimentaire (mesure des quantités d'eau et de nourriture consommées) ont été suivis. Les urines et déjections ont également été récupérés.

Des prélèvements de sang dans la jugulaire ont également été effectués sur différents groupes de rat à 3 et 5 jours, 8 et 10 jours. Le sang subit le même traitement que pour l'essai de toxicité aigue et le sérum obtenu est destiné aux mêmes analyses.

Les jours des sacrifices, aux 5 et 10 jours, les animaux sont anesthésiés à l'isoflurane puis la rate, les reins, le foie, le coeur et les poumons sont prélevés et traités de même que pour l'essai de toxicité aiguë.

### c) Résultats

### Evolution du poids des animaux :

Les animaux ont été pesés tous les jours dans le but de comparer l'évolution du poids des différents groupes. Une moyenne a été effectuée pour chaque jour et dans chacun de ces groupes.

Pour les tests de toxicité subaiguë, l'augmentation du poids observée avec le groupe du glucose est légèrement diminuée quand on administre le matériau. Cette variation est plus évidente quand la dose administrée est plus élevée

Les études en toxicité aiguë montrent que l'administration des matériaux MIL-88A et MIL-88Bt ne produit pas de variation significative du poids au cours du temps.

### Evolution de la consommation d'eau et de nourriture :

En toxicité subaiguë, l'évolution est globalement similaire pour le groupe témoin et le groupe ayant reçu une injection de 25 mg/kg. Une différence plus marquée est observée dans le groupe ayant reçu la plus forte dose et se caractérise par une plus faible consommation de nourriture pendant l'étude. Cette observation est confirmée et en totale concordance avec les résultats obtenus par l'évolution du poids.

### Comparaison du poids des organes prélevés :

Résultats de toxicité subaiguë : il n'apparait pas de différence significative entre le poids de rate, reins et coeur des différents groupes. Le poids des poumons parait être légèrement augmenté à 5 jours comme à 10 jours.

Toxicité aiguë : on observe une augmentation du poids de la rate jusqu'à une semaine après l'administration, et un retour a la normale à 1 et 3 mois pour le MIL-88A et MIL-88Bt, respectivement. Le poids du foie augmente de façon importante quand les matériaux sont injectés, ce qui traduit peut être l'accumulation du fer dans le foie. On observe que la situation revient à la normale pour le MIL-88A après 3 mois, mais pas pour le MIL-88Bt ou le poids reste élevé.

### Dosage du cytochrome P450 dans les suspensions microsomales :

Le cytochrome P450 est un enzyme associé à la face interne du réticulum endoplasmique lisse qui est fortement impliqué dans la dégradation des molécules exogènes. Cet enzyme possède une très faible spécificité de substrat et est capable de catalyser la transformation de composés nouvellement synthétisés tels que les médicaments. La plupart des cytochromes P450 peuvent être induits ou réprimés, au niveau transcriptionnel, par différents xénobiotiques ; ceci est souvent la cause d'effets secondaires des médicaments. Le dosage de cet enzyme permet de déterminer si le matériau MOF utilisé est métabolisé par le cytochrome P450 auquel cas il activerait ou inhiberait son activité.

La quantité de cytochrome n'est interprétable qu'à condition d'avoir été rapportée à la quantité totale de protéines contenue dans chaque échantillon. Le dosage de protéines contenues dans l'échantillon a été effectué grâce à l'utilisation d'un kit BCA fourni par PIERCE (lot #HI106096). Cette méthode combine la réduction du Cu²⁺ en Cu⁺ par les protéines en milieu alcalin avec la détection colorimétrique, très sensible et sélective, du cation Cu⁺ grâce à un réactif contenant l'acide Bicinchoninique (BCA).

Le rapport entre la concentration de cytochrome et la quantité de protéines totale donne l'activité du cytochrome exprimée en mol.g⁻¹. Les résultats en toxicité aiguë montrent qu'il n'y a pas de grosse différence d'activité entre le groupe témoin négatif (ayant reçu le glucose) et le groupe « MIL-88A » dont le matériau n'est pas métabolisé par le Cyp450. Le matériau MIL-88Bt ne semble pas non plus être métabolisé par le Cyp450.

### Dosage de l'interleukine 6 dans le sérum :

L'interleukine 6 (IL-6) est une cytokine multifonctionnelle qui joue un rôle important dans la défense de l'hôte, les réponses immunitaires les fonctions des cellules nerveuses et l'hématopoïèse. Un niveau élevé d'IL-6 dans le sérum a par exemple été observé lors d'infections virales et bactériologiques, de trauma, de maladies auto-immunes, d'inflammation, ou de cancer.

Cette étude a pour de déterminer s'il existe une réaction inflammatoire après l'administration des nanoparticules de carboxylates de fer. Ainsi, il est possible de voir si le niveau d'IL-6 est augmenté par rapport aux groupes témoins (injection de glucose, donc réaction inflammatoire locale par la piqûre)**.**

Le dosage a été effectué par l'utilisation d'un kit « Quantikine, Rat IL-6 » fourni par les laboratoires R&D Systems.

Résultats de toxicité subaiguë : les variations ne sont pas significatives. Une augmentation du taux plasmatique observé (activation de la production d'IL-6) semble être dû phénomène de piqûre lors des injections qui produisent une inflammation locale, si on compare de façon isolée les différents groupes avec le groupe témoin (glucose).

Résultats de toxicité aiguë : les variations ne sont pas significatives et conduisent aux mêmes conclusions que dans le cas de la toxicité subaiguë.

### Dosage des paramètres sériques :

Tous les dosages ont été réalisés en utilisant des dispositifs automatiques. Quelques paramètres clés ont été déterminés pour évaluer les conséquences des injections de nanoparticules au niveau du foie, des taux de transaminases (Alanine amino transférase ou ALAT et aspartate amino transférase ou ASAT), phosphatases alcalines (PAS), γ-glutamate transférase (GGT), bilirubine, cholestérol, albumine et fer sérique.

Les résultats montrent que les niveaux sériques de l'ALAT sont tout à fait normaux, tout comme les niveaux de bilirubine (< 2 µmol/L) et de γ-glutamique transférase (<2 UI/L).

Les niveaux d'albumine sérique ont légèrement diminués après le premier jour d'injection pour les deux matériaux, en accord avec un processus inflammatoire local du à la piqûre et avec l'augmentation de la IL-6 observée précédemment. Après 3 jours, les niveaux reviennent à la normale.

Les niveaux sériques de l'ASAT sont augmentés un jour après l'injection, ce qui peut indiquer un processus de cytolyse. Toutefois, 3 jours après l'administration des nanoparticules, les valeurs retournent à la normale. De la même façon, la phosphatase alcaline se trouve augmentés après 1 jour, indiquant un processus de cytolyse, mais la situation revient à la normale après 3 jours. Le retour à la normale après 3 jours indique qu'il s'agit d'un processus de cytolyse transitoire et non permanent. Il n'y a donc pas de perte de fonction cellulaire.

Les niveaux de cholestérol sont normaux.

Les niveaux de fer sériques sont diminués en comparaison avec le groupe témoin, et cela est plus prononcé dans le groupe MIL-88A. Cela pourrait s'expliquer par une complexation du fer sérique par les nanoparticules. La situation revient à la normale 3 jours après l'administration.

Les paramètres sériques ont aussi été dosés à 1 semaine et d'après ces résultats, il n'y a plus de différence entre les 3 groupes au niveau du fer sérique ; les rats traités au MIL-88A et MIL-88Bt ont récupéré une concentration en fer sérique comparable à celle du groupe témoin. Par ailleurs, concernant les niveaux des autres paramètres sériques, il n'y a pas une différence significative en comparaison avec le groupe témoin.

### Coupes histologiques :

Les coupes histologiques, d'une épaisseur de 5 µm, sont effectuées dans un cryostat, déshydratées et colorées (coloration hématoxyline/éosine puis coloration au bleu Proust : coloration du fer en bleu).

Par l'observation des coupes histologiques, il est possible de déterminer la voie d'élimination des composés du matériau ou leur stockage dans certains organes : foie, reins, rate et poumons, le coeur étant utilisé comme témoin.

Résultats de toxicité aiguë : les coupes histologiques de foie montrent une accumulation du fer dans le foie après l'injection des matériaux qui est plus importante pour le solide MIL-88A. Le matériau semble être sous sa forme de nanoparticules non dégradées. L'accumulation est moins importante pour le matériau MIL-88Bt, ce qui peut signifier une plus faible captation pour le foie ou la réutilisation plus rapide du fer stocké. Apres 1 et 3 mois le contenu en fer dans la rate et le foie revient à la normale.

### Dosage du fer dans les suspensions injectées et dans les organes :

Le dosage du fer contenu dans les suspensions de MIL-88A et MIL-88Bt injectées aux animaux est effectué par spectrophotométrie UV-Visible à la longueur d'onde de 520 nm, par colorimétrie spécifique des ions ferreux avec la bipyridine (formation d'un complexe rouge), après solubilisation de l'oxyde de fer dans de l'acide sulfurique concentré, et réduction des ions ferriques en ions ferreux par l'acide ascorbique.

Le dosage du fer dans les organes est réalisé de même que pour le dosage en fer des suspensions expliqué précédemment, après broyage de l'organe à tester. Ce dosage permet de déterminer la voie d'élimination des composés du matériau ou leur stockage dans certains organes : foie, reins, rate et poumons, le coeur étant utilisé comme témoin.

### d) Conclusion

Lors des essais de toxicité, l'observation minutieuse des animaux n'a révélé aucun signe apparent de nocivité du matériau injecté. En effet, les animaux ont gardé un comportement tout à fait normal. Pendant les études, les animaux ont bien grossi en comparaison avec le groupe témoin, même si pour l'étude de toxicité subaiguë l'augmentation du poids est moins importante que pour le groupe témoin, probablement lié a l'administration consécutive des doses importantes. La consommation d'eau reste elle globalement normale dans l'essai de toxicité subaiguë.

Un dosage du cytochrome P-450 a permis d'observer l'état de l'activité du cytochrome P-450 sur une longue période. Ce cytochrome est connu pour sa capacité à métaboliser certains xénobiotiques. L'étude montre que le taux d'activité, bien que fluctueux, reste en dessous des valeurs observées sur les rats témoins qui ont reçu une injection de phénobarbital, activateur de cytochrome P-450, ce qui indique que les matériaux ne sont pas métabolisés par voie Cyp450, en concordance avec la polarité importante des ligands dicarboxyliques.

Les résultats sont très prometteurs et indiquent déjà que les matériaux MIL-88A et MIL-88Bt n'induisent aucun signe de toxicité sévère, même si des études de toxicité complémentaires doivent être effectuées. Le devenir et les effets des nanoparticules dans l'organisme sont en cours d'étude afin de mettre en relation le bénéfice apporté par ces matériaux par la vectorisation de médicaments délicats à encapsuler et présentant un grand potentiel thérapeutique. Des études similaires sont également en cours de réalisation avec d'autres nanovecteurs de structure et/ou de composition différentes.

### Exemple 17 : Comparaison performances différents solides poreux pour l'adsorption et libération de NO

Les matériaux MOFs présentent nombreuses avantages par rapport aux solides poreux inorganiques, les zéolites. Ainsi, la grande stabilité des zéolites dans des milieux aqueux ne permet pas son élimination par l'organisme, en produisant le stockage du produit dans le corps. De plus, la plus part de zéolites ont de l'aluminium dans sa structure, élément avec une très importante toxicité.

De même, la composition d'autres MOFs, à base de métaux bien connus pour leur toxicité (cobalt, nickel, chrome ou cuivre) ne permet pas envisager des applications dans le domaine médical ou cosmétique de ces MOFs. Contrairement à ces MOFs, les solides de l'invention possèdent une composition *a priori* peu toxique (Fe, Ti). En effet, dans l'exemple 16, nous démontrons l'absence de toxicité de deux carboxylates de fer (fumarate de fer et tetramethylterephthalate de fer) par des tests de toxicité *in vivo* sur de rats.

De plus, avec les solides de l'invention il est possible de contrôler leur vitesse de dégradation selon les applications recherchées. Par exemple, le solide MIL-100(Fe) est stable sous des conditions hydrothermales, tandis que le solide MIL-101(Fe) est dégradé rapidement en présence d'eau a température ambiante.

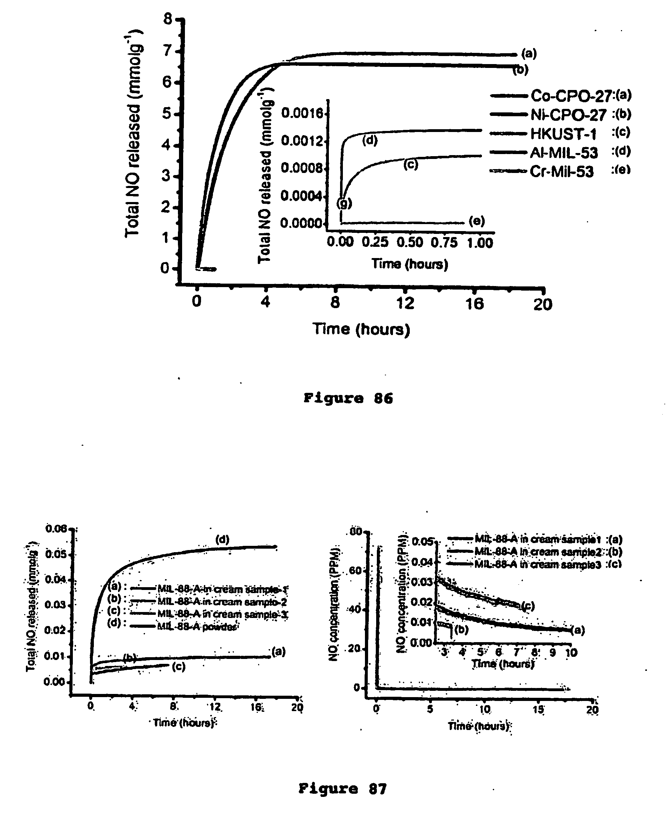

Les performances de nos MOFs ont été aussi comparés avec les MOFs rapportés dans la bibliographie (y compris dans la demande WO 2008/020218. Concernant leur capacité d'adsorption, les matériaux reportés dans cette invention ont montré des capacités toute a fait comparables a celles des autres MOF a base de métaux toxiques (4,5 mmol.g⁻¹) (Figure 84) avec des libérations contrôlées dans le temps. De plus, nous avons obtenu le solide CPO-27 a base de fer(II) (2,5-dihydroxotéréphthalate de fer), de composition biocompatible, dont la capacité d'adsorption escomptée de NO sera proche de celle des analogues a base de Ni et/ou Co (6-7 mmol.g⁻¹⁾ avec une libération controllée sur une très longue durée (Figure 86).

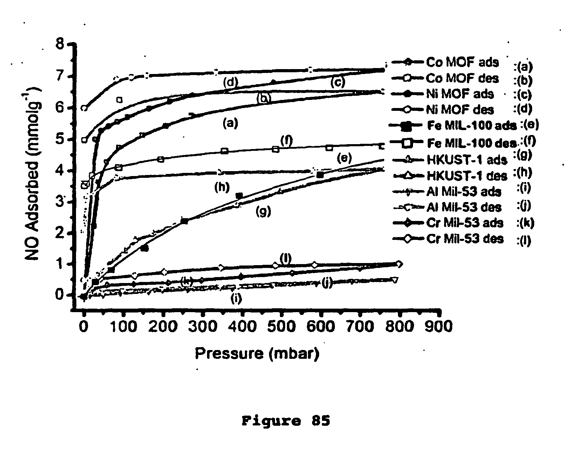

La figure 85 représente représente les isothermes d'adsorption de NO à 298 K pour les solides CPO-27(dihydroxotéréphthalate de Co) (courbes (a) et (b)), CPO-27(2,5-dihydroxotéréphthalate de Ni ; M₂(dhtp)(H₂O).xH₂O (M = Ni or Co dhtp = acide 2,5-dihydroxytéréphthalique, x-8)) (courbes (c) et (d)), MIL-100(trimésate de Fe) (courbes (e) et (f)), HKUST(trimésate de Cu) (courbes (g) et (h)), MIL-53(téréphthalate de Al) (courbes (i) et (j)) et MIL-53(téréphthalate de Cr) (courbes (k) et (1)).

La quantité de NO (NO_{abs} en mmol.g⁻¹) adsorbé et désorbé est représentée en fonction de la pression P (en mbar).

La figure 86 représente les profils de relargage de NO sous pression de vapeur d'eau du solide CPO-27(dihydroxotéréphthalate de Co) (courbe (a)), CPO-27(2,5-dihydroxotéréphthalate de Ni ; M₂(dhtp)(H₂O)xH₂O (M = Ni or Co dhtp = acide 2,5-dihydroxytéréphthalique acid, x-8)) (courbe (b)), HKUST-1 (trimésate de Cu) (courbe (c)), MIL-53(téréphthalate de Al) (courbe (d)) et MIL-53(téréphthalate de Cr) (courbe (e)). La quantité de NO relarguée (NOᵣₑₗ en mmol.g⁻¹) est exprimée en fonction du temps t (en heures).

### Exemple 18 : Formulation sous forme de crème comprenant un solide MOF selon l'invention

La crème utilisée est composée de 50% en poids de paraffine et 50% en poids de polyéthylène glycol (PEG), le deux sont mélanges à l'aide d'une pipette automatique pendant 30 secondes.

10% en poids du solide MIL-88A ou MIL-88A-nano chargé en NO sont ensuite mélangés de la même façon avec la crème.

Pour mesurer le relargage de NO par un échantillon sous forme de crème, de l'azote gazeux d'humidité connue (11% d'eau par passage du flux gazeux sur une solution aqueuse de LiCl) est passé sur le mélange de crème et de poudre ; le flux gazeux résultant est ensuite envoyé vers l'analyseur, et la quantité de NO (en ppm et ppb) enregistrée. Cette approche est valable pour des applications cutanées, où le solide est en contact avec la peau et la libération du NO se réalise en présence de l'humidité de la peau.

La quantité de NO libérée par la crème est 6-8 fois plus petite que la forme poudre (Figures 87 et 88) car la paraffine qui est hydrophobe, ne permet pas le contact du solide avec le flux de vapeur d'eau.

La libération à des concentrations biologiquement actives est réalisée pendant 10 heures dans le cas du solide MIL-88A de taille micrométrique (Figure 87). Par contre, on observe une libération très rapide (10 minutes ; Figure 88) lorsque la crème comprend du MIL-88A-nano. Ces résultats, en total concordance avec ceux obtenus avec la poudre, sont dues à une longueur de diffusion plus petite et aussi à un meilleur contact de la crème grâce à la surface plus importante de ces nanoparticules.

La figure 85 représente les profils de relargage de NO sous pression de vapeur d'eau du solide MIL-88A (3 échantillons sous les mêmes conditions) sous forme de crème. La quantité de NO relargué (NOᵣₑₗ en mmol.g⁻¹) est exprimée en fonction du temps t (en heures).

La figure 86 représente les profils de relargage de NO sous pression de vapeur d'eau du solide MIL-88A-nano sous forme de crème (courbe (b)) et sous forme de poudre (courbe (a)) en comparaison avec la libération dans une solution PBS (courbe (c)). La quantité de NO relarguée (NOᵣₑₗ en mmol.g⁻¹) est exprimée en fonction du temps t (en heures).

### LISTE DES REFERENCES

[1] Demande de brevet US 10/039,733
[2] Demande de brevet US 10/061,147
[3] Demande de brevet US 10/137,043
[4] Nitric oxide function in the skin, Cals-Grierson MM, Ormerod AD, NITRIC OXIDE-BIOLOGY AND CHEMISTRY 2004, 10(4), 179-193
   Topically applied nitric oxide induces T-Lymphocyte infiltration in human skin, but minimal inflammation, Mowbray M, Tan XJ, Wheatley PS, et al., JOURNAL OF INVESTIGATIVE DERMATOLOGY 2008, 128(2), 352-360
[5] Effects of nitric oxide and oxidation in vivo and postmortem on meat tenderness, Warner RD, Dunshea FR, Ponnampalam EN, et al. MEAT SCIENCE Volume: 71 Issue: 1 Pages: 205-217, 2005
[6] L.K. Keefer Nature Materials, 2, 357(2003)
[7] P.G. Parzuchowski et al, J. Am. Chem. Soc. 124, 12182(2002)
[8] J.T. Mitchell-Koch et al, Angew Chemie, 43, 2806 (2004)
[9] A.D. Ormerod et al, J. Invest Dermatol 113, 392-397(1999)
[10] WO 2005/003032
[11] P.S. Wheatley et al, Stud Surf Sci Catal, 158, 2033-2040 (2005) ;
   P.S. Wheatleyet al, J. Am. Chem. Soc., 128, 502-509, (2006)
   B. Xiao et al, J. Am. Chem. Soc., 129, 1203-1209 (2007)
[12] Pollution fighter turns clot buster, New Scientist, 5th February (2005)
[13] M. Mowbray et al, J. Invest Dermatol, 126, 102 (2006)
[14] C. T. Dziobkowski, T. J. Wrobleski, D. B. Brown, Inorg. Chem. 1982, 20, 671
[15] L. Anzalone et al., J. Org. Chem. 1985, 50, 2128
[16] S. Ameerunisha et al., J. chem. Soc. Perkin Trans. 2 1995, 1679
[17] C. Serre et al., Angew. Chem. Int. Ed. 2004, 43, 6286
   C. Serre et al., Chem. Comm. 2006, 284-286 [29]
[18] Serre et al., Science, 2007, 315, 1828 [30]
[19] C. Serre et al. J. Am. Chem. Soc., 2005, 127, 16273-16278
[20] K. Byrapsa, M. Yoshimura, "Handbook of hydrothermal technology », Noyes Publications, Parkridge, New Jersey USA, William Andrew Publishing, LLC, Norwich NY USA, 2001
[21] G. Tompsett, W. C. Conner, K. S. Yngvesson, ChemPhysChem. 2006, 7, 296
[22] S.-E. Park, J.-S. Chang, Y. K. Hwang, D. S. Kim, S. H. Jhung, J.-S. Hwang, Catal. Survey Asia 2004, 8, 91
[23] C. S. Cundy, Collect. Czech. Chem. Commum. 1998, 63, 1699
[24] S. H. Jhung, J.-H. Lee, J.-S. Chang, Bull. Kor. chem. Soc. 2005, 26, 880
[25] A. Pichon, Cryst. Eng. Comm. 8, 2006, 211-214
[26] D. Braga, Angew. chem. Int. Ed. 45, 2006, 142-246
[27] D. Braga, Dalton Trans., 2006, 1249-1263.
[28] R. ALBERTO AND R. MOTTERLINI, DALTON TRANS, 2007, 1650
[29] Sabine Balthasar, Kerstin Michaelis, Norbert Dinauer, Hagen von Briesen, Jörg Kreuter and Klaus Langer, "Preparation and characterisation of antibody modified gelatin nanoparticles as drug carrier system for uptake in lymphocytes", Biomaterials, 2005, 26, 15, 2723-2732.
[30] Ruxandra Gref, Patrick Couvreur, Gillian Barratt and Evgueni Mysiakine, "Surface-engineered nanoparticles for multiple ligand coupling", Biomaterials, 2003, 24, 24, 4529-4537.
[31] Keefer L. K., Nat. Mater. 2003, vol 2, 357.
[32] A. R. Butler and R. Nicholson, Life, Death and Nitric Oxide, The Royal Society of Chemistry, Cambridge, 2003; F. Murad, N. Engl. J. Med., 'Nitric Oxide and Cyclic GMP in Cell Signaling and Drug Development' 2006, 355, 2003.
[33] C. Napoli and L. J. Ignarro, 'NO-Releasing Drugs' Annu. Rev. Pharmacol. Toxicol., 2003, 43, 97.
[34] T. R. Johnson, B. E. Mann, J. E. Clark, R. Foresti, C. Green and R. Motterlini, 'Metal carbonyls: a new class of pharmaceuticals Angew. Chem., Int. Ed., 2003, 42, 3722; S. W. Ryter, J. Alam and A. M. K. Choi, 'CO-metal interaction: vital signalling from a lethal gas' Physiol. Rev., 2006, 86, 583 ; J. Boczkowski, J. J. Poderoso and R. Motterlini, 'CO-metal interaction: vital signalling from a lethal gas' Trends Biochem. sci 2006, 31, 614 ; R. Motterlini, B. E. Mann and R. Foresti, 'Therapeutic applications of carbon-monoxide-releasing molecules' Expert Opin. Invest. Drugs, 2005, 14, 1305.
[35] L. E. Otterbein, F. H. Bach, J. Alam, M. Soares, H. T. Lu, M.Wysk, R. J. Davis, R. A. Flavell, A. M. K. Choi, Nat. Med. 2000,6, 422 - 428.
[36] J. T. Chapman, L. E. Otterbein, J. A. Elias, A. M. K. Choi, Am. J. Physiol. Lung Cell Mol. Physiol. 2001, 281, L209 - L215.
[37] K. Sato, J. Balla, L. Otterbein, R. N. Smith, S. Brouard, Y. Lin, E. Csizmadia, J. Sevigny, S. C. Robson, G. Vercellotti, A. M. Choi,F. H. Bach, M. P. Soares, J. Immunol. 2001, 166, 4185 - 4194K.
[38] M. Stupfel, G. Bouley, Ann. N. Y. Acad. Sci. 1970, 174, 342 - 368.
[39] F. Amersi, X. D. Shen, D. Anselmo, J. Melinek, S. Iyer, D. J. Southard, M. Katori, H. D. Volk, R.W. Busuttil, R. Buelow, J. W. Kupiec-Weglinski, Hepatology 2002, 35, 815 - 823.
[40] L. Gunther, P. O. Berberat, M. Haga, S. Brouard, R. N. Smith, M. P. Soares, F. H. Bach, E. Tobiasch, Diabetes 2002, 51, 994 -999.
[41] N. Ozawa, N. Goda, N. Makino, T. Yamaguchi, Y. Yoshimura, M. Suematsu, J. Clin. Invest. 2002, 109, 457 - 467.
[42] S. A. Bainbridge, A. E. Farley, B. E. McLaughlin, C. H. Graham, G. S. Marks, K. Nakatsu, J. F. Brien, G. N. Smith, Placenta 2002, 23, 563 - 569.
[43] D. Morse, J. Sethi, A. M. K. Choi, Crit. Care Med. 2002, 30, S12 -S17.
[44] S. P. Gaine, G. Booth, L. Otterbein, N. A. Flavahan, A. M. Choi,C. M. Weiner, J. Vasc. Res. 1999, 36, 114 - 119; T. Morita, S. A Mitsialis, H. Koike, Y. Liu, S. Kourembanas, J. Biol. Chem. 1997, 272, 32 804 - 32809.
[45] R. Motterlini, A. Gonzales, R. Foresti, J. E. Clark, C. J. Green, R. M. Winslow, Circ. Res. 1998, 83, 568 - 577; I. A. Sammut, R Foresti, J. E. Clark, D. J. Exon, M. J. Vesely, P. Sarathchandra, C. J. Green, R. Motterlini, Br. J. Pharmacol. 1998, 125, 1437 -1444.
[46] L. E. Otterbein, B. S. Zuckerbraun, M. Haga, F. Liu, R. Song, A.Usheva, C. Stachulak, N. Bodyak, R. N. Smith, E. Csizmadia, S.Tyagi, Y. Akamatsu, R. J. Flavell, T. R. Billiar, E. Tzeng, F. H. Bach, A. M. K. Choi, M. G. Soares, Nat. Med. 2003, 9, 183 - 190.
[47] D. E. Baranano, S. Dore, C. D. Ferris, S. H. Snyder, Clin.Neurosci. Res. 2001, 1, 46 - 52) et de l'ovulation (W. Tschugguel, F. Stonek, Z. Zhegu, W. Dietrich, C. Schneeberger, T. Stimpfl, T. Waldhoer, W. Vycudilik, J. C. Huber, J. Clin. Endocrinol. Metab. 2001, 86, 3833 - 3839.
[48] M. McLean, M. Bowman, V. Clifton, R. Smith, A. B. Grossman, J. Clin. Endocrinol. Metab. 2000, 85, 2345 - 2349.
[49] R. Wang, FASEB J., 2002, 16, 1792; R. Wang, Antioxid. Redox Signaling, 2003, 5, 493.
[50] W. M. Zhoa, K. N. Houk and L. P. Olson, EMBO J., 2001, 20, 6008.
[51] Y. Liu, J.F. Eubank, A.J. Cairns, J. Eckert, V.Ch. Kravtsov, R. Luebke, M. Eddaoudi, Angew. Chem. Int. Ed. 2007, 46, 3278-3283.
[52] Robert A. Moss, Hugo Morales-Rojas, Saketh Vijayaraghavan and Jingzhi Tian, J. Am. Chem. Soc., 2004, 126,(35), 10923 - 10936.
[53] B. Baleux et al. C.R. Acad. Sciences Paris, série C, 274 (1972) pages 1617-1620.
[54] R. Gref et al. Colloids and Surfaces B: Biointerfaces, Volume 18, Issues 3-4, October 2000, pages 301-313.
[55] Kohler N. et al., J Am Chem Soc 2004; 126 ; 7206-7211.
[56] Peggy Chan et al., Biomaterials, volume 28, Issue 3, 2007, pp 540-549.
[57] J.H. Van Steenis et al., J Control Release 87 (2003), pp. 167-176.
[58] A. Gabizon, H. Shmeeda, A.T. Horowitz and S. Zalipsky, Tumor cell targeting of liposome-entrapped drugs with phospholipids-anchored folic acid-PEG conjugates, Adv Drug Deliv Rev 56 (2004), pp. 1177-1192.
[59] Ameerunisha et al., J. Chem. Soc. Perkin Trans. 2, 1679, 1995.
[60] C. Serre, G. Férey, Inorg. Chem. 1999, 38, 5370-5373.
[61] P.D.C. Dietzel, B. Panella, M. Hirscher, R. Blom, H. Fjellvag, Chem. Commun., 2006, 956-961 et P.D.C. Dietzel, R.E. Johnsen, R. Blom, H. Fjellvag, P.Chem. Eur. J., 2008, 14, 2389-2397.

## Revendications

1. Solide MOF cristallin poreux chargé en au moins un gaz base de Lewis choisi dans le groupe constitué de NO, CO et H₂S dont au moins une partie se coordine avec M, ledit solide comprenant une succession tridimensionnelle de motifs répondant à la formule (I) suivante :
MₘOₖXᵢLₚ (I)
dans laquelle :
• chaque occurrence de M représente un ion d'un métal de transition M²⁺ dans lequel M est Fe, et dans lequel z est de 2 à 4;
• m est 1 à 12 ;
• k est 0 à 4 ;
• 1 est 0 à 18 ;
• p est 1 à 6 ;
• X est un anion choisi dans le groupe constitué de OH⁻, Cl⁻, F⁻, I⁻, Br⁻, SO₄²⁻, NO₃⁻, ClO₄⁻, PF₆⁻, BF₄⁻, R-(COO)ₙ⁻ où R est tel que défini ci-dessous, R¹-(COO)ₙ⁻, R¹-(SO₃)ₙ⁻, R¹-(PO₃)ₙ⁻, où R¹ est un hydrogène, un alkyle en C₁ à C₁₂, linéaire ou ramifié, éventuellement substitué, un aryle où n est un nombre entier de 1 à 4 ;
• L est un ligand espaceur constitué d'un radical R comportant q groupements carboxylates 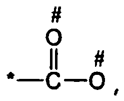 où
- q est 2, 3, 4, 5 ou 6 ; * désigne le point d'attachement du carboxylate avec le radical R ;
- # désigne les points d'attachement possibles du carboxylate audit ion métallique ;
- R représente :
(i) un radical C₁₋₁₃alkyle, C₂₋₁₂alcène ou C₂₋₁₂alcyne ;
(ii) un radical aryle mono- ou poly-cyclique, fusionné ou non, comprenant 6 à 50 atomes de carbone ;
(iii) un hétéroaryle mono- ou poly-cyclique, fusionné ou non, comprenant 1 à 50 atomes de carbone ;
(iv) un radical organique comprenant un élément métallique choisi dans le groupe comprenant le ferrocène, la porphyrine, la phthalocyanine;
le radical R étant éventuellement substitué par un ou plusieurs groupes R², indépendamment choisis dans le groupe comprenant C₁₋₁₀alkyle; C₂₋₁₀alcène; C₂₋₁₀alcyne; C₃₋₁₀cycloalkyle; C₁₋₁₀hétéroalkyle; C₁₋₁₀haloalkyle; C₆₋₁₀aryle; C₃₋₂₀hétérocyclique; C₁₋₁₀alkylC₆₋₁₀aryle ; C₁₋₁₀alkylC₃₋₁₀hétéroaryle ; F ; Cl ; Br; I; -NO₂ ; -CN ; -CF₃ ; -CH₂CF₃ ; -OH ; -CH₂OH ;
-CH₂CH₂OH ; -NH₂ ; -CH₂NH₂ ; -NHCHO ; -COOH ; -CONH₂ ; -SO₃H ; -CH₂SO₂CH₃ ; PO₃H₂ ; ou une fonction -GR^{G1} dans laquelle G est -O-, -S-, - NR^{G2}-, -C(=O)-, -S(=O)-, -SO₂-, -C(=O)O-. -C(=O)NR^{G2}-, -OC(=O)-, -NR^{G2}C(=O)-, -OC(=O)O-, -OC(=O)NR^{G2}-, -NR^{G2}C(=O)O-, -NR^{G2}C(=O)NR^{G2}-, -C(=S)-, où chaque occurrence de R^{G2} est indépendamment des autres occurrences de R^{G2} un atome d'hydrogène ; ou une fonction C₁₋₁₂alkyle, C₁₋₁₂hétéroalkyle, C₂₋₁₀alcène ou C₂₋₁₀alcyne, linéaire, ramifiée ou cyclique, éventuellement substituée ; ou un groupe C₆₋₁₀aryle, C₃₋₁₀hétéroaryle, C₅₋₁₀hétérocyclique, C₁₋₁₀alkyleC₆₋₁₀aryle ou C₁₋₁₀alkyleC₃₋₁₀hétéroaryle dans lequel le radical aryle, hétéroaryle ou hétérocyclique est éventuellement substitué ; ou bien, lorsque G représente -NR^{G2}-, R^{G1} et R^{G2} conjointement avec l'atome d'azote auquel ils sont liés forment un hétérocycle ou un hétéroaryle éventuellement substitué.

2. Solide selon la revendication 1, dans lequel le ligand L est un ligand di-, tri-, tétra- ou hexacarboxylate choisi dans le groupe coonstitué de : fumarate, succinate, glutarate, muconate, adipate, 2,5-thiophènedicarboxylate, téréphtalate, 2,5-pyrazine dicarboxylate, naphtalène-2,6-dicarboxylate, biphényle-4,4'-dicarboxylate, azobenzènedicarboxylate, dichloroazobenzènedicarboxylate, azobenzènetetracarboxylate, dihydroxoazobenzènedicarboxylate), benzène-1,2,4-tricarboxylaté, benzène-1,3,5-tricarboxylate, benzène-1,3,5-tribenzoate, 1,3,5-tris[4'- carboxy(1,1'-biphenyl-4-yl)benzene, benzène-1,2,4,5-tétracarboxylate, naphtalène-2,3,6,7-tétracarboxylate, naphtalène-1,4,5,8-tétracarboxylate, biphényl-3,5,3',5'-têtracarboxylate, et les analogues modifiés choisis dans le groupe comprenant le 2-aminotéréphtalate, le 2-nitrotéréphtalate, le 2-méthyltéréphtalate, le 2-chlorotéréphtalate, le 2-bromotéréphtalate, le 2,5-dihydroxotéréphtalate, le tétrafluorotéréphtalate, le 2,5-dicarboxytéréphtalate, le diméthyl-4,4'-biphénydicarboxylate, le tétraméthyl-4,4'-biphénydicarboxylate, le dicarboxy-4,4'-biphénydicarboxylate.

3. Solide selon l'une des revendications 1 ou 2, dans lequel l'anion X est choisi dans le groupe constitué de OH⁻, Cl⁻, F⁻, R-(COO)ₙ⁻, PF₆⁻, ClO₄⁻, avec R et n tel que défini dans la revendication 1.

4. Solide selon l'une quelconque des revendications 1 à 3, comprenant un pourcentage massique de M en phase sèche de 5 à 50% et/ou,
dans lequel la taille de pores du matériau MOF est de 0,4 à 6 nm et/ou,
dans lequel le solide a un volume poreux de 0 à 4 cm³/g et/ou,dans lequel le solide a une capacité de charge en gaz de 0,5 à 50 mmol de gaz par gramme de solide sec.

5. Solide selon l'une quelconque des revendications 1 à 4, dans lequel ledit solide comprend une succession tridimensionnelle de motifs répondant à la formule (I) choisis dans le groupe constitué de :
- Fe₃OX[C₂H₂(CO₂)₂]₃ de structure flexible
- Fe₃OX[C₆H₄(CO₂)₂]₃ de structure flexible
- Fe₃OX[C₁₀H₆(CO₂)₂]₃ de structure flexible
- Fe₃OX[C₁₂H₈(CO₂)₂]₃ de structure flexible
- Fe₃OX[C₄H₄(CO₂)₂]₃ de structure flexible
- Fe₁₂O(OH)₁₈(H₂O)₃[C₆H₃(CO₂)₃]₆ de structure rigide
- Fe₃OX[C₆H₃(CO₂)₃]₂ de structure rigide
- Fe₃OX[C₆H₄(CO₂)₂]₃ de structure rigide
- Fe₆O₂X₂[C₁₀H₂(CO₂)₄]₃ de structure rigide
dans laquelle, X est tel que défini dans les revendications 1 ou 2, structure rigide signifiant une structure qui se gonfle ou se contracte avec une amplitude jusqu'à 10 % et structure flexible signifiant qui se gonfle ou se contracte avec une amplitude supérieure à 10 %.

6. Solide selon l'une quelconque des revendications 1 à 5, dans lequel le gaz est NO.

7. Solide selon l'une quelconque des revendications 1 à 6, comprenant à sa surface au moins un agent de surface organique.

8. Solide selon la revendication 7, dans lequel l'agent de surface organique est choisi dans le groupe comprenant
- un oligosaccharide comme les cyclodextrines,
- un polysaccharide comme le chitosan, le dextran, le fucoïdane, l'alginate, la pectine, l'amylose, l'amidon, la cellulose, le xylane,
- un glycosaminoglycanne comme l'acide hyaluronique, l'héparine,
- un polymère comme le polyéthylène glycol (PEG), l'alcool polyvinylique, le polyéthylène imine,
- un tensioactif comme le pluronic, la lécithine,
- des vitamines comme la biotine,
- des coenzymes comme l'acide lipoique,
- des anticorps ou fragments d'anticorps,
- des aminoacides ou peptides.

9. Solide selon l'une des revendications 7 ou 8, dans lequel l'agent de surface organique est une molécule de ciblage choisie dans le groupe comprenant : la biotine, le chitosan, l'acide lipoique, un anticorps ou fragment d'anticorps, un peptide.

10. Procédé de préparation d'un solide tel que défini dans l'une quelconque des revendications 1 à 9, comprenant au moins une étape réactionnelle consistant
(i) à mélanger dans un solvant polaire :
- au moins une solution comprenant au moins un précurseur inorganique métallique se présentant sous la forme de métal M, d'un sel métallique de M ou d'un complexe de coordination comprenant un ion métallique de M,
- au moins un ligand L' comprenant un radical R comportant q groupements *-C(=O)-R³, ou
• q et R sont tels que définis précédemment,
• * désigne le point d'attachement du groupement avec le radical R,
• R³ est choisi dans le groupe comprenant un OH, un OY, ou Y est un cation alcalin, un halogène, un radical -OR⁴, -O-C(=O)R⁴, -NR⁴R^{4'}, où R⁴ et R^{4'} sont des radicaux alkyles en C₁₋₁₂,
pour obtenir un matériau MOF ;
(ii) à activer le matériau MOF obtenu en (i) ; et
(iii) à mettre en contact le matériau MOF obtenu à l'étape (ii) avec un gaz base de Lewis dont au moins une partie se coordine avec M, de façon à obtenir ledit solide.

11. Procédé selon la revendication 10, dans lequel l'étape (ii) est également une étape de réduction des centres métalliques M dudit matériau MOF en ions M²⁺ dans lequel z est de 2 à 4.

12. Procédé selon l'une des revendications 10 ou 11, dans lequel l'étape (ii) d'activation est réalisée à une température de 25 à 300°C.

13. Procédé selon l'une quelconque des revendications 10 à 12, dans lequel l'étape (ii) d'activation est réalisée à une pression de 1 à 10⁻² Pa.

14. Procédé selon l'une quelconque des revendications 10 à 13, dans lequel, dans l'étape (iii) de mise en contact, le gaz est sous forme pure ou en mélange avec un gaz inerte.

15. Procédé de préparation selon l'une quelconque des revendications 10 à 14 comprenant, en outre, une étape (iv) de fixation d'au moins un agent de surface organique, laquelle étape intervenant pendant ou après l'étape réactionnelle (i) ou après l'étape d'activation (ii) et avant l'étape (iii) de mise en contact du matériau MOF avec le gaz.

16. Procédé selon l'une quelconque des revendications 10 à 14, dans lequel dans l'étape (iii) le matériau MOF obtenu à l'étape (ii) est mis en contact avec le NO.

17. Procédé selon la revendication 15, dans lequel l'étape (iii) est réalisée à une température de -100°C à +50°C ou
dans lequel l'étape (iii) est réalisée à une pression de 10⁵ à 10⁶ Pa.

18. Composition pharmaceutique, cosmétique ou dermatologique comprenant un solide selon l'une quelconque des revendications 1 à 9 et un véhicule pharmaceutiquement ou cosmétiquement acceptable.

## Claims

1. A porous crystalline MOF solid loaded with at least one Lewis base gas chosen from the group made up of NO, CO and H₂S, at least a part of which coordinates with M, said solid comprising a three-dimensional succession of units corresponding to the following formula (I):
MₘOₓXₗLₚ (I)
in which:
• each occurrence of M represents an ion of a transition metal M^{z+} in which M is Fe, and in which z is 2 to 4;
• m is 1 to 12;
• k is 0 to 4;
• is 0 to 18;
• p is 1 to 6;
• X is an anion chosen from the group made up of OH⁻, Cl⁻, F⁻, I⁻, Br⁻, SO₄²⁻, NO₃⁻, ClO₄⁻, PF₆⁻, BF₄⁻, R-(COO)ₙ⁻ where R is as defined below, R¹-(COO)ₙ⁻,R¹-(SO₃)ₙ⁻, R¹-(PO₃)ₙ⁻, where R¹ is a hydrogen, a linear or branched, optionally substituted, C₁ to C₁₂ alkyl, or an aryl, and where n is an integer from 1 to 4;
• L is a spacer ligand made up of a radical R comprising q carboxylate groups 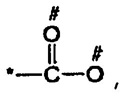 where
- q is 2, 3, 4, 5 or 6; * denotes the point of attachment of the carboxylate with the radical R;
- # denotes the possible points of attachment of the carboxylate to the said metal ion;
- R represents:
(i) a C₁₋₁₂ alkyl, C₂₋₁₂ alkene or C₂₋₁₂ alkyne radical;
(ii) a fused or nonfused, monocyclic or polycyclic aryl radical containing 6 to 50 carbon atoms;
(iii) a fused or nonfused, monocyclic or polycyclic heteroaryl containing 1 to 50 carbon atoms;
(iv) an organic radical comprising a metal element chosen from the group comprising ferrocene, porphyrin and phthalocyanin;
the R radical being optionally substituted with one or more R² groups, independently chosen from the group comprising C₁₋₁₀ alkyl; C₂₋₁₀ alkene; C₂₋₁₀ alkyne; C₃₋₁₀ cycloalkyl; C₁₋₁₀ heteroalkyl; C₁₋₁₀ haloalkyl; C₆₋₁₀ aryl; C₃₋₂₀ heterocyclic; (C₁₋₁₀) alkyl (C₆₋₁₀) aryl; (C₁₋₁₀) alkyl (C₃₋₁₀)heteroaryl; F; Cl; Br; I; -NO₂; -CN; -CF₃; -CH₂CF₃; -OH; -CH₂OH; -CH₂CH₂OH; -NH₂; -CH₂NH₂; -NHCHO; -COOH; -CONH₂; -SO₃H; -CH₂SO₂CH₃; -PO₃H₂; or a -GR^{G1} function in which G is -O-, -S-, -NR^{G2}-, -C(=O)-, -S(=O)-, -SO₂-, -C(=O)O-, -C(=O)NR^{G2}-, -OC(=O)-, -NR^{G2}C(=O)-, -OC(=O)O-, -OC(=O)NR^{G2}-, -NR^{G2}C(=O)O-, -NR^{G2}C(=O)NR^{G2}- or -C(=S)-, where each occurrence of R^{G2} is, independently of the other occurrences of R^{G2}, a hydrogen atom; or a linear, branched or cyclic, optionally substituted, C₁₋₁₂ alkyl, C₁₋₁₂ heteroalkyl, C₂₋₁₀ alkene or C₂₋₁₀ alkyne function; or a C₆₋₁₀ aryl, C₃₋₁₀ heteroaryl, C₅₋₁₀ heterocyclic, (C₁₋₁₀) alkyl (C₆₋₁₀) aryl or (C₁₋₁₀) alkyl (C₃₋₁₀) heteroaryl group in which the aryl, heteroaryl or heterocyclic radical is optionally substituted; or else, when G represents -NR^{G2}-, R^{G1} and R^{G2}, together with the nitrogen atom to which they are bonded, form a heterocycle or a heteroaryl which is optionally substituted.

2. The solid as claimed in claim 1, in which the ligand L is a di-, tri-, tetra- or hexacarboxylate ligand chosen from the group made up of: fumarate, succinate, glutarate, muconate, adipate, 2,5-thio-phenedicarboxylate, terephthalate, 2,5-pyrazine-dicarboxylate, naphthalene-2,6-dicarboxylate, biphenyl-4,4'-dicarboxylate, azobenzene-dicarboxylate, dichloroazobenzenedicarboxylate, azobenzenetetracarboxylate, dihydroxoazobenzene-dicarboxylate, benzene-1,2,4-tricarboxylate, benzene-1,3,5-tricarboxylate, benzene-1,3,5-tribenzoate, 1,3,5-tris[4'-carboxy(1,1'-biphenyl-4-yl)]benzene, benzene-1,2,4,5-tetracarboxylate, naphthalene-2,3,6,7-tetracarboxylate, naphthalene-1,4,5,8-tetracarboxylate, biphenyl-3,5,3',5'-tetracarboxylate, and the modified analogs chosen from the group comprising 2-aminoterephthalate, 2-nitroterephthalate, 2-methylterephthalate, 2-chloroterephthalate, 2-bromoterephthalate, 2,5-dihydroxoterephthalate, tetrafluoroterephthalate, 2,5-dicarboxyterephthalate, dimethyl-4,4'-biphenyldicarboxylate, tetramethyl-4,4'-biphenyldicarboxylate and dicarboxy-4,4'-biphenyldicarboxylate.

3. The solid as claimed in either of claim 1 or 2, in which the anion X is chosen from the group made up of OH⁻, Cl⁻, F⁻, R-(COO)ₙ⁻, PF₆⁻ and ClO₄⁻, with R and n as defined in claim 1.

4. The solid as claimed in any one of claims 1 to 3, comprising a mass percentage of M in the dry phase of from 5 to 50% and/or,
in which the pore size of the MOF material is from 0.4 to 6 nm and/or,
in which the solid has a pore volume of from 0 to 4 cm³/g and/or,
in which the solid has a gas loading capacity of from 0.5 to 50 mmol of gas per gram of dry solid.

5. The solid as claimed in any one of claims 1 to 4, in which said solid comprises a three-dimensional succession of units corresponding to formula (I), which are chosen from the group made up of:
- Fe₃OX[C₂H₂(CO₂)₂]₃ of flexible structure,
- Fe₃OX[C₆H₄(CO₂)₂]₃ of flexible structure,
- Fe₃OX[C₁₀H₆(CO₂)₂]₃ of flexible structure,
- Fe₃OX[C₁₂H₈(CO₂)₂]₃ of flexible structure,
- Fe₃OX[C₄H₄(CO₂)₂]₃ of flexible structure,
- Fe₁₂O(OH)₁₈(H₂O)₃C₆H₃(CO₂)₃]₆ of rigid structure,
- Fe₃OX[C₆H₃(CO₂)₃]₂ of rigid structure,
- Fe₃OX[C₆H₄(CO₂)₂]₃ of rigid structure,
- Fe₆O₂X₂[C₁₀H₂(CO₂)₄]₃ of rigid structure,
in which X is as defined in claim 1 or 2, rigid structure meaning a structure that swells or contracts with an amplitude up to 10% and flexible structure meaning that swells or contracts with an amplitude greater than 10%.

6. The solid as claimed in any one of claims 1 to 5, in which the gas is NO.

7. The solid as claimed in any one of claims 1 to 6, comprising at its surface at least one organic surface agent.

8. The solid as claimed in claim 7, in which the organic surface agent is chosen from the group comprising:
- an oligosaccharide, for instance cyclodextrins,
- a polysaccharide, for instance chitosan, dextran, fucoidan, alginate, pectin, amylose, starch, cellulose, xylan,
- a glycosaminoglycan, for instance hyaluronic acid, heparin,
- a polymer, for instance polyethylene glycol (PEG), polyvinyl alcohol, polyethyleneimine,
- a surfactant, for instance pluronic, lecithin,
- vitamins, for instance biotin,
- coenzymes, for instance lipoic acid,
- antibodies or antibody fragments,
- amino acids or peptides.

9. The solid as claimed in either of claim 7 or 8, in which the organic surface agent is a targeting molecule chosen from the group comprising: biotin, chitosan, lipoic acid, an antibody or antibody fragment, and a peptide.

10. A method for preparing a solid as defined in any one of claims 1 to 9, comprising at least one reaction step which consists:
(i) in mixing in a polar solvent:
- at least one solution comprising at least one metal inorganic precursor in the form of a metal M, of a metal salt of M or of a coordination complex comprising a metal ion of M,
- at least one ligand L' comprising a radical R comprising q groups *-C(=O)-R³, in which
• q and R are as defined above,
• * denotes the point of attachment of the group with the radical R,
• R³ is chosen from the group comprising an OH, an OY, where Y being an alkali metal cation, a halogen, or a radical -OR⁴, -O-C(=O)R⁴ or -NR⁴R^{4'}, where R⁴ and R^{4'} are C₁₋₁₂ alkyl radicals,
so as to obtain a MOF material;
(ii) in activating the MOF material obtained in (i); and
(iii) in bringing the MOF material obtained in step (ii) into contact with a Lewis base gas, at least a part of which coordinates with M, so as to obtain said solid.

11. The method as claimed in claim 10, in which step (ii) is also a step of reducing the metal centers M of said MOF material to give M^{z+} ions in which z is from 2 to 4.

12. The method as claimed in either of claim 10 or 11, in which activation step (ii) is carried out at a temperature of from 25 to 300°C.

13. The method as claimed in any one of claims 10 to 12, in which activation step (ii) is carried out at a pressure of from 1 to 10⁻² Pa.

14. The method as claimed in any one of claims 10 to 13, in which, in step (iii) of bringing into contact, the gas is in pure form or as a mixture with an inert gas.

15. The method of preparation as claimed in any one of claims 10 to 14, also comprising a step (iv) of attaching at least one organic surface agent, said step being carried out during or after reaction step (i) or after activation step (ii) and before step (iii) of bringing the MOF material into contact with the gas.

16. The method as claimed in any one of claims 10 to 14, in which, in step (iii), the MOF material obtained in step (ii) is brought into contact with NO.

17. The method as claimed in claim 15, in which step (iii) is carried out at a temperature of from -100°C to +50°C or in which step (iii) is carried out at a pressure of from 10⁵ to 10⁶ Pa.

18. A pharmaceutical, cosmetic or dermatological composition comprising a solid as claimed in any one of claims 1 to 9 and a pharmaceutically or cosmetically acceptable vehicle.

## Patentansprüche

1. Poröser kristalliner MOF-Feststoff, der mit mindestens einem Lewis-Base-Gas aus der Gruppe bestehend aus NO, CO und H₂S, von dem mindestens ein Teil mit M koordiniert, beladen ist, wobei der Feststoff eine dreidimensionale Abfolge von Einheiten der folgenden Formel (I) umfasst:
MₘOₖXₗLₚ (I)
worin:
• M jeweils für ein Ion eines Übergangsmetalls M^{z+}, worin M für Fe steht und z für 2 bis 4 steht, steht;
• m für 1 bis 12 steht;
• k für 0 bis 4 steht;
• l für 0 bis 18 steht;
• p für 1 bis 6 steht;
• X für ein Anion aus der Gruppe bestehend aus OH⁻, Cl⁻, F⁻, I⁻, Br⁻, SO₄²⁻, NO₃⁻, ClO₄⁻, PF₆⁻, BF₄⁻, R-(COO)ₙ⁻, wobei R die unten angegebene Bedeutung besitzt, R¹-(COO)ₙ-, R¹-(SO₃)ₙ⁻, R¹-(PO₃)ₙ⁻, wobei R¹ für Wasserstoff, ein lineares oder verzweigtes, gegebenenfalls substituiertes C₁- bis C₁₂-Alkyl oder ein Aryl steht und n für eine ganze Zahl von 1 bis 4 steht;
• L für einen Spacer-Liganden steht, der aus einem Rest R mit q Carboxylatgruppen 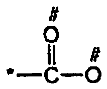 besteht, wobei
- q für 2, 3, 4, 5 oder 6 steht; * den Verknüpfungspunkt des Carboxylats mit dem Rest R bezeichnet;
- # die möglichen Verknüpfungspunkte des Carboxylats mit dem Metallion bezeichnet;
- R für:
(i) einen C₁₋₁₂-Alkyl-, C₂₋₁₂-Alken- oder C₂₋₁₂-Alkinrest;
(ii) einen kondensierten oder nicht kondensierten, monocyclischen oder polycyclischen Arylrest mit 6 bis 50 Kohlenstoffatomen;
(iii) ein kondensiertes oder nicht kondensiertes, monocyclisches oder polycyclisches Heteroaryl mit 1 bis 50 Kohlenstoffatomen;
(iv) einen organischen Rest mit einem Metallelement aus der Gruppe bestehend aus Ferrocen, Porphyrin oder Phtalocyanin
steht, wobei der Rest R gegebenenfalls durch eine oder mehrere Gruppen R² substituiert ist, die unabhängig voneinander ausgewählt sind aus der Gruppe bestehend aus C₁₋₁₀-Alkyl; C₂₋₁₀-Alken; C₂₋₁₀-Alkin; C₃₋₁₀-Cycloalkyl; C₁₋₁₀-Heteroalkyl; C₁₋₁₀-Halogenalkyl; C₆₋₁₀-Aryl; C₃₋₂₀-Heterocyclyl; (C₁₋₁₀) Alkyl (C₆₋₁₀) aryl; (C₁₋₁₀)Alkyl(C₃₋₁₀)heteroaryl; F; Cl; Br; I; -NO₂; -CN; -CF₃; -CH₂CF₃; -OH; -CH₂OH; -CH₂CH₂OH; -NH₂; -CH₂NH₂; -NHCHO; -COOH; -CONH₂; -SO₃H; -CH₂SO₂CH₃; -PO₃H₂ oder einer -GR^{G1}-Funktion, in der G für -O-, -S-, -NR^{G2}--C(=O)-, -S(=O)-, -SO₂-, -C(=O)O-, -C(=O)NR^{G2}-, -OC(=O)-, -NR^{G2}C(=O)-, -OC(=O)O-, -OC(=O)NR^{G2}-, -NR^{G2}C(=O)O-, -NR^{G2}C(=O)NR^{G2}- oder -C(=S)- steht, wobei R^{G2} jeweils unabhängig voneinander für ein Wasserstoffatom oder eine lineare, verzweigte oder cyclische, gegebenenfalls substituierte C₁₋₁₂-Alkyl-, C₁₋₁₂-Heteroalkyl-, C₂₋₁₀-Alken- oder C₂₋₁₀-Alkinfunktion oder eine C₆₋₁₀-Aryl-, C₃₋₁₀-Heteroaryl-, C₅₋₁₀-Heterocyclyl-, (C₁₋₁₀)Alkyl-(C₆₋₁₀)aryl- oder (C₁₋₁₀)Alkyl(C₃₋₁₀)heteroaryl-gruppe, in der der Aryl-, Heteroaryl- oder Heterocyclylrest gegebenfalls substituiert ist, steht; oder dann, wenn G für -NR^{G2}-steht, R^{G1} und R^{G2} auch zusammen mit dem Stickstoffatom, an das sie gebunden sind, einen Heterocyclus oder ein Heteroaryl, der bzw. das gegebenenfalls substituiert ist, bilden.

2. Feststoff nach Anspruch 1, wobei es sich bei dem Liganden L um einen Di-, Tri-, Tetra- oder Hexacarboxylatliganden handelt, der ausgewählt ist aus der Gruppe bestehend aus: Fumarat, Succinat, Glutarat, Muconat, Adipat, 2,5-Thiophendicarboxylat, Terephthalat, 2,5-Pyrazindicarboxylat, Naphthalin-2,6-dicarboxylat, Biphenyl-4,4'-di-carboxylat, Azobenzoldicarboxylat, Dichlorazobenzoldicarboxylat, Azobenzoltetracarboxylat, Dihydroxoazobenzoldicarboxylat, Benzol-1,2,4-tri-carboxylat, Benzol-1,3,5-tricarboxylat, Benzol-1,3,5-tribenzoat, 1,3,5-Tris[4'-carboxy(1,1'-biphenyl-4-yl)]benzol, Benzol-1,2,4,5-tetra-carboxylat, Naphthalin-2,3,6,7-tetracarboxylat, Naphthalin-1,4,5,8-tetracarboxylat, Biphenyl-3,5,3',5'-tetracarboxylat und den modifizierten Analogen aus der Gruppe bestehend aus 2-Aminoterephthalat, 2-Nitroterephthalat, 2-Methylterephthalat, 2-Chlorterephthalat, 2-Bromterephthalat, 2,5-Dihydroxoterephthalat, Tetrafluorterephthalat, 2,5-Dicarboxyterephthalat, Dimethyl-4,4'-biphenyldicarboxylat, Tetramethyl-4,4'-biphenyldicarboxylat und Dicarboxy-4,4'-biphenyldicarboxylat.

3. Feststoff nach Anspruch 1 oder 2, wobei das Anion X aus der Gruppe bestehend aus OH⁻, Cl⁻, F⁻, R-(COO)ₙ⁻, PF₆⁻ and ClO₄⁻ ausgewählt ist, wobei R und n die in Anspruch 1 angegebene Bedeutung besitzen.

4. Feststoff nach einem der Ansprüche 1 bis 3,
der einen prozentualen Massenanteil von M in der Trockenphase von 5 bis 50% umfasst und/oder wobei die Porengröße des MOF-Materials 0,4 bis 6 nm beträgt und/oder
wobei der Feststoff ein Porenvolumen von 0 bis 4 cm³/g aufweist und/oder
wobei der Feststoff eine Gasbeladungskapazität von 0,5 bis 50 mmol Gas pro Gramm trockenem Feststoff aufweist.

5. Feststoff nach einem der Ansprüche 1 bis 4, wobei der Feststoff eine dreidimensionale Abfolge von Einheiten der Formel (I) umfasst, die ausgewählt sind aus der Gruppe bestehend aus:
- Fe₃OX[C₂H₂(CO₂)₂]₃ mit flexibler Struktur,
- Fe₃OX[C₆H₄(CO₂)₂]₃ mit flexibler Struktur,
- Fe₃OX[C₁₀H₆(CO₂)₂]₃ mit flexibler Struktur,
- Fe₃OX[C₁₂H₈(CO)₂)₂]₃ mit flexibler Struktur,
- Fe₃OX[C₄H₄(CO₂)₂]₃ mit flexibler Struktur,
- Fe₁₂O (OH)₁₈(H₂O)₃[C₆H₃(CO₂)₃]₆ mit starrer Struktur,
- Fe₃OX[C₆H₃(CO₂)₃]₂ mit starrer Struktur,
- Fe₃OX[C₆H₄(CO₂)₂]₃ mit starrer Struktur,
- Fe₆O₂X₂[C₁₀H₂(CO₂)₄]₃ mit starrer Struktur,
wobei X die in Anspruch 1 oder 2 angebebene Bedeutung besitzt, starre Struktur eine Struktur bedeutet, die sich mit einer Amplitude von bis zu 10% ausdehnt oder zusammenzieht, und flexible Struktur eine Struktur bedeutet, die sich mit einer Amplitude von mehr als 10% ausdehnt oder zusammenzieht.

6. Feststoff nach einem der Ansprüche 1 bis 5, wobei es sich bei dem Gas um NO handelt.

7. Feststoff nach einem der Ansprüche 1 bis 6, der an seiner Oberfläche mindestens ein organisches Oberflächenmittel umfasst.

8. Feststoff nach Anspruch 7, wobei das organische Oberflächenmittel ausgewählt ist aus der Gruppe umfassend:
- ein Oligosaccharid, beispielsweise Cyclodextrine,
- ein Polysaccharid, beispielsweise Chitosan, Dextran, Fucoidan, Alginat, Pektin, Amylose, Stärke, Cellulose, Xylan,
- ein Glykosaminoglykan, beispielsweise Hyaluronsäure, Heparin,
- ein Polymer, beispielsweise Polyethylenglykol (PEG), Polyvinylalkohol, Polyethylenimin,
- ein Tensid, beispielsweise Pluronic, Lecithin,
- Vitamine, beispielsweise Biotin,
- Coenzyme, beispielsweise Liponsäure,
- Antikörper oder Antikörperfragmente,
- Aminosäuren oder Peptide.

9. Feststoff nach Anspruch 7 oder 8, wobei es sich bei dem organischen Oberflächenmittel um ein Zielsteuerungsmolekül handelt, das ausgewählt ist aus der Gruppe umfassend: Biotin, Chitosan, Liponsäure, einen Antikörper oder ein Antikörperfragment und ein Peptid.

10. Verfahren zur Herstellung eines Feststoffs gemäß einem der Ansprüche 1 bis 9, das mindestens einen Reaktionsschritt umfasst, der daraus besteht, dass man:
(i) in einem polaren Lösungsmittel:
- mindestens eine Lösung, die mindestens eine anorganische Metallvorstufe in Form eines Metalls M, eines Metallsalzes von M oder eines Koordinationskomplexes mit einem Metallion von M umfasst,
- mindestens einen Liganden L' mit einem Rest R mit q Gruppen *-C(=O)-R³, worin
• q und R die oben angegebene Bedeutung besitzen,
• * den Verknüpfungspunkt der Gruppe mit dem Rest R bezeichnet,
• R³ ausgewählt ist aus der Gruppe umfassend ein OH, ein OY, wobei Y für ein Alkalimetallkation steht, ein Halogen oder einen Rest -OR⁴, -O-C(=O)R⁴ oder -NR⁴R^{4'}, wobei R⁴ und R^{4'} für C₁₋₁₂-Alkyl stehen,
mischt, wodurch man ein MOF-Material erhält;
(ii) das in (i) erhaltene MOF-Material aktiviert und
(iii) das in Schritt (ii) erhaltene MOF-Material mit einem Lewis-Base-Gas kontaktiert, von dem mindestens ein Teil mit M koordiniert, wodurch man den Feststoff erhält.

11. Verfahren nach Anspruch 10, bei dem Schritt (ii) auch ein Schritt der Reduktion der Metallzentren M des MOF-Materials zu M^{z+}-Ionen, worin z für 2 bis 4 steht, ist.

12. Verfahren nach Anspruch 10 oder 11, bei dem man den Aktivierungsschritt (ii) bei einer Temperatur von 25 bis 300°C durchführt.

13. Verfahren nach einem der Ansprüche 10 bis 12, bei dem man den Aktivierungsschritt (ii) bei einem Druck von 1 bis 10⁻² Pa durchführt.

14. Verfahren nach einem der Ansprüche 10 bis 13, bei dem im Kontaktierungsschritt (iii) das Gas in reiner Form oder als Mischung mit einem Inertgas vorliegt.

15. Verfahren nach einem der Ansprüche 10 bis 14, das auch einen Schritt (iv) der Fixierung mindestens eines organischen Oberflächenmittels umfasst, wobei man den Schritt während oder nach dem Reaktionsschritt (i) oder nach dem Aktivierungsschritt (ii) und vor dem Schritt (iii) des Kontaktierens des MOF-Materials mit dem Gas durchführt.

16. Verfahren nach einem der Ansprüche 10 bis 14, bei dem man in Schritt (iii) das in Schritt (ii) erhaltene MOF-Material mit NO kontaktiert.

17. Verfahren nach Anspruch 15, bei dem man Schritt (iii) bei einer Temperatur von -100°C bis +50°C durchführt oder bei dem man Schritt (iii) bei einem Druck von 10⁵ bis 10⁶ Pa durchführt.

18. Pharmazeutische, kosmetische oder dermatologische Zusammensetzung, die einen Feststoff nach einem der Ansprüche 1 bis 9 und ein pharmazeutisch oder kosmetisch unbedenkliches Vehikel umfasst.
